# EUROPEAN PATENT SPECIFICATION

(11) **EP 3 336 181 B1**
(45) Date of publication and mention of the grant of the patent: **12.01.2022**
(21) Application number: 17189679.8
(22) Date of filing: 18.04.2013
(51) Int. Cl.: C12N 9/12

(54) **EGFR AND ROS1 IN CANCER**
EGFR UND ROS1 BEI KREBS
EGFR ET ROS1 DANS LE CANCER

(30) Priority: 18.04.2012 US 201261635057 P; 15.03.2013 US 201361787033 P
(43) Date of publication of application: 20.06.2018
(62) Divisional of application: 13778278.5
(73) Proprietor: Cell Signaling Technology, Inc., Danvers, MA 01923 (US)
(72) Inventor: CROSBY, Katherine Eleanor, Middleton, MA 01949 (US); MCGUINNESS RIMKUNAS, Victoria, Reading, MA 01867 (US); SILVER, Matthew Ren, Rockport, MA 01966 (US); HAACK, Herbert, South Hamilton, MA 01982 (US)
(74) Representative: Hoffmann Eitle

(56) References cited:
- CHENGUANG LI ET AL: "Spectrum of Oncogenic Driver Mutations in Lung Adenocarcinomas from East Asian Never Smokers", PLOS ONE, vol. 6, no. 11, 30 November 2011 (2011-11-30), page e28204, XP055209333, DOI: 10.1371/journal.pone.0028204
- TAKAAKI SASAKI ET AL: "A novel ALK secondary mutation and EGFR signaling cause resistance to ALK kinase inhibitors", CANCER RESEARCH, AMERICAN ASSOCIATION FOR CANCER RESEARCH, US, vol. 71, no. 18, 15 September 2011 (2011-09-15), pages 6051-6060, XP003031319, ISSN: 0008-5472, DOI: 10.1158/0008-5472.CAN-11-1340 [retrieved on 2011-07-26]
- T. SASAKI ET AL: "New Strategies for Treatment of ALK-Rearranged Non-Small Cell Lung Cancers", CLINICAL CANCER RESEARCH, vol. 17, no. 23, 18 October 2011 (2011-10-18), pages 7213-7218, XP055090585, ISSN: 1078-0432, DOI: 10.1158/1078-0432.CCR-11-1404
- V. M. RIMKUNAS ET AL: "Analysis of Receptor Tyrosine Kinase ROS1-Positive Tumors in Non-Small Cell Lung Cancer: Identification of a FIG-ROS1 Fusion", CLINICAL CANCER RESEARCH, vol. 18, no. 16, 15 August 2012 (2012-08-15), pages 4449-4457, XP055209410, ISSN: 1078-0432, DOI: 10.1158/1078-0432.CCR-11-3351

## Description

### BACKGROUND

This disclosure relates generally to proteins and genes involved in cancer (e.g., human cancer), and to the detection, diagnosis and treatment of cancer.

Many cancers are characterized by disruptions in cellular signaling pathways that lead to aberrant control of cellular processes including growth and proliferation. These disruptions are often caused by changes in the activity of particular signaling proteins, such as kinases.

In some cases, aberrant expression or activity of protein kinase proteins can be a causative agent of (and a driver of) cancer. Aberrant expression or activity can be caused, e.g., by activating mutations that increase kinase activity of the protein, fusion of the protein (or kinase portion thereof) with a secondary protein (or portion there), expression of a truncated portion of the protein, or by abnormal regulation of expression of the full-length protein.

The oncogenic role of receptor tyrosine kinases (RTKs) have been implicated in blood cancers such as lymphoma and leukemia, as well as many types of solid tumor cancers including, lung cancer, colon cancer, liver cancer, brain cancer, and breast cancer. Unfortunately solid tumors cancer is often not diagnosed at an early stage, and it often does not respond completely to surgery even when combined with chemotherapy or radiotherapy. For example, non-small cell lung carcinoma NSCLC is the leading cause of cancer death in the United States, and accounts for about 87% of all lung cancers. *See* "*Cancer Facts and Figures 2005,*" American Cancer Society.

Thus, it would be useful to discover new ways to identify cancer at an early stage, and new ways (and new reagents) to treat cancer.

### SUMMARY

This disclosure is based, at least in part, upon the discovery that epidermal growth factor receptor (EGFR) mutations and c-ros oncogene 1 (ROS1) fusions may be co-expressed in tumors. This discovery can allow for identification of patients whose tumors can benefit from therapy with one or both of an EGFR-inhibiting therapeutic or a ROS1-inhibting therapeutic.

Accordingly, in one aspect the disclosure features methods that include detecting in a biological sample from a patient having or suspected of having cancer the presence of a polypeptide having ROS1 kinase activity and detecting in the biological sample the presence of a mutant EGFR polypeptide as recited in the claims In some embodiments, the sample is from a tumor. In some embodiments, the sample is from the lung of the patient. The sample may be from a cancer or carcinoma, e.g., a lung cancer (e.g., a non-small cell lung cancer).

The polypeptide having ROS1 kinase activity can be, e.g., a ROS1 fusion. Exemplary ROS1 fusions include SLC34A2-ROS1 fusion proteins, CD74-ROS1 fusion proteins, and FIG-ROS1 fusion proteins, TPM3-ROS1 fusion proteins (Takeuchi et al., 2012, Nat. Med., 18:378-381), SDC4-ROS1 fusion proteins (Id.), EZR-ROS1 fusion proteins (Id.), and LRIG3-ROS1 fusion proteins (Id.).

The mutant EGFR polypeptide can include a mutation in the kinase domain of EGFR. Exemplary mutations include deletions in exon 19 and the mutation of leucine at residue 858 to arginine (L858R).

One or both of detecting the presence of a polypeptide having ROS1 kinase activity and detecting the presence of a mutant EGFR polypeptide can include, e.g., the use of an antibody (e.g., a ROS1 fusion or EGFR mutant specific antibody) and/or the use of mass spectrometry.

The methods further include treating the patient with an inhibitor of ROS1 kinase activity and an EGFR inhibitor. In some embodiments, the patient is treated with one compound that inhibits ROS1 kinase activity and EGFR, e.g., AP26113.

In another aspect, the disclosure features methods of treating a patient for cancer that include determining that a biological sample from a tumor in the patient includes a polypeptide having ROS1 kinase activity or a polynucleotide encoding the same and a mutant EGFR polypeptide or a polynucleotide encoding the same and administering to the patient a therapeutically effective amount of a ROS1 inhibitor (e.g., crizotinib, ASP3026, NVP TAE-684, CH5424802, and AP26113) and an EGFR inhibitor (e.g., gefitinib, erlotinib, cetuximab, afatinib, necitumumab, nimotuzumab, PF299804, RO5083945, ABT-806, and AP26113), thereby treating the patient for cancer.

In some embodiments, the tumor is a lung cancer, e.g., a carcinoma or non-small cell lung cancer.

The polypeptide having ROS1 kinase activity can be, e.g., a ROS1 fusion. Exemplary ROS1 fusions include SLC34A2-ROS1 fusion proteins, CD74-ROS1 fusion proteins, and FIG-ROS1 fusion proteins.

The mutant EGFR polypeptide can include a mutation in the kinase domain of EGFR. Exemplary mutations include deletions in exon 19 and the mutation of leucine at residue 858 to arginine (L858R).

In another aspect, the disclosure provides methods of treating a patient for cancer, that include: detecting the presence in a biological sample from a patient having or suspected of having cancer of one or more polypeptides selected from the group consisting of a polypeptide having ROS1 kinase activity, a polypeptide having anaplastic lymphoma kinase (ALK) kinase activity, and a mutant EGFR polypeptide; and administering a therapeutically effective amount of one or more of an ALK/ROS1-inhibiting therapeutic and an EGFR-inhibiting therapeutic to the patient, thereby treating the patient for cancer. In some embodiments, the detecting step is performed by using a reagent that specifically binds to a polypeptide having ROS1 kinase activity, a polypeptide having ALK kinase activity, or a mutant EGFR polypeptide.

The detecting step is performed by using a reagent that specifically binds to a polynucleotide encoding a polypeptide having ROS1 kinase activity, a polypeptide having ALK kinase activity, or a mutant EGFR polypeptide.

In a further aspect, the disclosure provides methods for identifying a patient with cancer or suspected of having cancer as a patient likely to respond to an ALK-inhibiting therapeutic that include: contacting a biological sample from a patient with a first reagent that specifically binds a polypeptide having ROS1 kinase activity and a second reagent that specifically binds to a polypeptide having ALK kinase activity and detecting whether the first reagent or the second reagent specifically binds to the biological sample, wherein detection of binding of either the first reagent or the second reagent to the biological sample identifies the patient as a patient likely to respond to an ALK-inhibiting therapeutic.

In a further aspect, the disclosure provides methods for identifying a patient with cancer or suspected of having cancer as a patient likely to respond to an ALK-inhibiting therapeutic, that include: contacting a biological sample from a patient with a first reagent that specifically binds a polypeptide having ROS1 kinase activity or specifically binds to a polynucleotide encoding a polypeptide having ROS1 kinase activity and a second reagent that specifically binds to a polypeptide having ALK kinase activity or specifically binds to a polynucleotide encoding a polypeptide having ALK kinase activity and detecting whether the first reagent or the second reagent specifically binds to the biological sample, wherein detection of binding of either the first reagent or the second reagent to the biological sample identifies the patient as a patient likely to respond to an ALK-inhibiting therapeutic.

In various embodiments, the first reagent specifically binds to full length ROS1 kinase protein. In various embodiments, the second reagent specifically binds to full length ALK kinase protein. In various embodiments, the first reagent specifically binds to the kinase domain of ROS1 kinase protein. In various embodiments, the second reagent specifically binds to the kinase domain of ALK kinase protein. In some embodiments, the first reagent is an antibody. In some embodiments, the second reagent is an antibody.

In the above methods, the patient is a human patient and the cancer (or suspected cancer) is from a human. In some embodiments, the ROS1-inhibiting therapeutic or the ALK-inhibiting therapeutic is PF-02341066, NVP TAE-684, or AP26113. In some embodiments, the ROS1-inhibiting therapeutic or ALK-inhibiting therapeutic is AP26113, CEP-14083, CEP-14513, CEP11988, CH5424802, WHI-P131 and WHI-P154.

In various embodiments of the above methods, the biological sample is from the cancer or suspected cancer of the patient. In some embodiments, the cancer is a solid tumor cancer. In some embodiments, the cancer is leukemia. In some embodiments, the cancer is lymphoma. In some embodiments, the cancer is a lung cancer (e.g., a non-small cell lung carcinoma or a small cell lung carcinoma). In some embodiments, the cancer is a brain cancer (e.g., glioblastoma). In some embodiments, the cancer is a liver cancer (e.g., cholangiocarcinoma). In some embodiments, the cancer is colon cancer. In some embodiments, the cancer is breast cancer. In some embodiments, the cancer is ovarian cancer.

In further embodiments of the above methods, the biological sample is selected from the group consisting of a tumor biopsy, a bronchoalveolar lavage, a circulating tumor cell, a tumor resection, a fine needle aspirate, a lymph node, a bone marrow sample, and an effusion (e.g., pleural effusion).

In some embodiments of the above methods, the detection reagents (e.g., antibodies) are detectably labeled. In another embodiment, a reagent (or first reagent) specifically binds to a full length ROS1 polypeptide. In another embodiment, a reagent (or first reagent) specifically binds to a ROS1 kinase domain. In another embodiment, a reagent (or first reagent) specifically binds to a ROS1 fusion polypeptide (e.g., specifically binds to a CD74-ROS1 fusion polypeptide, an SLC34A2-ROS1(S) polypeptide, an SLC34A2-ROS1(L) polypeptide, an SLC34A2-ROS1(VS) polypeptide, a FIG-ROS1 (L) polypeptide, a FIG-ROS1(S) polypeptide, or a FIG-ROS1(VL) polypeptide. In various embodiments, the polypeptide having ROS1 kinase activity (to which a detection reagent specifically binds) includes the amino acid sequence of SEQ ID NO: 1, SEQ ID NO: 27, SEQ ID NO: 24, SEQ ID NO: 22, SEQ ID NO: 26, SEQ ID NO: 13, SEQ ID NO: 7, SEQ ID NO: 5, or SEQ ID NO: 11. These ROS1 fusions have been previously described (see U.S. Patent Publication Nos. 20100221737 and 20100143918, and PCT Publication No, WO2010/093928, all of which are hereby incorporated by reference in their entirety).

In some embodiments of the above methods, a reagent (e.g., or first reagent) specifically binds to full length ALK polypeptide. In some embodiments, the reagent specifically binds to an ALK kinase domain. In some embodiments, the reagent (or second reagent) specifically binds to an ALK fusion polypeptide selected from the group consisting of NPM-ALK, ALO17-ALK, TFG-ALK, MSN-ALK, TPM3-ALK, TPM4-ALK, ATIC-ALK, MYH9-ALK, CLTC-ALK, SEC31L1-ALK, RANBP2-ALK, CARS-ALK, EML4-ALK, KIF5B-ALK, and TFG-ALK.

In various embodiments of the above methods, the polypeptide having ROS1 kinase activity is a full-length ROS1 polypeptide. In another embodiment, the polypeptide is a ROS1 fusion polypeptide. In another embodiment, the ROS1 fusion polypeptide is selected from the group consisting of a CD74-ROS1 fusion polypeptide, an SLC34A2-ROS1(S) polypeptide, an SLC34A2-ROS1(L) polypeptide, an SLC34A2-ROS1(VS) polypeptide, a FIG-ROS1 (L) polypeptide, a FIG-ROS1(S) polypeptide, and a FIG-ROS1(VL) polypeptide. In various embodiments, the polypeptide having ROS1 kinase activity includes the amino acid sequence of SEQ ID NO: 1, SEQ ID NO: 27, SEQ ID NO: 24, SEQ ID NO: 22, SEQ ID NO: 26, SEQ ID NO: 13, SEQ ID NO: 7, SEQ ID NO: 5, or SEQ ID NO: 11.

In various embodiments of the above methods, the polypeptide having ALK kinase activity is full length ALK polypeptide. In another embodiment, the polypeptide is an ALK fusion polypeptide. In another embodiment, the ALK fusion polypeptide is selected from the group consisting of NPM-ALK, ALO17-ALK,TFG-ALK, MSN-ALK, TPM3-ALK, TPM4-ALK, ATIC-ALK, MYH9-ALK, CLTC-ALK, SEC31L1-ALK, RANBP2-ALK, CARS-ALK, EML4-ALK, KIF5B-ALK, and TFG-ALK.

In some embodiments, the above methods are implemented in a format selected from the group consisting of a flow cytometry assay, an in vitro kinase assay, an immunohistochemistry (IHC) assay, an immunofluorescence (IF) assay, an Enzyme-linked immunosorbent assay (ELISA) assay, and a western blotting analysis assay.

In some embodiments of the above methods, the kinase activity of a polypeptide (e.g., a polypeptide having ALK kinase activity, a polypeptide having ROS1 kinase activity, or a mutant EGFR polypeptide) is detected. In further embodiments, a detection reagent is a heavy-isotope labeled (AQUA) peptide. In another embodiment, the heavy-isotope labeled (AQUA) peptide includes an amino acid sequence that includes a fusion junction of an ROS1 fusion polypeptide, a fusion junction of an ALK fusion polypeptide, or a fragment of a mutant EGFR polypeptide that includes a mutant amino acid sequence. In another embodiment, the method is implemented using mass spectrometry analysis.

Where the polynucleotide is detected, a detection reagent (e.g., or the first reagent and second reagent) is a nucleic acid probe. The reagent is detectably labeled. the nucleic acid probe is a fluorescence in-situ hybridization (FISH) probe and said method is implemented in a FISH assay or the nucleic acid probe is a polymerase chain reaction (PCR) probe and said method is implemented in a PCR assay.

In another aspect, the disclosure provides methods for inhibiting the progression of a mammalian cancer or suspected mammalian cancer that expresses one or more of a polypeptide having ROS1 kinase activity, a polypeptide having ALK kinase activity, and a mutant EGFR polypeptide, said methods including the step of inhibiting the expression and/or activity of one or more of the polypeptide having ROS1 kinase activity, the polypeptide having ALK kinase activity, and the mutant EGFR polypeptide in said mammalian cancer or suspected mammalian cancer.

In another aspect, the disclosure provides methods for inhibiting the progression of a mammalian cancer or suspected mammalian cancer that expresses one or more polynucleotides encoding a polypeptide having ROS1 kinase activity, a polypeptide having ALK kinase activity, and a mutant EGFR polypeptide that include a step of inhibiting the expression of one or more of the polynucleotides in said mammalian cancer or suspected mammalian cancer.

In further aspects, the disclosure provides methods for determining whether a compound inhibits the progression of a mammalian lung cancer or suspected mammalian lung cancer characterized by the expression of a first polypeptide with ROS1 activity, a second polypeptide with ALK activity, and/or a third polypeptide with EGFR activity that include a step of determining whether said compound inhibits the expression of said first, second, or third polypeptide in said cancer. In some embodiments, the cancer is from a human.

### BRIEF DESCRIPTION OF THE DRAWINGS

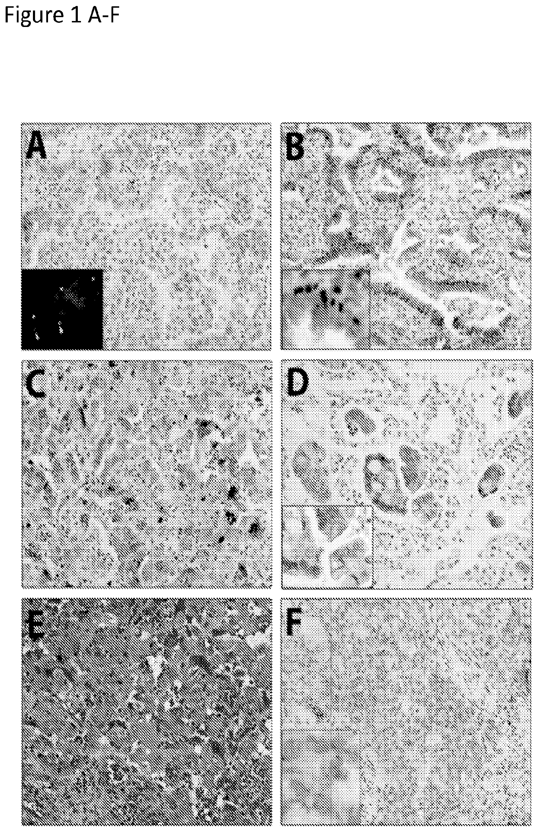

Figs. 1A-1F are photographs showing immunohistochemistry and FISH of ROS1 protein and ROS1 nucleic acid in non-small cell lung cancer (NSCLC) FFPE tumor tissues. The variation in ROS1 protein localization are shown as follows: (A) diffuse cytoplasmic with yellow arrows in inset (A) illustrating balanced translocation of the *c-ros* locus by FISH. (B) Strong punctate localization of ROS1 in adenocarcinoma with zoom (i.e., enlarged image) in inset. (C) Cytoplasmic localization of ROS1 staining in large cell carcinoma and corresponding hematoxylin and eosin stain in panel E. (D) Adenocarcinoma with unique cytoplasm staining and membrane localization with zoom in inset showing membrane staining. (E) Hematoxylin and eosin stain corresponding to ROS1 staining in panel C. (F) Punctate vesicular staining with zoom in inset showing vesicle staining.
Fig. 2A and B are images showing specific detection of the ROS1 fusion/translocation (in a human NSCLC cell line) by FISH using a 2-color break-a-part probe. Figure 2A shows the locations on the ROS1 gene where the FISH probes hybridize, and Fig. 2B shows the rearrangement of the ROS1 gene in a human NSCLC cell line (left) and a human NSCLC tumor, resulting in separate orange and green signals.
Fig. 3 is a schematic diagram showing where the DNA probes of the two probe sets hybridize to the ROS1 gene and the FIG gene. The proximal probe of both probe sets, namely RP1-179P9, will give an orange signal while all three distal probes will give a green signal. Probe set 1 was derived from c-ros, and if a balanced translocation occurs, the orange will separated from the green; however if a FIG-ROS1 translocation occurs, the green signal will disappear. Probe set 2 was derived from c-ros (orange RP1-179P9) and fig (green RP11-213A17).

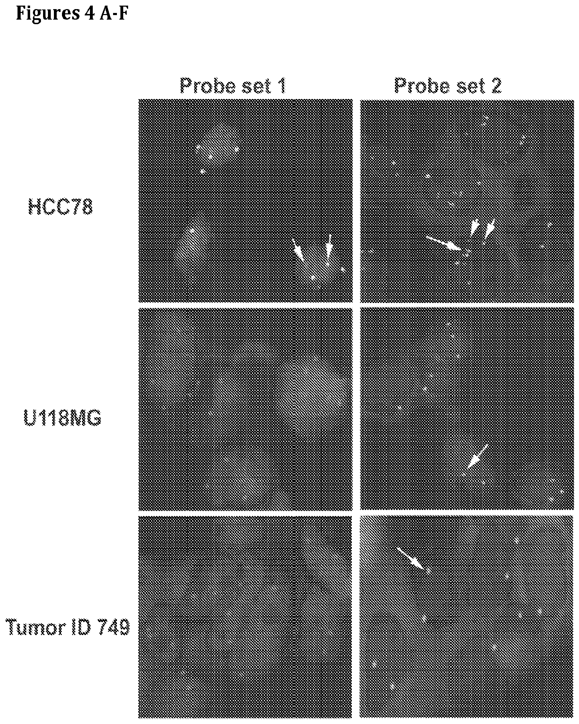

Figures 4 A-4F are photographs showing the results of FISH analysis of HCC78 cells (panels A and B), U118MG cells (panels C and D) and FFPE tumor ID 749 (panels E and F). HCC78 cells probed with probe set 1 (A) and probe set 2 (B) shows results expected from the SLC34A2-ROS1 fusion present in these cells. Yellow arrows point to split signals indicative of balanced translocation in HCC78 cells and white arrows point to intact chromosome. U118MG cells probed with probe set 1 (C) and probe set 2 (D) shows results expected from the FIG-ROS1 fusion present in these cells. FFPE tumor 749 probed with probe set 1 (E) and probe set 2 (F) is identical to U118MG cells. In both U-118 MG and Tumor ID 749 probed with probe set 1 only the c-ros (orange) probe anneals and the deleted region (green probe) is not present (panels C and E, respectively). In U-118 MG and Tumor ID 749 probed with probe set 2 (panels E and F, respectively), the c-ros (orange) and fig (green) probes come together indicating a fig-ros fusion.
Figure 5 shows the results of cDNA sequencing of the ROS1 fusion protein from tumor 749 (in "sbjt" line) and its alignment with the FIG-ROS1(S) nucleotide sequence (as "query").
Fig. 6 is a line graph showing the cellular growth response in the presence of OnM, 3nM, 10 nM, 30 nM, 100 nM, 300 nM or 1000 nM TAE-684 of BaF3 expressing FIG-ROS1(S) (red squares), BaF3 expressing FIG-ROS1(L) (blue diamonds), BaF3 expressing FLT3ITD (green triangles), and Karpas 299 cells (purple Xs).
Fig. 7 is a bar graph showing that BaF3 expressing either FIG-ROS1(S) or FIG-ROS1(L) die by apoptosis in the presence of TAE-684.
Fig. 8 is a depiction of a western blotting analysis showing that phosphorylation of both FIG-ROS1(S) and FIG-ROS1(L), as well as their downstream signaling molecules, are inhibited by TAE-684.
Figs. 9A and 9B are line graphs showing the cellular growth response in the presence of TAE-684 (Fig. 9A) or crizotinib (Fig. 9B) at 0uM, 0.01uM, 0.03uM, 0.10uM, 0.3uM, 1.0uM of BaF3 cells transduced with neo-myc (negative control; blue diamonds); BaF3 expressing FIG-ROS1(S) (purple X's), BaF3 expressing FIG-ROS1(L) (green triangles), BaF3 expressing FLT3ITD (red squares), and Karpas 299 cells (blue asterisks).
Fig. 10 is a depiction of a western blotting analysis showing that phosphorylation of both FIG-ROS1(S) and FIG-ROS1(L), as well as ALK and additional signaling molecules are inhibited by crizotinib.
Figs. 11A-D are photomicrographs depicting mutant EGFR immunohistochemical staining of ROS1/L858R positive lung adenocarcinoma case 147 stained with ROS1 D4D6 (11A), total EGFR (11B), EGFR L858R (11C) and EGFR A746-E750del (11D) antibodies.
Figs. 11E-H are photomicrographs depicting mutant EGFR immunohistochemical staining of ROS1/EGFR A746-E750del positive lung adenocarcinoma case 702 stained with ROS1 D4D6 (11E), total EGFR (11F), EGFR A746-E750 (11G) and EGFR L858R (11H) antibodies.

### DETAILED DESCRIPTION OF THE PREFERRED EMBODIMENTS

This disclosure is based, at least in part, upon the discovery that EGFR mutations and ROS1 fusions may be co-expressed in tumors. This discovery can allow for identification of patients whose tumors can benefit from therapy with one or both of an EGFR pathway-inhibiting therapeutic or a ROS1 pathway-inhibiting therapeutic.

The published patents, patent applications, websites, company names, and scientific literature referred to herein establish the knowledge that is available to those with skill in the art.

Any conflict between any reference cited herein and the specific teachings of this specification shall be resolved in favor of the latter.

Further aspects, advantages, and embodiments are described in more detail below. The patents, published applications, and scientific literature referred to herein establish the knowledge of those with skill in the art.

Any conflict between any reference cited herein and the specific teachings of this specification shall be resolved in favor of the latter. Likewise, any conflict between an art-understood definition of a word or phrase and a definition of the word or phrase as specifically taught in this specification shall be resolved in favor of the latter. As used herein, the following terms have the meanings indicated. As used in this specification, the singular forms "a," "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

Technical and scientific terms used herein have the meaning commonly understood by one of skill in the art to which the present disclosure pertains, unless otherwise defined. Reference is made herein to various methodologies and materials known to those of skill in the art. Standard reference works setting forth the general principles of antibody and recombinant DNA technology, include Harlow and Lane, Antibodies, a Laboratory Manual, Cold Spring Harbor Laboratory Press, New York (1988), Ausubel et al. Current Protocols in Molecular Biology, John Wiley & Sons, New York, N.Y. (1989 and updates through September 2010), Sambrook et al., Molecular Cloning: A Laboratory Manual, 2nd Ed., Cold Spring Harbor Laboratory Press, New York (1989); Kaufman et al., Eds., Handbook of Molecular and Cellular Methods in Biology in Medicine, CRC Press, Boca Raton (1995); McPherson, Ed., Directed Mutagenesis: A Practical Approach, IRL Press, Oxford (1991). Standard reference works setting forth the general principles of pharmacology, include Goodman and Gilman's The Pharmacological Basis of Therapeutics, 11th Ed., McGraw Hill Companies Inc., New York (2006).

Accordingly, in a first aspect, the disclosure provides methods of treating a patient for cancer that include: detecting in a biological sample from a patient having or suspected of having cancer the presence of one or both of a polypeptide selected from the group consisting of a polypeptide having ROS1 kinase activity and an mutant EGFR polypeptide; and administering a therapeutically effective amount of one or both of an EGFR pathway-inhibiting therapeutic or a ROS1 pathway-inhibiting therapeutic, thereby treating the patient for cancer. In some embodiments, the detecting step is performed by using a reagent that specifically binds to either a polypeptide having ROS1 kinase activity or a mutant EGFR polypeptide.

Human ROS1 kinase protein (encoded by the ROS1 gene) is a 2347 amino acid long receptor tyrosine kinase that is prone to aberrant expression leading to cancer. A description of full length human ROS1 kinase (with the amino acid sequence of the human ROS1 protein) can be found at UniProt Accession No. P08922. As shown in Table 1, the signal peptide, extracellular, transmembrane, and kinase domains of ROS1 are found at the following amino acid residues in SEQ ID NO: 1:

**Table 1**

| Domain | Amino acid residues in SEQ ID NO: 1 |
|---|---|
| Signal peptide | 1-27 |
| Extracellular domain | 28-1859 |
| Transmembrane domain | 1860-1882 |
| Kinase domain | 1945-2222 |

The coding DNA sequence of human ROS1 is provided herein as SEQ ID NO: 2.

Additionally, there are multiple known naturally-occurring variants of ROS1 (see, e.g., Greenman et al., Nature 446: 153-158, 2007). The nucleotide and amino acid sequences of murine full-length ROS1 are known (see, e.g., UniProt Accession No. Q78DX7). Using routine experimentation, the ordinarily skilled biologist would be readily able to determine corresponding sequences in non-human mammalian ROS1 homologues.

By "wild-type" ROS1 is meant the expression and/or activation of full length ROS1 kinase (i.e., for human ROS1, the 2347 amino acid long polypeptide or 2320 amino acid long polypeptide following removal of the signal peptide sequence) in healthy (or normal) tissue (e.g., non-cancerous tissue) of a normal individual (e.g., a normal individual who is not suffering from cancer). ROS1 kinase (full length or truncated) does not appear to be expressed in normal lung tissue in humans (e.g., see below in the Examples). However, using the methods described in the below Examples, the inventors have made the surprising discovery of ROS1 kinase expression in lung cancer. Such expression in an atypical cell (in this case a cancerous cell) where no expression is seen in a typical cell (e.g., a non-cancerous lung cell) is aberrant.

Aberrantly expressed ROS1 kinase, in the form of a fusion with another protein, namely FIG, has been reported in a glioblastoma cell line (see Charest et al., Genes Chromosomes Cancer 37: 58-71, 2003; Charest et al., Proc. Natl. Acad. Sci. USA 100: 916-921, 2003) and in liver cancer (see, e.g., PCT Publication No. WO2010/093928).

As used herein, the term "ROS1 fusion" refers to a portion of the ROS1 polypeptide that includes the kinase domain of the ROS1 protein (or polynucleotide encoding the same) fused to all or a portion of another polypeptide (or polynucleotide encoding the same), where the name of that second polypeptide or polynucleotide is named in the fusion. (The term "fusion" simply means all or a portion of a polypeptide or polynucleotide from first gene fused to all or a portion of a polypeptide or a polynucleotide from a second gene). For example, an SLC34A2-ROS1 fusion is a fusion between a portion of the SLC34A2 polypeptide (or polynucleotide encoding the same) and a portion of the ROS1 polypeptide (or polynucleotide encoding the same) that includes the kinase domain ROS1. An ROS1 fusion often results from a chromosomal translocation or inversion. There are numerous known ROS1 fusions, all of which are ROS1 fusions are useful herein, and include, without limitation, the SLC34A2-ROS1 fusion proteins whose members include SLC34A2-ROS1(VS), SLC34A2-ROS1(S), SLC34A2-ROS1(L) (see U.S. Patent Publication No. 20100143918), CD74-ROS1 (see U.S. Patent Publication No. 20100221737), the FIG-ROS1 fusion proteins whose members include FIG-ROS1(S), FIG-ROS1(L), and FIG-ROS1(XL) (see PCT Publication No. WO2010/093928), , TPM3-ROS1 fusion proteins (Takeuchi et al., 2012, Nat. Med., 18:378-381), SDC4-ROS1 fusion proteins (Id.), EZR-ROS1 fusion proteins (Id.), and LRIG3-ROS1 fusion proteins (Id.). See also WO 2011/0162295.

All of the known ROS1 fusion proteins include the full kinase domain of full length ROS1. Thus, as used herein, by a "polypeptide with ROS1 kinase activity" (or "polypeptide having ROS1 kinase activity") is meant a protein (or polypeptide) that includes the full kinase domain of full length ROS1 protein and, thus, retains ROS1 kinase activity. Non-limiting examples of proteins with ROS1 kinase activity include, without limitation, full length ROS 1 protein, the SLC34A2-ROS1 fusion proteins, whose members include SLC34A2-ROS1(VS), SLC34A2-ROS1(S), SLC34A2-ROS1(L) (see U.S. Patent Publication No. 20100143918), CD74-ROS1 (see U.S. Patent Publication No. 20100221737) and the FIG-ROS1 fusion proteins whose members include FIG-ROS1(S), FIG-ROS1(L), and FIG-ROS1(XL) (see PCT Publication No. WO2010/093928), and any truncated or mutated form of ROS1 kinase that retains the kinase domain of full-length ROS1 kinase protein. As the kinase domain of ROS1 is set forth in SEQ ID NO: 27, a "polypeptide with ROS1 kinase activity" is one whose amino acid sequence includes SEQ ID NO: 27 or a sequence at least 95% identical to SEQ ID NO: 27.

ALK (anaplastic lymphoma kinase) is a 1620 amino acid long receptor tyrosine kinase that is prone to aberrant expression leading to cancer. A description of full-length human ALK kinase (with the amino acid sequence of the human ALK protein) can be found at UniProt Accession No. Q9UM73 (see also U.S. Pat. No. 5,770,421, entitled "Human ALK Protein Tyrosine Kinase"). As shown in Table 2, the signal peptide, extracellular, transmembrane, and kinase domains of ALK are found at the following amino acid residues in SEQ ID NO: 35:

**Table 2**

| Domain | Amino acid residues in SEQ ID NO: 71 |
|---|---|
| | |
| Signal peptide | 1-18 |
| Extracellular domain | 19-1038 |
| Transmembrane domain | 1039-1059 |
| Kinase domain | 1116-1392 |

Additionally, there are multiple known naturally-occurring variants of ALK (see, e.g., Greenman et al., Nature 446: 153-158, 2007). The nucleotide and amino acid sequences of murine full-length ALK are known (see Iawahara et al., Oncogene 14: 439-449, 1997). Using routine experimentation, the ordinarily skilled biologist would be readily able to determine corresponding sequences in non-human mammalian ALK homologues.

By "wild-type" ALK is meant the expression and/or activation of full length ALK kinase (i.e., 1620 amino acid long polypeptide or 1602 amino acid long polypeptide following removal of the signal peptide sequence) in healthy (or normal) tissue (e.g., non-cancerous tissue) of a normal individual (e.g., a normal individual who is not suffering from cancer). Pulford et al., J. Cell. Physiol., 199:330-358, 2004 provides a comprehensive review relating to ALK and fusion polypeptides that include portions of the full length ALK polypeptide. In normal humans, full-length ALK expression has been detected in the brain and central nervous system, and has been reported in the small intestine and testis (see, e.g., Morris et al., Oncogene 14:2175-88, 1997). However, ALK kinase (full length or truncated) does not appear to be expressed in normal ovarian tissue in humans, a finding which the inventors have confirmed using various commercially available ALK-specific antibodies (e.g., Catalog Nos. 3791, 3633, and 3333 from Cell Signaling Technology, Inc., Danvers, MA). However, using the methods described in the below Examples, the inventors have made the surprising discovery of ALK kinase expression in ovarian cancer. Such expression in an atypical cell (in this case a cancerous cell) where no expression is seen in a typical cell (e.g., a non-cancerous ovarian cell) is aberrant.

Numerous examples of aberrantly expressed ALK kinase have been found in other cancers. For example, point mutations within the kinase domain have been found in neuroblastoma, overexpression of ALK has been found in numerous cancers (including, e.g., retinoblastoma, breast cancer, and melanoma), and fusion proteins that include the kinase domain (but not the transmembrane domain) of ALK fused to all or a portion of a second protein have been discovered in various cancers including non-small cell lung cancer (NSCLC) in inflammatory myofibroblastic tumor. See review in Palmer et al., Biochem. J. 420: 345-361 (2009), herein incorporated by reference in its entirety.

Accordingly, as used herein, the term "ALK fusion" refers to a portion of the ALK polypeptide that includes the kinase domain of ALK (polynucleotide encoding the same) fused to all or a portion of another polypeptide (or polynucleotide encoding the same), where the name of that second polypeptide or polynucleotide is named in the fusion. (The term "fusion" simply means all or a portion of a polypeptide or polynucleotide from first gene fused to all or a portion of a polypeptide or a polynucleotide from a second gene). For example, an NPM-ALK fusion is a fusion between a portion of the NPM polypeptide or polynucleotide and a portion of the ALK polypeptide (or polynucleotide encoding the same) that includes the kinase domain of ALK. An ALK fusion often results from a chromosomal translocation or inversion. There are numerous known ALK fusions, all of which are ALK fusions useful herein, and include, without limitation, NPM-ALK, ALO17-ALK, TFG-ALK, MSN-ALK, TPM3-ALK, TPM4-ALK, ATIC-ALK, MYH9-ALK, CLTC-ALK, SEC31L1-ALK, RANBP2-ALK, CARS-ALK, EML4-ALK, KIF5B-ALK, and TFG-ALK (see, e.g., Palmer et al., Biochem. J. 420: 345-361, 2009 (and the articles cited therein), U.S. Patent No. 5,770,421; Rikova et al., Cell 131: 1190-1203, 2007; Soda et al., Nature 448: 561-566, 2007; Morris et al., Science 263: 1281-84, 1994; Du et al., J. Mol. Med 84: 863-875, 2007; Panagopoulos et al., Int. J. Cancer 118: 1181-86, 2006; Cools et al., Genes Chromosomes Cancer 34: 354-362, 2002; Debelenko et al., Lab. Invest. 83: 1255-65, 2003; Ma et al., Genes Chromosomes Cancer 37: 98-105, 2003; Lawrence et al., Am. J. Pathol. 157: 377-384, 1995; Hernandez et al., Blood 94: 3265-68, 1999; Takeuchi K., Clin Cancer Res. 15:3143-49, 2009; Tort et al., Lab. Invest. 81: 419-426, 2001; Trinei et al., Cancer Res. 60: 793-798, 2000; and Touriol et al., Blood 95: 3204-07, 2000, all hereby incorporated by reference in their entirety. Some of these ALK fusions have multiple variants, all of which are considered ALK fusions and, thus, are included in the definition of ALK fusion. For example, there are multiple variants of TFG-ALK (see, e.g., Hernandez et al., Amer. J. Pathol. 160: 1487-94, 2002) and at least nine known variants of EML4-ALK (see, e.g., Horn et al., J. of Clinical Oncology 27(26): 4232-35, 2009, U.S. Patent Nos. 7,700,339 and 7,728,120 and EP Patent No. 1 914 240, all hereby incorporated by reference in their entirety).

As used herein, by the term "polypeptide with ALK kinase activity" is meant any polypeptide that retains the full kinase domain of ALK and thus, has ALK kinase activity. Non-limiting polypeptides with ALK kinase activity include full length ALK, ALK fusion polypeptides (e.g., NPM-ALK fusion, various EML4-ALK fusions, ATIC-ALK fusion, CARS-ALK fusion, ALO17-ALK fusion, TFG-ALK fusion, MSN-ALK fusion, TPM3-ALK fusion, TPM4-ALK fusion, MYH9-ALK fusion, CLTC-ALK fusion, SEC31L1-ALK fusion, RANBP2-ALK fusion, KIF5B-ALK fusion, and TFG-ALK fusion).

The epidermal growth factor receptor (EGFR; also known as ErbB-1 and HER1 in humans) is the cell-surface receptor for members of the epidermal growth factor family (EGF-family) of extracellular protein ligands. The amino acid sequence of an exemplary wild-type human EGFR (including the signal sequence) is provided herein as SEQ ID NO: 3; the amino acid sequence of wild-type human EGFR (minus the signal sequence) is provided herein as SEQ ID NO: 4. The nucleotide sequence of an exemplary wild-type human EGFR mRNA is provided herein as SEQ ID NO: 9. Patients having a non-small cell lung cancer (NSCLC) carrying a somatic mutation of epidermal growth factor receptor (EGFR) have been shown to be hyperresponsive to the EGFR tyrosine kinase inhibitors Gefitinib [Lynch, T.J., et al., N Engl J Med, 2004. 350(21): p. 2129-39, and Paez, J.G., et al., Science, 2004. 304(5676): p. 1497-500] and Erlotinib [Pao, W., et al., Proc Natl Acad Sci U S A, 2004. 101(36): p. 13306-11].

Mutations are known to arise in the EGFR molecule. As used herein, the term "mutant" or "mutation" refers to a molecule (e.g., a polypeptide or a polynucleotide) that has a different structure than the wild-type molecule. That difference in structure from the wild-type molecule includes, without limitation, a different sequence (e.g., a different amino acid or nucleotide sequence), additional sequences, missing sequences (i.e., a portion of the sequence is missing), changes in modification (methylation, phosphorylation, etc.), and/or fusion of all or part of the wild-type molecule with another molecule. By "wild-type" is meant that form of the molecule that naturally occurs in the majority of individuals of the species from which the mutant molecule is derived, and/or the form of the molecule that naturally occurs in an healthy individual (e.g., noncancerous) individual of a species from which the mutant molecule is derived. The sequence of the wild-type molecule is that typically provided in the GenBank database. For example, an amino acid sequence of wild-type human EGFR is provided in SEQ ID NO: 3 (without the 24 amino acid long signal sequence) and SEQ ID NO: 4 (with the signal sequence).

As used herein, an "EGFR mutant" includes any type of mutation (i.e., change) in an EGFR molecule that renders the EGFR mutant different than wildtype EGFR such that the mutation increases the kinase activity of the EGFR molecule and/or renders a tumor cell sensitive to one or more EGFR inhibitors. In some embodiments, the mutation is in the kinase domain of EGFR. In some embodiments, the mutation is in one of exons 18 to 21 of the human EGFR gene. The most common EGFR mutations are deletions within exon 19 (e.g., a 15-bp nucleotide in-frame deletion in exon 19 (Del E746-A750) and a point mutation replacing leucine with arginine at codon 858 in exon 21 (L858R). These two classes of EGFR mutants account for 85-90% of activating EGFR mutations [Riely, G.J., et al, Clin Cancer Res, 2006. 12:7232-41]. Exon 19 deletions include Del E746_A750; Del E746_S752>V; Del E746_T751>A; Del E746_T751; Del L747_A750>P; Del L747_E749; Del L747_P753>Q; Del L747_P753>S; Del L747_S752; Del L747_T751>P; Del L747_T751, and Del S752_I759. Other mutations include T790M, S768I, L861Q, 2240del12, G719C (Lynch et al., supra), G791A and G719S (Paez et al., 2004, Science, 304:1497-1500), and insertions in exon 19 (He et al., 2011, Clin. Cancer Res., 18:1790-97). The ability to detect mutated gene products in cancer cells can identify patients most likely benefit from such therapies, and make clinical trials more efficient and informative.

EGFR mutants can be detected by standard means known in the art. For example, mutants can be detected at the nucleotide level by sequencing, nucleic acid amplification using primers and/or probes specific for the wild-type or mutant sequence, and amplification and length analysis to detect deletional mutants. Exemplary methods to determine EGFR mutational status are disclosed in Rosell et al., 2009, N. Engl. J. Med., 361:958-967 and Li et al., 2011, PLoS ONE, 6: e28204. Kits for nucleic acid analysis are commercially available, e.g., EGFR Pyro Kit (QIAGEN), EGFR PCR Kit (QIAGEN), and EGFR RGQ PCR Kit (QIAGEN). The EGFR RGQ PCR Kit is capable of detecting 29 mutations in the EGFR gene, including 19 deletions in exon 19, T790M, L858R, L861A, S768I, and G719X (detects the presence of G719S, G719A, or G719C but does not distinguish among them).

Specific EGFR mutants can be detected at the polypeptide level (e.g., by western blot or immunohistochemistry) using mutant-specific antibodies, e.g., EGF Receptor (E746-A750del Specific) (6B6) XP^{®} Rabbit mAb or EGF Receptor (L858R Mutant Specific) (43B2) Rabbit mAb, both from Cell Signaling Technology, Inc. (Danvers, MA) or mutation-specific AQUA peptides (Stemmann et al., 2001, Cell, 107: 715-726). Mutant-specific antibodies can be prepared that bind specifically to other identified mutant EGFR polypeptides. Exemplary mutant specific antibodies are disclosed in WO 2009/126306.

As used herein, by "polypeptide" (or "amino acid sequence" or "protein") refers to a polymer formed from the linking, in a defined order, of preferably, α-amino acids, D-, L-amino acids, and combinations thereof. The link between one amino acid residue and the next is referred to as an amide bond or a peptide bond. Non-limiting examples of polypeptides include refers to an oligopeptide, peptide, polypeptide, or protein sequence, and fragments or portions thereof, and to naturally occurring or synthetic molecules. Polypeptides also include derivatized molecules such as glycoproteins and lipoproteins as well as lower molecular weight polypeptides. "Amino acid sequence" and like terms, such as "polypeptide" or "protein", are not meant to limit the indicated amino acid sequence to the complete, native amino acid sequence associated with the recited protein molecule.

It will be recognized in the art that some amino acid sequences of an indicated polypeptide (e.g., a FIG-ROS1(S) polypeptide) can be varied without significant effect of the structure or function of the mutant protein. If such differences in sequence are contemplated, it should be remembered that there will be critical areas on the protein that determine activity (e.g. the kinase domain of ROS1). In general, it is possible to replace residues that form the tertiary structure, provided that residues performing a similar function are used. In other instances, the type of residue may be completely unimportant if the alteration occurs at a non-critical region of the protein.

Thus, a polypeptide with ROS1 activity or a polypeptide with ALK activity further includes variants of the polypeptides described herein that shows substantial ROS1 kinase activity or ALK kinase activity. Some non-limiting conservative substitutions include the exchange, one for another, among the aliphatic amino acids Ala, Val, Leu and Ile; exchange of the hydroxyl residues Ser and Thr; exchange of the acidic residues Asp and Glu; exchange of the amide residues Asn and Gln; exchange of the basic residues Lys and Arg; and exchange of the aromatic residues Phe and Tyr. Further examples of conservative amino acid substitutions known to those skilled in the art are: Aromatic: phenylalanine tryptophan tyrosine *(e.g.,* a tryptophan residue is replaced with a phenylalanine); Hydrophobic: leucine isoleucine valine; Polar: glutamine asparagines; Basic: arginine lysine histidine; Acidic: aspartic acid glutamic acid; Small: alanine serine threonine methionine glycine. As indicated in detail above, further guidance concerning which amino acid changes are likely to be phenotypically silent *(i.e.,* are not likely to have a significant deleterious effect on a function) can be found in Bowie *et al*., Science *247, supra.*

In some embodiments, a variant may have "nonconservative" changes, e.g., replacement of a glycine with a tryptophan. Similar variants may also include amino acid deletions or insertions, or both. Guidance in determining which amino acid residues may be substituted, inserted, or deleted without abolishing biological or immunological activity may be found using computer programs well known in the art, for example, DNASTAR software.

The polypeptides having ROS1 kinase activity can include the full length human ROS1 protein (having an amino acid sequence set forth in SEQ ID NO: 1) and the ROS1 fusion polypeptides having the amino sequences set forth in SEQ ID NOs: 5, 7, 11, 13, 22, 24, and 26 (whether or not including a leader sequence), an amino acid sequence encoding a polypeptide that includes at least six contiguous amino acids encompassing the fusion junction (i.e., the sequences at the junction between the non-ROS1 partner protein and the ROS1 protein; see Table 3, as well as polypeptides that have at least 90% similarity, more preferably at least 95% similarity, and still more preferably at least 96%, 97%, 98% or 99% similarity to those described above.

The polypeptides having ALK kinase activity include the full length human ALK protein (having an amino acid sequence set forth in SEQ ID NO: 35) and the various ALK fusion polypeptides described herein, an amino acid sequence encoding a polypeptide that includes at least six contiguous amino acids encompassing the fusion junction (i.e., the sequences at the junction between the non-ALK partner protein and the ALK protein, as well as polypeptides that have at least 90% similarity, more preferably at least 95% similarity, and still more preferably at least 96%, 97%, 98% or 99% similarity to those described above.

Full length ROS1-specific reagents and the ROS1 fusion polypeptide specific reagents (such as polyclonal and monoclonal antibodies) or full length ALK-specific reagents and the ALK fusion polypeptide specific reagents (such as polyclonal and monoclonal antibodies) which are useful in assays for detecting ROS1 or ALK polypeptide expression and/or ROS1 or ALK kinase activity as described below or as ROS1-inhibiting therapeutics or ALK-inhibiting therapeutics capable of inhibiting ROS1 protein function/activity and/or ALK protein function/activity. Further, such polypeptides can be used in the yeast two-hybrid system to "capture" binding proteins, which are also candidate ROS1-inhibiting therapeutics or ALK-inhibiting therapeutics according to the present disclosure. The yeast two hybrid system is described in Fields and Song, Nature 340: 245-246 (1989).

In some embodiments, a detection reagent may further include a detectable label (e.g., a fluorescent label or an infrared label). By "detectable label" with respect to a polypeptide, polynucleotide, or reagent (e.g., antibody or FISH probe) disclosed herein means a chemical, biological, or other modification of or to the polypeptide, polynucleotide, or antibody, including but not limited to fluorescence (e.g., FITC or phycoerythrin), infrared, mass (e.g., an isobaric tag), residue, dye (chromophoric dye), radioisotope (e.g., ³²P), label, or tag (myc tag or GST tag) modifications, etc., by which the presence of the molecule of interest may be detected. Such a polypeptide, polynucleotide, or reagent thus called "detectably labeled." The detectable label may be attached to the polypeptide, polynucleotide, or binding agent by a covalent (e.g., peptide bond or phosphodiester bond) or non-covalent chemical bond (e.g., an ionic bond).

Reagents useful in the methods disclosed herein include, without limitation, reagents such as antibodies or binding fractions thereof, that specifically bind to full length ROS1 protein or one of the many ROS1 fusion proteins, or to full length ROS1 protein or one of the many ROS 1 fusion proteins expressed in cancer, or to an EGFR mutant polypeptide. By "specifically binding" or "specifically binds" means that a reagent or binding agent *(e.g.,* a nucleic acid probe, an antibody) interacts with its target molecule where the interaction is dependent upon the presence of a particular structure (e.g., the antigenic determinant or epitope on the polypeptide or the nucleotide sequence of the polynucleotide); in other words, the reagent is recognizing and binding to a specific polypeptide or polynucleotide structure rather than to all polypeptides or polynucleotides in general. By "binding fragment thereof' means a fragment or portion of a reagent that specifically binds the target molecule (e.g., an Fab fragment of an antibody).

A reagent that specifically binds to the target molecule may be referred to as a target-specific reagent or an anti-target reagent. For example, an antibody that specifically binds to a FIG-ROS1(L) polypeptide may be referred to as a FIG-ROS1(L)-specific antibody or an anti-FIG-ROS1(L) antibody. Similarly, a nucleic acid probe that specifically binds to a FIG-ROS1(L) polynucleotide may be referred to as a FIG-ROS1(L)-specific nucleic acid probe or an anti-FIG-ROS1(L) nucleic acid probe.

In some embodiments, where the target molecule is a polypeptide, a reagent that specifically binds a target molecule has a binding affinity (K_{D}) for its target molecule of 1× 10⁻⁶ M or less. In some embodiments, a reagent that specifically binds to a target molecule has for its target molecule a K_{D} of 1 ×10⁻⁷ M or less, or a K_{D} of 1 ×10⁻⁸ M or less, or a K_{D} of 1 × 10⁻⁹ M or less, or a K_{D} of 1 × 10⁻¹⁰ M or less, of a K_{D} of 1 × 10⁻¹¹ M or less, of a K_{D} of 1 × 10⁻¹² M or less. In certain embodiments, the K_{D} of a reagent that specifically binds to a target molecule is 1 pM to 500 pM, or between 500 pM to 1 µM, or between 1 µM to 100 nM, or between 100 mM to 10 nM for its target molecule. Non-limiting examples of a target molecule to which a reagent specifically binds to include full length ROS1 polypeptide, the full length ALK polypeptide, one of the many ALK fusion proteins and/or the ROS1 fusion polypeptides described herein, or an EGFR mutant polypeptide.

Where the target molecule is a polynucleotide, a reagent that specifically binds its target molecule is a reagent that hybridizes under stringent conditions to it target polynucleotide. The term "stringent conditions" with respect to nucleotide sequence or nucleotide probe hybridization conditions is the "stringency" that occurs within a range from about Tₘ minus 5 °C (*i*.*e*., 5 °C below the melting temperature (Tₘ) of the reagent or nucleic acid probe) to about 20 °C to 25 °C below Tₘ. Typical stringent conditions are: overnight incubation at 42 °C in a solution comprising: 50% formamide, 5 X SSC (750 mM NaCl, 75 mM trisodium citrate), 50 mM sodium phosphate (pH 7.6), 5X Denhardt's solution, 10% dextran sulfate, and 20 micrograms/ml denatured, sheared salmon sperm DNA, followed by washing the filters in 0.1X SSC at about 65 °C. As will be understood by those of skill in the art, the stringency of hybridization may be altered in order to identify or detect identical or related polynucleotide sequences. By a "reagent (e.g., a polynucleotide or nucleotide probe) that hybridizes under stringent conditions to a target polynucleotide (*e.g*., a full length ROS1 polynucleotide)" is intended that the reagent (e.g., the polynucleotide or nucleotide probe (*e.g.*, DNA, RNA, or a DNA-RNA hybrid)) hybridizes along the entire length of the reference polynucleotide or hybridizes to a portion of the reference polynucleotide that is at least about 15 nucleotides (nt), or to at least about 20 nt, or to at least about 30 nt, or to about 30-70 nt of the reference polynucleotide. These nucleotide probes are useful as diagnostic probes (e.g., for FISH) and primers (*e.g*., for PCR) as discussed herein.

The reagents useful in the practice of the disclosed methods, include, among others, full length ROS1-specific and ROS1 fusion polypeptide-specific antibodies, full length ALK-specific and ALK fusion polypeptide-specific antibodies, EGFR mutant-specific antibodies, and AQUA peptides (heavy-isotope labeled peptides) corresponding to, and suitable for detection and quantification of, the indicated polypeptide's expression in a biological sample. Thus, a "ROS1 polypeptide-specific reagent" is any reagent, biological or chemical, capable of specifically binding to, detecting and/or quantifying the presence/level of expressed ROS1 polypeptide in a biological sample. Likewise, an "ALK polypeptide-specific reagent" is any reagent, biological or chemical, capable of specifically binding to, detecting and/or quantifying the presence/level of expressed ALK polypeptide in a biological sample. An "EGFR mutant polypeptide-specific reagent" is any reagent, biological or chemical, capable of specifically binding to, detecting and/or quantifying the presence/level of expressed EGFR mutant polypeptide in a biological sample. The terms include, but are not limited to, the antibodies and AQUA peptide reagents discussed below, and equivalent binding agents are within the scope of the present disclosure.

The antibodies that specifically bind to full length ROS1 protein, to one of the ROS1 fusion polypeptides, to full length ALK protein, to one of the ALK fusion polypeptides, or to a mutant EGFR polypeptide in cancer may also bind to highly homologous and equivalent epitopic peptide sequences in other mammalian species, for example murine or rabbit, and vice versa. Antibodies useful in practicing the methods disclosed herein include (a) monoclonal antibodies, (b) purified polyclonal antibodies that specifically bind to the target polypeptide (*e.g.*, the fusion junction of the fusion polypeptide, (c) antibodies as described in (a)-(b) above that specifically bind equivalent and highly homologous epitopes or phosphorylation sites in other non-human species (e.g., mouse, rat), and (d) fragments of (a)-(c) above that specifically bind to the antigen (or more preferably the epitope) bound by the exemplary antibodies disclosed herein.

The term "antibody" or "antibodies" refers to all types of immunoglobulins, including IgG, IgM, IgA, IgD, and IgE, including binding fragments thereof (*i.e.*, fragments of an antibody that are capable of specifically binding to the antibody's target molecule, such as F_{ab}, and F(ab')₂ fragments), as well as recombinant, humanized, polyclonal, and monoclonal antibodies and/or binding fragments thereof. Antibodies can be derived from any species of animal, such as from a mammal. Non-limiting exemplary natural antibodies include antibodies derived from human, chicken, goats, and rodents (*e.g*., rats, mice, hamsters and rabbits), including transgenic rodents genetically engineered to produce human antibodies (see, *e.g*., Lonberg et al., WO93/12227; U.S. Pat. No. 5,545,806; and Kucherlapati, et al., WO91/10741; U.S. Pat. No. 6,150,584, which are herein incorporated by reference in their entirety). Antibodies may be also be chimeric antibodies. *See, e.g.,* M. Wroser et al., Molec. Immunol. 26: 403-11 (1989); Morrision et al., Proc. Nat'l. Acad. Sci. 81: 6851 (1984); Neuberger et al., Nature 312: 604 (1984)). The antibodies may be recombinant monoclonal antibodies produced according to the methods disclosed in U.S. Pat. No. 4,474,893 (Reading) or U.S. Pat. No. 4,816,567 (Cabilly et al.). The antibodies may also be chemically constructed specific antibodies made according to the method disclosed in U.S. Pat. No. 4,676,980 (Segel et al.).

Natural antibodies are the antibodies produced by a host animal, however the disclosure also contemplates genetically altered antibodies wherein the amino acid sequence has been varied from that of a native antibody. Because of the relevance of recombinant DNA techniques to this application, one need not be confined to the sequences of amino acids found in natural antibodies; antibodies can be redesigned to obtain desired characteristics. The possible variations are many and range from the changing of just one or a few amino acids to the complete redesign of, for example, the variable or constant region. Changes in the constant region will, in general, be made in order to improve or alter characteristics, such as complement fixation, interaction with membranes and other effector functions. Changes in the variable region will be made in order to improve the antigen binding characteristics. The term "humanized antibody", as used herein, refers to antibody molecules in which amino acids have been replaced in the non-antigen binding regions in order to more closely resemble a human antibody, while still retaining the original binding ability. Other antibodies specifically contemplated are oligoclonal antibodies. As used herein, the phrase "oligoclonal antibodies" refers to a predetermined mixture of distinct monoclonal antibodies. *See, e.g.,* PCT publication WO 95/20401; U.S. Patent Nos. 5,789,208 and 6,335,163. In one embodiment, oligoclonal antibodies consisting of a predetermined mixture of antibodies against one or more epitopes are generated in a single cell. In other embodiments, oligoclonal antibodies include a plurality of heavy chains capable of pairing with a common light chain to generate antibodies with multiple specificities (*e.g*., PCT publication WO 04/009618). Oligoclonal antibodies are particularly useful when it is desired to target multiple epitopes on a single target molecule. In view of the assays and epitopes disclosed herein, those skilled in the art can generate or select antibodies or mixtures of antibodies that are applicable for an intended purpose and desired need.

Recombinant antibodies are also included in the present disclosure. These recombinant antibodies have the same amino acid sequence as the natural antibodies or have altered amino acid sequences of the natural antibodies. They can be made in any expression systems including both prokaryotic and eukaryotic expression systems or using phage display methods (see, *e.g.,* Dower et al., WO91/17271 and McCafferty et al., WO92/01047; U.S. Pat. No. 5,969,108, which are herein incorporated by reference in their entirety). Antibodies can be engineered in numerous ways. They can be made as single-chain antibodies (including small modular immunopharmaceuticals or SMIPs^{™}), Fab and F(ab')₂ fragments, etc. Antibodies can be humanized, chimerized, deimmunized, or fully human. Numerous publications set forth the many types of antibodies and the methods of engineering such antibodies. For example, see U.S. Patent Nos. 6,355,245; 6,180,370; 5,693,762; 6,407,213; 6,548,640; 5,565,332; 5,225,539; 6,103,889; and 5,260,203. The genetically altered antibodies may be functionally equivalent to the above-mentioned natural antibodies. In certain embodiments, modified antibodies can provide improved stability or/and therapeutic efficacy.

Non-limiting examples of modified antibodies include those with conservative substitutions of amino acid residues, and one or more deletions or additions of amino acids that do not significantly deleteriously alter the antigen binding utility. Substitutions can range from changing or modifying one or more amino acid residues to complete redesign of a region as long as the therapeutic utility is maintained. Antibodies can be modified post-translationally *(e.g.,* acetylation, and/or phosphorylation) or can be modified synthetically (e.g., the attachment of a labeling group). Antibodies with engineered or variant constant or Fc regions can be useful in modulating effector functions, such as, for example, antigen-dependent cytotoxicity (ADCC) and complement-dependent cytotoxicity (CDC). Such antibodies with engineered or variant constant or Fc regions may be useful in instances where a parent singling protein is expressed in normal tissue; variant antibodies without effector function in these instances may elicit the desired therapeutic response while not damaging normal tissue. Accordingly, certain aspects and methods of the present disclosure relate to antibodies with altered effector functions that include one or more amino acid substitutions, insertions, and/or deletions. The term "biologically active" refers to a protein having structural, regulatory, or biochemical functions of a naturally occurring molecule. Likewise, "immunologically active" refers to the capability of the natural, recombinant, or synthetic polypeptide (e.g., one of the ROS1 or ALK fusion polypeptides described herein), or any oligopeptide thereof, to induce a specific immune response in appropriate animals or cells and to bind with specific antibodies.

Also within the present disclosure are antibody molecules with fewer than 4 chains, including single chain antibodies, Camelid antibodies and the like and components of an antibody, including a heavy chain or a light chain. In some embodiments an immunoglobulin chain may include in order from 5' to 3', a variable region and a constant region. The variable region may include three complementarity determining regions (CDRs), with interspersed framework (FR) regions for a structure FR1, CDR1, FR2, CDR2, FR3, CDR3 and FR4. Also within the disclosure are heavy or light chain variable regions, framework regions and CDRs. An antibody may include a heavy chain constant region that includes some or all of a CH1 region, hinge, CH2 and CH3 region.

One non-limiting epitopic site of a fusion polypeptide-specific antibody is a peptide fragment consisting essentially of about 11 to 17 amino acids of a fusion polypeptide sequence, which fragment encompasses the fusion junction between the ROS1 or ALK portion of the molecule and the portion of the molecule from the non-ROS1 or -ALK fusion partner. It will be appreciated that antibodies that specifically binding shorter or longer peptides/epitopes encompassing the fusion junction of a ROS1 or ALK fusion polypeptide are within the scope of the present disclosure.

The disclosure is not limited to use of antibodies, but includes equivalent molecules, such as protein binding domains or nucleic acid aptamers, which bind, in a ROS1 or ALK protein-specific or ROS1 or ALK fusion protein-specific or EGFR mutant-specific manner, to essentially the same epitope to which an antibody useful in the disclosed methods binds. *See, e.g.,* Neuberger et al., Nature 312: 604 (1984). Such equivalent non-antibody reagents may be suitably employed in the methods disclosed herein.

Polyclonal antibodies useful in practicing the methods disclosed herein may be produced according to standard techniques by immunizing a suitable animal (*e.g*., rabbit, goat, etc.) with an antigen encompassing a desired fusion-protein or mutant protein specific epitope (e.g. the fusion junction between the non-ROS 1 or ALK protein partner and the ROS1 or ALK protein partner in a ROS1 or ALK fusion polypeptide or a fragment of a mutant EGFR polypeptide that includes one or more mutant residues), collecting immune serum from the animal, and separating the polyclonal antibodies from the immune serum, and purifying polyclonal antibodies having the desired specificity, in accordance with known procedures. The antigen may be a synthetic peptide antigen that includes the desired epitopic sequence, selected and constructed in accordance with well-known techniques. *See, e.g.,* ANTIBODIES: A LABORATORY MANUAL, Chapter 5, p. 75-76, Harlow & Lane Eds., Cold Spring Harbor Laboratory (1988); Czernik, Methods In Enzymology, 201: 264-283 (1991); Merrifield, J. Am. Chem. Soc. 85: 21-49 (1962)). Polyclonal antibodies produced as described herein may be screened and isolated as further described below.

Monoclonal antibodies may also be beneficially employed in the methods disclosed herein, and may be produced in hybridoma cell lines according to the well-known technique of Kohler and Milstein. Nature 265: 495-97 (1975); Kohler and Milstein, Eur. J. Immunol. 6: 511 (1976); *see also*, CURRENT PROTOCOLS IN MOLECULAR BIOLOGY, Ausubel et al. Eds. (Wiley and Sins, New York, NY 1989 and yearly updates up to and including 2010). Monoclonal antibodies so produced are highly specific, and improve the selectivity and specificity of assay methods provided by the present disclosure. For example, a solution containing the appropriate antigen (*e.g.* a synthetic peptide that includes the fusion junction of ROS1 fusion polypeptide) may be injected into a mouse and, after a sufficient time (in keeping with conventional techniques), the mouse sacrificed and spleen cells obtained. The spleen cells are then immortalized by fusing them with myeloma cells, typically in the presence of polyethylene glycol, to produce hybridoma cells. Rabbit fusion hybridomas, for example, may be produced as described in U.S Patent No. 5,675,063. The hybridoma cells are then grown in a suitable selection media, such as hypoxanthine-aminopterin-thymidine (HAT), and the supernatant screened for monoclonal antibodies having the desired specificity, as described below. The secreted antibody may be recovered from tissue culture supernatant by conventional methods such as precipitation, ion exchange or affinity chromatography, or the like.

Monoclonal Fab fragments may also be produced in *Escherichia coli* by recombinant techniques known to those skilled in the art. *See, e.g.,* W. Huse, Science 246: 1275-81 (1989); Mullinax et al., Proc. Nat'l Acad. Sci. 87: 8095 (1990). If monoclonal antibodies of one isotype are desired for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Nat'l. Acad. Sci., 82: 8653 (1985); Spira et al., J. Immunol. Methods, 74: 307 (1984)). The antigen combining site of the monoclonal antibody can be cloned by PCR and single-chain antibodies produced as phage-displayed recombinant antibodies or soluble antibodies *in E. coli (see, e.g.,* ANTIBODY ENGINEERING PROTOCOLS, 1995, Humana Press, Sudhir Paul editor.)

Further still, U.S. Pat. No. 5,194,392, Geysen (1990) describes a general method of detecting or determining the sequence of monomers (amino acids or other compounds) which is a topological equivalent of the epitope *(i.e.,* a "mimotope") which is complementary to a particular paratope (antigen binding site) of an antibody of interest. More generally, this method involves detecting or determining a sequence of monomers which is a topographical equivalent of a ligand which is complementary to the ligand binding site of a particular receptor of interest. Similarly, U.S. Pat. No. 5,480,971, Houghten et al. (1996) discloses linear C₁-C-rosyl perrosylated oligopeptides and sets and libraries of such peptides, as well as methods for using such oligopeptide sets and libraries for determining the sequence of a perrosylated oligopeptide that preferentially binds to an acceptor molecule of interest. Thus, non-peptide analogs of the epitope-bearing peptides also can be made routinely by these methods.

Antibodies useful in the methods disclosed herein, whether polyclonal or monoclonal, may be screened for epitope and fusion protein specificity according to standard techniques. *See*, *e.g*., Czernik et al., Methods in Enzymology, 201: 264-283 (1991). For example, the antibodies may be screened against a peptide library by ELISA to ensure specificity for both the desired antigen and, if desired, for reactivity only with the full-length ROS1 or ALK protein, a particular ROS1 or ALK fusion polypeptide (e.g., an SLC34A2-ROS1(S) polypeptide), a particular EGFR mutant polypeptide, or fragments thereof. The antibodies may also be tested by western blotting against cell preparations containing target protein to confirm reactivity with the only the desired target and to ensure no appreciable binding to other proteins. The production, screening, and use of fusion protein-specific antibodies are known to those of skill in the art, and have been described. *See, e.g.,* U.S. Patent Publication No. 20050214301.

Antibodies useful in the methods disclosed herein may exhibit some limited cross-reactivity with similar epitopes in other proteins or polypeptides, such as similar fusion polypeptides. This is not unexpected as most antibodies exhibit some degree of cross-reactivity, and anti-peptide antibodies will often cross-react with epitopes having high homology or identity to the immunizing peptide. *See*, *e.g., Czernik, supra.* Cross-reactivity with other fusion proteins is readily characterized by western blotting alongside markers of known molecular weight. Undesirable cross-reactivity can be removed by negative selection using antibody purification on peptide columns.

ROS1-specific antibodies and ROS1 fusion polypeptide-specific antibodies that are useful in practicing the methods disclosed herein are ideally specific for human fusion polypeptide, but are not limited only to binding the human species, *per se.* The disclosure includes the production and use of antibodies that also bind conserved and highly homologous or identical epitopes in other mammalian species (*e.g.*, mouse, rat, monkey). Highly homologous or identical sequences in other species can readily be identified by standard sequence comparisons, such as using BLAST, with the human ROS1 protein sequence (SEQ ID NO: 1), and the human ROS1 fusion polypeptide sequences disclosed herein (SEQ ID NOs: 5, 7, 11, 13, 22, 24, and 26).

ALK-specific antibodies and ALK fusion polypeptide-specific antibodies that are useful in practicing the methods disclosed herein are ideally specific for human fusion polypeptide, but are not limited only to binding the human species, *per se.* The disclosure includes the production and use of antibodies that also bind conserved and highly homologous or identical epitopes in other mammalian species (*e.g.*, mouse, rat, monkey). Highly homologous or identical sequences in other species can readily be identified by standard sequence comparisons, such as using BLAST, with the human ALK protein sequence (SEQ ID NO: 35), and the human ALK fusion polypeptide sequences previously described.

EGFR mutant polypeptide-specific antibodies of the disclosure that are useful in practicing the methods disclosed herein are ideally specific for human fusion polypeptide, but are not limited only to binding the human species, *per se.* The disclosure includes the production and use of antibodies that also bind conserved and highly homologous or identical epitopes in other mammalian species (*e.g*., mouse, rat, monkey). Highly homologous or identical sequences in other species can readily be identified by standard sequence comparisons, such as using BLAST, with the human EGFR protein sequence (SEQ ID NO: 3), and the human EGFR mutant polypeptide sequences previously described.

Antibodies employed in the methods disclosed herein may be further characterized by, and validated for, use in a particular assay format, for example FC, IHC, and/or ICC. The use of full-length ROS1 protein-specific and/or ROS1 fusion polypeptide-specific antibodies and/or EGFR mutant polypeptide-specific antibodies in such methods is further described herein. The antibodies described herein, used alone or in the below-described assays, may also be advantageously conjugated to fluorescent dyes (e.g. ALEXA FLUOR 488, phycoerythrin), or labels such as quantum dots, for use in multi-parametric analyses along with other signal transduction (phospho-AKT, phospho-Erk 1/2) and/or cell marker (cytokeratin) antibodies, as further described below.

In practicing the methods disclosed herein, the expression and/or activity of a ROS1 fusion polypeptide and/or of full-length ROS1 in a given biological sample may also be advantageously examined using antibodies specific for (i.e., that specifically bind to) full length ROS1 protein or antibodies specific for ROS1 fusion polypeptides. For example, ROS1-specific antibodies (i.e., antibodies that specifically bind full-length ROS1) are commercially available (see Santa Cruz Biotech., Inc. (Santa Cruz, CA) Catalog No. sc-6347; Cell Signaling Technology, Inc. (Danvers, MA), Catalog Nos. 3078, 3266, and 3287); and Abcam (Cambridge, MA), Catalog Nos. ab5512 and ab108492, for example). In some embodiments, ROS1-specific antibodies used in the methods disclosed herein specifically bind the kinase domain of ROS1 and, thus, will detect full-length ROS1 and all of the ROS1 fusion polypeptides described herein. In some embodiments, ROS1-specific antibodies used in the methods disclosed herein specifically bind a region on the ROS1 protein that is C-terminal to the kinase domain of ROS1 and, thus, will detect full-length ROS1 and all of the ROS1 fusion polypeptides described herein. Such antibodies may also be produced according to standard methods.

Likewise, the expression and/or activity of an ALK fusion polypeptide and/or of full-length ALK in a given biological sample may also be advantageously examined using antibodies specific for (i.e., that specifically bind to) full length ALK protein or antibodies specific for ALK fusion polypeptides. For example, ALK-specific antibodies (i.e., antibodies that specifically bind full-length ALK) are commercially available (see CELL SIGNALING TECHNOLOGY, INC., Danvers, MA, Catalog Nos. 3333, 3633, and 3791; Abcam, 2010 Catalogue, #ab17127, ab59286, and Sigma-Aldrich, 2010 Catalog, #HPA010694, for example). In some embodiments, ALK-specific antibodies used in the methods disclosed herein specifically bind the kinase domain of ALK and, thus, will detect full-length ALK and the ALK fusion polypeptides described herein. Furthermore, ALK antibodies specific for phosphorylated ALK or ALK C-terminal regions (e.g., in ALK fusion proteins) are commercially available (see CELL SIGNALING TECHNOLOGY, INC., Beverly MA, Catalog #'s 3343S (phospho-ALK), 3983 (phospho-ALK), Abcam, 2010 Catalogue, #ab4061 (C-terminal ALK), and Thermo Scientific, 2010 Catalogue, #PA1-37060 (C-terminal ALK), for example). Such antibodies may also be produced according to standard methods, as described above.

Detection of expression and/or activity of full-length ROS1 and/or a ROS1 fusion polypeptide expression or a mutant EGFR polypeptide expression, in a biological sample *(e.g.* a tumor sample) can provide information on whether the kinase protein alone is driving the tumor, or whether aberrantly expressed full length ROS1 or mutant EGFR is also present and driving the tumor. Such information is clinically useful in assessing whether targeting the fusion protein or the full-length protein(s), or both, or is likely to be most beneficial in inhibiting progression of the tumor, and in selecting an appropriate therapeutic or combination thereof.

In some embodiments, a reagent that can be used to detect full length ROS1 or a ROS 1 fusion polypeptide, full length ALK or an ALK fusion polypeptide, or a mutant EGFR polypeptide is a heavy-isotope labeled peptide *(i.e.,* an AQUA peptide) that, for example, corresponds to a peptide sequence that includes the fusion junction of a ROS1 or an ALK fusion polypeptide or a mutant-specific sequence of a mutant EGFR polypeptide. Such an AQUA peptide may be suitable for the absolute quantification of an expressed ROS1 or ALK fusion polypeptide or mutant EGFR polypeptide in a biological sample. As used herein, the term "heavy-isotope labeled peptide" is used interchangeably with "AQUA peptide". The production and use of AQUA peptides for the absolute quantification or detection of proteins (AQUA) in complex mixtures has been described. *See* WO/03016861, "Absolute Quantification of Proteins and Modified Forms Thereof by Multistage Mass Spectrometry," Gygi et al. and also Gerber et al., Proc. Natl. Acad. Sci. U.S.A. 100: 6940-45 (2003) (the teachings of which are hereby incorporated herein by reference, in their entirety). The term "specifically detects" with respect to such an AQUA peptide means the peptide will only detect and quantify polypeptides and proteins that contain the AQUA peptide sequence and will not substantially detect polypeptides and proteins that do not contain the AQUA peptide sequence.

The AQUA methodology employs the introduction of a known quantity of at least one heavy-isotope labeled peptide standard (which has a unique signature detectable by LC-SRM chromatography) into a digested biological sample in order to determine, by comparison to the peptide standard, the absolute quantity of a peptide with the same sequence and protein modification in the biological sample. Briefly, the AQUA methodology has two stages: peptide internal standard selection and validation and method development; and implementation using validated peptide internal standards to detect and quantify a target protein in sample. The method is a powerful technique for detecting and quantifying a given peptide/protein within a complex biological mixture, such as a cell lysate, and may be employed, *e.g.*, to quantify change in protein phosphorylation as a result of drug treatment, or to quantify differences in the level of a protein in different biological states.

Generally, to develop a suitable internal standard, a particular peptide (or modified peptide) within a target protein sequence is chosen based on its amino acid sequence and the particular protease to be used to digest. The peptide is then generated by solid-phase peptide synthesis such that one residue is replaced with that same residue containing stable isotopes (¹³C, ¹⁵N). The result is a peptide that is chemically identical to its native counterpart formed by proteolysis, but is easily distinguishable by MS via a 7-Da mass shift. The newly synthesized AQUA internal standard peptide is then evaluated by LC-MS/MS. This process provides qualitative information about peptide retention by reverse-phase chromatography, ionization efficiency, and fragmentation via collision-induced dissociation. Informative and abundant fragment ions for sets of native and internal standard peptides are chosen and then specifically monitored in rapid succession as a function of chromatographic retention to form a selected reaction monitoring (LC-SRM) method based on the unique profile of the peptide standard.

The second stage of the AQUA strategy is its implementation to measure the amount of a protein or modified protein from complex mixtures. Whole cell lysates are typically fractionated by SDS-PAGE gel electrophoresis, and regions of the gel consistent with protein migration are excised. This process is followed by in-gel proteolysis in the presence of the AQUA peptides and LC-SRM analysis. (*See* Gerber *et al., supra*.) AQUA peptides are spiked in to the complex peptide mixture obtained by digestion of the whole cell lysate with a proteolytic enzyme and subjected to immunoaffinity purification as described above. The retention time and fragmentation pattern of the native peptide formed by digestion (*e.g*., trypsinization) is identical to that of the AQUA internal standard peptide determined previously; thus, LC-MS/MS analysis using an SRM experiment results in the highly specific and sensitive measurement of both internal standard and analyte directly from extremely complex peptide mixtures.

Since an absolute amount of the AQUA peptide is added (*e.g.,* 250 fmol), the ratio of the areas under the curve can be used to determine the precise expression levels of a protein or phosphorylated form of a protein in the original cell lysate. In addition, the internal standard is present during in-gel digestion as native peptides are formed, such that peptide extraction efficiency from gel pieces, absolute losses during sample handling (including vacuum centrifugation), and variability during introduction into the LC-MS system do not affect the determined ratio of native and AQUA peptide abundances.

An AQUA peptide standard is developed for a known sequence previously identified by the IAP-LC-MS/MS method within in a target protein. If the site is modified, one AQUA peptide incorporating the modified form of the particular residue within the site may be developed, and a second AQUA peptide incorporating the unmodified form of the residue developed. In this way, the two standards may be used to detect and quantify both the modified an unmodified forms of the site in a biological sample.

Peptide internal standards may also be generated by examining the primary amino acid sequence of a protein and determining the boundaries of peptides produced by protease cleavage. Alternatively, a protein may actually be digested with a protease and a particular peptide fragment produced can then sequenced. Suitable proteases include, but are not limited to, serine proteases *(e.g.* trypsin, hepsin), metallo proteases *(e.g.,* PUMP1), chymotrypsin, cathepsin, pepsin, thermolysin, carboxypeptidases, etc.

A peptide sequence within a target protein is selected according to one or more criteria to optimize the use of the peptide as an internal standard. Preferably, the size of the peptide is selected to minimize the chances that the peptide sequence will be repeated elsewhere in other non-target proteins. Thus, a peptide is preferably at least about 6 amino acids. The size of the peptide is also optimized to maximize ionization frequency. Thus, in some embodiments, the peptide is not longer than about 20 amino acids. In some embodiments, the peptide is between about 7 to 15 amino acids in length. A peptide sequence is also selected that is not likely to be chemically reactive during mass spectrometry, thus sequences that contain cysteine, tryptophan, or methionine are avoided.

A peptide sequence that does not include a modified region of the target region may be selected so that the peptide internal standard can be used to determine the quantity of all forms of the protein. Alternatively, a peptide internal standard encompassing a modified amino acid may be desirable to detect and quantify only the modified form of the target protein. Peptide standards for both modified and unmodified regions can be used together, to determine the extent of a modification in a particular sample (*i.e.* to determine what fraction of the total amount of protein is represented by the modified form). For example, peptide standards for both the phosphorylated and unphosphorylated form of a protein known to be phosphorylated at a particular site can be used to quantify the amount of phosphorylated form in a sample.

The peptide is labeled using one or more labeled amino acids *(i.e.,* the label is an actual part of the peptide) or less preferably, labels may be attached after synthesis according to standard methods. Preferably, the label is a mass-altering label selected based on the following considerations: The mass should be unique to shift fragments masses produced by MS analysis to regions of the spectrum with low background; the ion mass signature component is the portion of the labeling moiety that preferably exhibits a unique ion mass signature in MS analysis; the sum of the masses of the constituent atoms of the label is preferably uniquely different than the fragments of all the possible amino acids. As a result, the labeled amino acids and peptides are readily distinguished from unlabeled ones by the ion/mass pattern in the resulting mass spectrum. Preferably, the ion mass signature component imparts a mass to a protein fragment that does not match the residue mass for any of the 20 natural amino acids.

The label should be robust under the fragmentation conditions of MS and not undergo unfavorable fragmentation. Labeling chemistry should be efficient under a range of conditions, particularly denaturing conditions, and the labeled tag preferably remains soluble in the MS buffer system of choice. The label preferably does not suppress the ionization efficiency of the protein and is not chemically reactive. The label may contain a mixture of two or more isotopically distinct species to generate a unique mass spectrometric pattern at each labeled fragment position. Stable isotopes, such as ²H , ¹³C, ¹⁵N, ¹⁷O, ¹⁸O, or ³⁴S, are some non-limiting labels. Pairs of peptide internal standards that incorporate a different isotope label may also be prepared. Non-limiting amino acid residues into which a heavy isotope label may be incorporated include leucine, proline, valine, and phenylalanine.

Peptide internal standards are characterized according to their mass-to-charge (m/z) ratio, and preferably, also according to their retention time on a chromatographic column (e.g., an HPLC column). Internal standards that co-elute with unlabeled peptides of identical sequence are selected as optimal internal standards. The internal standard is then analyzed by fragmenting the peptide by any suitable means, for example by collision-induced dissociation (CID) using, e.g., argon or helium as a collision gas. The fragments are then analyzed, for example by multi-stage mass spectrometry (MSⁿ) to obtain a fragment ion spectrum, to obtain a peptide fragmentation signature. Preferably, peptide fragments have significant differences in m/z ratios to enable peaks corresponding to each fragment to be well separated, and a signature is that is unique for the target peptide is obtained. If a suitable fragment signature is not obtained at the first stage, additional stages of MS are performed until a unique signature is obtained.

Fragment ions in the MS/MS and MS³ spectra are typically highly specific for the peptide of interest, and, in conjunction with LC methods, allow a highly selective means of detecting and quantifying a target peptide/protein in a complex protein mixture, such as a cell lysate, containing many thousands or tens of thousands of proteins. Any biological sample potentially containing a target protein/peptide of interest may be assayed. Crude or partially purified cell extracts are preferably employed. Generally, the sample has at least 0.01 mg of protein, typically a concentration of 0.1-10 mg/mL, and may be adjusted to a desired buffer concentration and pH.

A known amount of a labeled peptide internal standard, preferably about 10 femtomoles, corresponding to a target protein to be detected/quantified is then added to a biological sample, such as a cell lysate. The spiked sample is then digested with one or more protease(s) for a suitable time period to allow digestion. A separation is then performed (e.g. by HPLC, reverse-phase HPLC, capillary electrophoresis, ion exchange chromatography, etc.) to isolate the labeled internal standard and its corresponding target peptide from other peptides in the sample. Microcapillary LC is a one non-limiting method.

Each isolated peptide is then examined by monitoring of a selected reaction in the MS. This involves using the prior knowledge gained by the characterization of the peptide internal standard and then requiring the MS to continuously monitor a specific ion in the MS/MS or MSⁿ spectrum for both the peptide of interest and the internal standard. After elution, the area under the curve (AUC) for both peptide standard and target peptide peaks are calculated. The ratio of the two areas provides the absolute quantification that can be normalized for the number of cells used in the analysis and the protein's molecular weight, to provide the precise number of copies of the protein per cell. Further details of the AQUA methodology are described in Gygi *et al*., and Gerber *et al. supra.*

AQUA internal peptide standards (heavy-isotope labeled peptides) may desirably be produced, as described above, to detect any quantify any unique site *(e.g.,* the fusion junction within a ROS1 or ALK fusion polypeptide or a mutant-specific sequence within a mutant EGFR polypeptide) within a polypeptide disclosed herein. For example, an AQUA phosphopeptide may be prepared that corresponds to the fusion junction sequence of one of the ROS1 or ALK fusion polypeptides. Peptide standards for may be produced for the fusion junction and such standards employed in the AQUA methodology to detect and quantify the fusion junction (*i*.*e*. the presence of that fusion polypeptide) in a biological sample.

For example, one non-limiting AQUA peptide includes the amino acid sequence *AGS***TLP** (SEQ ID NO: 32), which corresponds to the three amino acids immediately flanking each side of the fusion junction in the short variant of FIG-ROS1 fusion polypeptide (*i.e.,* FIG-ROS1(S) fusion polypeptide), where the amino acids encoded by the FIG gene are italicized and the amino acids encoded by the ROS1 gene in bold. It will be appreciated that larger AQUA peptides including the fusion junction sequence (and additional residues downstream or upstream of it) may also be constructed. Similarly, a smaller AQUA peptide including less than all of the residues of such sequence (but still including the point of fusion junction itself) may alternatively be constructed. Such larger or shorter AQUA peptides are within the scope of the present disclosure, and the selection and production of AQUA peptides may be carried out as described above (*see* Gygi *et al*., Gerber *et al*., *supra*.).

It should be noted that because the sequence of the AQUA peptide spanning the fusion junction of one of the ROS1 fusion proteins described herein may also be (or be included in) the epitope to which a ROS1 fusion-specific antibody specifically binds. An "epitope" refers to either an immunogenic epitope *(i.e.,* capable of eliciting an immune response) or an antigenic epitope (*i.e.,* the region of a protein molecule to which an antibody can specifically bind. The number of immunogenic epitopes of a protein generally is less than the number of antigenic epitopes. *See,* for instance, Geysen et al., Proc. Natl. Acad. Sci. USA 81:3998-4002 (1983).

Table 3 provides a list of the sequences of all the fusion junctions of exemplary ROS1 fusion polypeptides, where the amino acids encoded by the non-ROS1 gene are italicized and the amino acids encoded by the ROS1 gene in bold.

**Table 3**

| **Fusion** | **Junction Sequence** | **SEQ ID NO:** |
|---|---|---|
| SLC34A2-ROS1 (very short) | *VGV***WHR** | 28 |
| SLC34A2-ROS1 (short) | *LVG**DDF*** | 29 |
| SLC34A2-ROS1 (long) | *LVG***AGV** | 30 |
| CD74-ROS1 | *PPK***DDF** | 31 |
| FIG-ROS1 (short) | *AGS***TLP** | 32 |
| FIG-ROS1 (long) | *LQV***WHR** | 33 |
| FIG-ROS1 (Extra Long) | *VLQ**AGV*** | 34 |

A longer depiction of the sequence of CD74-ROS1 including the fusion junction is provided in SEQ ID NO: 15.

In some embodiments, the mammalian cancer is from a human. In various embodiments, the biological sample is from the cancer or suspected cancer of the patient. In some embodiments, the cancer is a solid tumor cancer. In some embodiments, the cancer is leukemia. In some embodiments, the cancer is lymphoma. In some embodiments, the cancer is a lung cancer (e.g., a non-small cell lung carcinoma or a small cell lung carcinoma). In some embodiments, the cancer is a brain cancer (e.g., glioblastoma). In some embodiments, the cancer is a liver cancer (e.g., cholangiocarcinoma). In some embodiments, the cancer is colon cancer. In some embodiments, the cancer is breast cancer. In some embodiments, the cancer is ovarian cancer.

In some embodiments, the mammalian lung cancer is NSCLC (non-small cell lung carcinoma). In some embodiments, the mammalian lung cancer is SCLC (small cell lung carcinoma). In further embodiments of the methods disclosed herein, the mammal is a human, and the human may be a candidate for a ROS1-inhibiting therapeutic, an EGFR-inhibiting therapeutic, or both, for the treatment of a lung cancer. The human candidate may be a patient currently being treated with, or considered for treatment with, a ROS1 kinase inhibitor or EGFR kinase inhibitor. In another embodiment, the mammal is large animal, such as a horse or cow, while in other embodiments, the mammal is a small animal, such as a dog or cat, all of which are known to develop lung cancers, such as NSCLC and SCLC.

As used throughout the specification, the term "biological sample" is used in its broadest sense, and means any biological sample suspected of containing a polypeptide with ROS1 kinase activity, a polypeptide with ALK kinase activity, or a mutant EGFR polypeptide including, without limitation, a ROS1 or ALK fusion polypeptide or a full length ROS1 or ALK protein (with or without the signal peptide sequence) or fragments having ROS1 or ALK kinase activity thereof. Biological samples include, without limitation, saliva, mucous, tears, blood, circulating tumor cells, serum, tissues, bone marrow, lymph/interstitial fluids, buccal cells, mucosal cells, cerebrospinal fluid, semen, feces, plasma, urine, a suspension of cells, or a suspension of cells and viruses or extracts thereof, and may include a cell, chromosomes isolated from a cell *(e.g.,* a spread of metaphase chromosomes), genomic DNA (in solution or bound to a solid support such as for Southern analysis), RNA (in solution or bound to a solid support such as for northern analysis), cDNA (in solution or bound to a solid support). In some embodiments, the biological sample contains lung cells suspected of being cancerous.

The methods disclosed herein can include detection of two or more analytes (e.g., a polypeptide with ROS1 kinase activity, a polypeptide with ALK kinase activity, and a mutant EGFR polypeptide (and nucleic acids encoding the same)) in the same biological sample. In these methods, a biological sample may be divided into one or more fractions (e.g., portions of a liquid sample or sections of a tissue sample) prior to detection and each analyte detected in a separate fraction. The methods do not require detection of the analytes in the same fraction of a sample or in the same cell within a sample. In other embodiments, the methods disclosed herein can be used to detect two or more analytes (e.g., a polypeptide with ROS1 kinase activity, a polypeptide with ALK kinase activity, and a mutant EGFR polypeptide (and nucleic acids encoding the same)) in separate biological samples from the same subject. For example, two separately obtained samples from the same tumor or the same organ or tissue of a patient may be assayed independently for each of the two or more analytes. The separately obtained samples can be obtained at approximately the same time or at different times (e.g., within days, weeks, months, or years of each other).

Any biological sample that includes cells (or extracts of cells) from a mammalian cancer is suitable for use in the methods disclosed herein. In one embodiment, the biological sample includes cells obtained from a tumor biopsy or a tumor resection. The biopsy or resection may be obtained, according to standard clinical techniques, from primary tumors occurring in an organ of a mammal, or by secondary tumors that have metastasized in other tissues. In some instances, the biopsy or resection is frozen or fixed with formalin and embedded in paraffin. Frozen or fixed samples may be sectioned for further analysis.

In another embodiment, the biological sample includes cells obtained from a fine needle aspirate taken from a tumor, and techniques for obtaining such aspirates are well known in the art (*see* Cristallini et al., Acta Cytol. 36(3): 416-22 (1992)). In certain embodiments, the biological sample includes a bronchial scraping.

The biological sample may also include cells obtained from an effusion, such as a pleural effusion. Pleural effusions (liquid that forms outside the lung in the thoracic cavity and which contains cancerous cells) are known to form in many patients with advanced lung cancer (including NSCLC), and the presence of such effusion is predictive of a poor outcome and short survival time. Standard techniques for obtaining pleural effusion samples have been described and are well known in the art (*see* Sahn, Clin Chest Med. 3(2): 443-52 (1982)).

The biological sample may include cells obtained from a bronchoalveolar lavage. Bronchoalveolar lavage is a standard medical procedure in which a bronchoscope is passed through the mouth or nose into the lungs and fluid is squirted into a small part of the lung and then recollected for examination.

In some embodiments, the biological sample includes circulating tumor cells. Circulating tumor cells ("CTCs") may be purified, for example, using the kits and reagents sold under the trademarks Vita-Assays^{™}, Vita-Cap^{™}, and CellSearch^{®} (commercially available from Vitatex, LLC (a Johnson and Johnson corporation). Other methods for isolating CTCs are described (see, for example, PCT Publication No. WO/2002/020825, Cristofanilli et al., New Engl. J. of Med. 351 (8):781-791 (2004), and Adams et al., J. Amer. Chem. Soc. 130(27): 8633-8641 (July 2008)). In a particular embodiment, a circulating tumor cell ("CTC") may be isolated and identified as having originated from the lung.

Accordingly, the disclosure provides a method for isolating a CTC, and then screening the CTC one or more assay formats to identify the presence of a polypeptide with ROS1 kinase activity, a mutant EGFR polypeptide, or a nucleic acid molecule encoding either of the same in the CTC.

Cellular extracts of the biological samples described herein may be prepared, either crude or partially (or entirely) purified, in accordance with standard techniques, and used in the methods disclosed herein. Alternatively, biological samples including whole cells may be utilized in assay formats such as in vitro kinase assay, ELISA assays, immunohistochemistry (IHC), flow cytometry (FC), and immunofluorescence (IF), immunohistochemistry (IHC), fluorescence *in situ* hybridization (FISH) and polymerase chain reaction (PCR), according to standard methods such as those described below (see, also, e.g., Ausubel et al., supra). Such whole-cell assays are advantageous in that they minimize manipulation of the tumor cell sample and thus reduce the risks of altering the *in vivo* signaling/activation state of the cells and/or introducing artifact signals. Whole cell assays are also advantageous because they characterize expression and signaling only in tumor cells, rather than a mixture of tumor and normal cells.

Thus, biological samples useful in the practice of the methods disclosed herein may be obtained from any mammal in which a cancer or suspected cancer characterized by the presence of a polypeptide having ROS1 kinase activity, a polypeptide having ALK kinase activity, or a mutant EGFR polypeptide is present or might be present or developing. As used herein, the phrase "characterized by" with respect to a cancer (or suspected cancer) and indicated molecule *(e.g.,* a polypeptide with ROS1 kinase activity or a polypeptide with ALK kinase activity) is meant a cancer (or suspected cancer) in which a gene translocation or mutation and/or an expressed polypeptide is present, as compared to another cancer or a normal tissue in which such translocation or aberrant expression is not present. The presence of such mutation or aberrant expression may drive *(i.e.,* stimulate or be the causative agent of), in whole or in part, the growth and survival of such cancer or suspected cancer.

Accordingly, any biological sample (e.g., CTC, pleural effusion, needle aspirate, tumor biopsy) from a patient that is identified as having a polypeptide with ROS1 kinase activity, a mutant EGFR polypeptide, or polynucleotide encoding either of the same (e.g., a full length ROS1 polypeptide or polynucleotide or a ROS1 fusion polypeptide or polynucleotide) may indicate that the patient's originating cancer (e.g., an lung cancer such as NSCLC or SCLC) is being driven by the polypeptide with ROS1 kinase activity and/or the mutant EGFR polypeptide and thus is likely to respond to a treatment regimen that includes one or both of a ROS1 kinase-inhibiting therapeutic and an EGFR kinase-inhibiting therapeutic.

As used herein, by "likely to respond" is meant that a cancer is more likely to show growth retardation or abrogation in response to *(e.g.,* upon contact with or treatment by) a ROS1 inhibiting therapeutic and/or EGFR inhibiting therapeutic. In some embodiments, a cancer that is likely to respond to a ROS1 inhibiting therapeutic and/or EGFR inhibiting therapeutic is one that dies *(e.g.,* the cancer cells apoptose) in response to the ROS1 inhibiting therapeutic and/or EGFR inhibiting therapeutic.

In assessing the presence of a polypeptide with ROS1 kinase activity, a polypeptide with ALK kinase activity, or a mutant EGFR polypeptide (or polynucleotides encoding the same) in a biological sample that includes cells from a mammalian cancer tumor, a control sample representing a cell in which such a polypeptide does not occur (e.g., healthy lung cells) may desirably be employed for comparative purposes. Ideally, the control sample includes cells from a subset of the particular cancer (e.g., lung cancer) that is representative of the subset in which the polypeptide (or polynucleotide encoding the same) does not occur. Comparing the level in the control sample versus the test biological sample thus identifies whether the mutant polynucleotide and/or polypeptide is/are present. Alternatively, since a polypeptide with ROS1 kinase activity, a polypeptide with ALK kinase activity, or a mutant EGFR polypeptide (or polynucleotides encoding the same) may not be present in the majority of cancers, any tissue that similarly does not express a polypeptide with ROS1 kinase activity, a polypeptide with ALK kinase activity, or a mutant EGFR polypeptide (or polynucleotides encoding the same) may be employed as a control.

The methods described herein have valuable diagnostic utility for cancers characterized by the presence of a polypeptide with ROS1 kinase activity, a polypeptide with ALK kinase activity, or a mutant EGFR polypeptide, and treatment decisions pertaining to the same. For example, biological samples may be obtained from a subject that has not been previously diagnosed as having a cancer characterized by the presence of polypeptide with ROS1 kinase activity and/or a mutant EGFR polypeptide, nor has yet undergone treatment for such cancer, and the method is employed to diagnostically identify a tumor in such subject as belonging to a subset of tumors (e.g., NSCLC or SCLC) in which a polypeptide with ROS1 kinase activity and/or a mutant EGFR polypeptide (or polynucleotide encoding the same) is present/expressed.

Alternatively, a biological sample may be obtained from a subject that has been diagnosed as having a cancer characterized by the presence of one type of kinase, such as EGFR, and has been receiving therapy, such as EGFR inhibitor therapy (e.g., erlotinib, gefitinib) for treatment of such cancer, and a method disclosed herein is employed to identify whether the subject's tumor is also characterized by the presence of polypeptide with ROS1 kinase activity (or polynucleotide encoding the same) such as full length ROS1 protein or one of the many ROS1 fusion polypeptides (e.g., SLC34A2-ROS1(S)), and is therefore likely to fully respond to the existing therapy and/or whether alternative or additional ROS1-inhibiting therapy is desirable or warranted. The methods of disclosed herein may also be employed to monitor the progression or inhibition of a polypeptide with ROS1 kinase activity-expressing cancer following treatment of a subject with a composition that includes a ROS1-inhibiting therapeutic or combination of therapeutics.

Such diagnostic assay may be carried out subsequent to or prior to preliminary evaluation or surgical surveillance procedures. The identification methods disclosed herein may be advantageously employed as a diagnostic to identify patients having cancer, such as lung cancer (e.g., non-small cell lung cancer) or colon cancer, characterized by the presence of a polypeptide with ROS1 kinase activity or ALK kinase activity, or a mutant EGFR polypeptide, which patients would be most likely to respond to therapeutics targeted at inhibiting ROS1 or ALK kinase activity or EGFR kinase activity. The ability to select such patients would also be useful in the clinical evaluation of efficacy of future ROS1-, ALK-, and/or EGFR-inhibiting therapeutics as well as in the future prescription of such drugs to patients.

The ability to selectively identify cancers in which a polypeptide with ROS1 kinase activity (or polynucleotide encoding the same) or a polypeptide with ALK kinase activity (or polynucleotide encoding the same) or a mutant EGFR polypeptide (or polynucleotide encoding the same) is/are present enables important new methods for accurately identifying such tumors for diagnostic purposes, as well as obtaining information useful in determining whether such a tumor is likely to respond to a ROS1-, ALK-, and/or EGFR-inhibiting therapeutic composition, or likely to be partially or wholly non-responsive to an inhibitor targeting a different kinase when administered as a single agent for the treatment of the cancer.

As used herein, by "cancer" or "cancerous" is meant a cell that shows abnormal growth as compared to a normal *(i.e.,* non-cancerous) cell of the same cell type. For example, a cancerous cell may be metastatic or non-metastatic. A cancerous cell may also show lack of contact inhibition where a normal cell of that same cell type shows contact inhibition. In some embodiments, the cancer is lung cancer (e.g., non-small cell lung cancer or small cell lung cancer). As used herein, by "suspected cancer" (as in "suspected mammalian lung cancer") or "tissue suspected of being cancerous" is meant a cell or tissue that has some aberrant characteristics (e.g., hyperplastic or lack of contact inhibition) as compared to normal cells or tissues of that same cell or tissue type as the suspected cancer, but where the cell or tissue is not yet confirmed by a physician or pathologist as being cancerous.

In some embodiments, the various methods disclosed herein may be carried out in a variety of different assay formats known to those of skill in the art. Some non-limiting examples of methods include immunoassays and peptide and nucleotide assays.

### Immunoassays.

Immunoassays useful in the practice of the methods disclosed herein may be homogenous immunoassays or heterogeneous immunoassays. In a homogeneous assay the immunological reaction usually involves a specific reagent *(e.g.* a ROS1-specific antibody, an ALK-specific antibody, or a mutant EGFR-specific antibody), a labeled analyte, and the biological sample of interest. The signal arising from the label is modified, directly or indirectly, upon the binding of the antibody to the labeled analyte. Both the immunological reaction and detection of the extent thereof are carried out in a homogeneous solution. Immunochemical labels that may be employed include free radicals, radio-isotopes, fluorescent dyes, enzymes, bacteriophages, coenzymes, and so forth. Semi-conductor nanocrystal labels, or "quantum dots", may also be advantageously employed, and their preparation and use has been well described. *See generally,* K. Barovsky, Nanotech. Law & Bus. 1(2): Article 14 (2004) and patents cited therein.

In a heterogeneous assay approach, the materials are usually the biological sample, binding reagent (e.g., an antibody), and suitable means for producing a detectable signal. Biological samples as further described below may be used. The antibody is generally immobilized on a support, such as a bead, plate or slide, and contacted with the sample suspected of containing the antigen in a liquid phase. The support is then separated from the liquid phase and either the support phase or the liquid phase is examined for a detectable signal employing means for producing such signal. The signal is related to the presence of the analyte in the biological sample. Means for producing a detectable signal include the use of radioactive labels, fluorescent labels, enzyme labels, quantum dots, and so forth. For example, if the antigen to be detected contains a second binding site, an antibody which binds to that site can be conjugated to a detectable group and added to the liquid phase reaction solution before the separation step. The presence of the detectable group on the solid support indicates the presence of the antigen in the test sample. Examples of suitable immunoassays are the radioimmunoassay, immunofluorescence methods, enzyme-linked immunoassays, and the like.

Immunoassay formats and variations thereof, which may be useful for carrying out the methods disclosed herein, are well known in the art. *See generally* E. Maggio, Enzyme-Immunoassay, (1980) (CRC Press, Inc., Boca Raton, Fla.); *see also, e.g.,* U.S. Pat. No. 4,727,022 (Skold *et al.,* "Methods for Modulating Ligand-Receptor Interactions and their Application"); U.S. Pat. No. 4,659,678 (Forrest et al., "Immunoassay of Antigens"); U.S. Pat. No. 4,376,110 (David et al., "Immunometric Assays Using Monoclonal Antibodies"). Conditions suitable for the formation of reagent-antibody complexes are well known to those of skill in the art. *See id.* ROS1-specific antibodies may be used in a "two-site" or "sandwich" assay, with a single hybridoma cell line serving as a source for both the labeled monoclonal antibody and the bound monoclonal antibody. Such assays are described in U.S. Pat. No. 4,376,110. The concentration of detectable reagent should be sufficient such that the binding of the antigen of interest is detectable compared to background.

Antibodies useful in the practice of the methods disclosed herein may be conjugated to a solid support suitable for a diagnostic assay (e.g., beads, plates, slides or wells formed from materials such as latex or polystyrene) in accordance with known techniques, such as precipitation. Antibodies or other binding reagents binding reagents may likewise be conjugated to detectable groups such as radiolabels (*e.g.,* ³⁵S, ¹²⁵I, ¹³¹I), enzyme labels *(e.g.,* horseradish peroxidase, rosaline phosphatase), and fluorescent labels (e.g., fluorescein) in accordance with known techniques.

Cell-based assays, such flow cytometry (FC), immunohistochemistry (IHC), or immunofluorescence (IF) are particularly desirable in practicing the methods disclosed herein, since such assay formats are clinically-suitable, allow the detection of expression of a protein with ROS1 kinase activity or a protein with ALK kinase activity *in vivo,* and avoid the risk of artifact changes in activity resulting from manipulating cells obtained from, e.g. a tumor sample in order to obtain extracts. Accordingly, in some embodiments, the methods disclosed herein are implemented in a flow-cytometry (FC), immunohistochemistry (IHC), or immunofluorescence (IF) assay format.

Flow cytometry (FC) may be employed to determine the expression of polypeptide with ROS1 kinase activity or ALK kinase activity or a mutant EGFR polypeptide in a mammalian tumor before, during, and after treatment with one or more drugs targeted at inhibiting ROS1, ALK, and/or EGFR kinase activity. For example, tumor cells from a fine needle aspirate may be analyzed by flow cytometry for expression and/or activation of a polypeptide with ROS1 kinase activity or ALK kinase activity or a mutant EGFR polypeptide or polynucleotide encoding the same, as well as for markers identifying cancer cell types, etc., if so desired. Flow cytometry may be carried out according to standard methods. *See, e.g.* Chow et al., Cytometry (Communications in Clinical Cytometry) 46: 72-78 (2001). Briefly and by way of example, the following protocol for cytometric analysis may be employed: fixation of the cells with 2% paraformaldehyde for 10 minutes at 37 °C followed by permeabilization in 90% methanol for 10 minutes on ice. Cells may then be stained with the primary antibody (e.g., a full-length ROS1-specific or a ROS1 fusion polypeptide-specific antibody or an EGFR mutant-specific antibody), washed and labeled with a fluorescent-labeled secondary antibody. The cells would then be analyzed on a flow cytometer (e.g. a Beckman Coulter FC500) according to the specific protocols of the instrument used. Such an analysis would identify the level of expressed full-length ROS 1 or ALK or a ROS1 fusion or ALK fusion polypeptide or mutant EGFR polypeptide in the tumor. Similar analysis after treatment of the tumor with one or more ROS1-, ALK-, or EGFR-inhibiting therapeutics would reveal the responsiveness of the tumor to the targeted inhibitor of ROS1 or ALK kinase or EGFR kinase.

Immunohistochemical (IHC) staining may be also employed to determine the expression and/or activation status of polypeptide with ROS1 kinase activity or a mutant EGFR polypeptide in a mammalian cancer *(e.g.,* a lung cancer) before, during, and after treatment with a therapeutic targeted at inhibiting ROS1 kinase activity and/or EGFR kinase activity. IHC may be carried out according to well-known techniques. *See, e.g.,* ANTIBODIES: A LABORATORY MANUAL, Chapter 10, Harlow & Lane Eds., Cold Spring Harbor Laboratory (1988). Briefly, and by way of example, paraffin-embedded tissue (e.g. tumor tissue from a biopsy) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody (e.g., a ROS1-specific antibody or EGFR mutant-specific antibody) and secondary antibody; and finally detecting using avidin/biotin method.

Immunofluorescence (IF) assays may be also employed to determine the expression and/or activation status of a polypeptide with ROS1 kinase activity (e.g., full length ROS1 polypeptide or a ROS1 fusion polypeptide) or a mutant EGFR polypeptide in a mammalian cancer before, during, and after treatment with a therapeutic targeted at inhibiting ROS1 kinase activity and/or EGFR kinase activity. IF may be carried out according to well-known techniques. *See, e.g.,* J.M. Polak and S. Van Noorden (1997) INTRODUCTION TO IMMUNOCYTOCHEMISTRY, 2nd Ed.; ROYAL MICROSCOPY SOCIETY MICROSCOPY HANDBOOK 37, BioScientific/Springer-Verlag. Briefly, and by way of example, patient samples may be fixed in paraformaldehyde followed by methanol, blocked with a blocking solution such as horse serum, incubated with a primary antibody against (i.e., that specifically binds to) a polypeptide with ROS1 kinase activity (e.g., a CD74-ROS1 fusion polypeptide) or a polypeptide with ALK kinase activity (e.g., an EML4-ALK fusion polypeptide) or a mutant EGFR polypeptide followed by a secondary antibody labeled with a fluorescent dye such as ALEXA FLUOR 488 and analyzed with an epifluorescent microscope.

A variety of other protocols, including enzyme-linked immunosorbent assay (ELISA), radio-immunoassay (RIA), western blotting analysis, in vitro kinase assay, and fluorescent-activated cell sorting (FACS), for measuring expression and/or activity of a polypeptide with ROS1 kinase activity are known in the art and provide a basis for diagnosing the presence of the polypeptide with ROS1 kinase activity (e.g., a full-length ROS1, or an ROS1 fusion polypeptide such as an FIG-ROS1(S) fusion polypeptide) or the presence of a polypeptide with ALK kinase activity (e.g., full length ALK or an ALK fusion polypeptide such as NPM-ALK fusion polypeptide) or the presence of a mutant EGFR polypeptide. Normal or standard values for ALK or ROS1 (full length or fusion) polypeptide expression are established by combining body fluids or cell extracts taken from normal mammalian subjects, preferably human, with an antibody that specifically binds to a polypeptide with ROS1 kinase activity or a polypeptide with ALK kinase activity under conditions suitable for complex formation. The amount of standard complex formation may be quantified by various methods, but preferably by photometric means. Quantities of full length ROS1 polypeptide expressed in subject, control, and disease samples from biopsied tissues are compared with the standard values. Deviation between standard and subject values establishes the parameters for diagnosing disease. Note that in some tissues (e.g., lung cancer) since the proteins with ROS1 kinase activity or proteins with ALK kinase activity (e.g., SLC34A2-ROS1(S) and EML4-ALK (796aa variant)) or mutant EGFR polypeptides were discovered in cancerous tissue, no normal lung tissue biological samples are expected to contain these proteins with ROS1 kinase activity or ALK kinase activity (or polynucleotides encoding the same) or mutant EGFR polypeptides.

In another aspect, the disclosure provides methods for detecting the presence of a polynucleotide encoding a polypeptide with ROS1 kinase activity or ALK kinase activity or a polynucleotide encoding a mutant EGFR polypeptide in a biological sample from a mammalian lung cancer or suspected mammalian lung cancer, said methods including the steps of: (a) obtaining a biological sample from a mammalian lung cancer or suspected mammalian lung cancer and (b) utilizing a reagent that specifically binds to said polynucleotide encoding said polypeptide to determine whether said polynucleotide is present in said biological sample, wherein detection of specific binding of said reagent to said biological sample indicates said polynucleotide encoding said polypeptide with ROS1 kinase activity or ALK kinase activity or mutant EGFR polypeptide is present in said biological sample.

The presence of a polynucleotide encoding a polypeptide having ROS1 kinase activity or ALK kinase activity or a mutant EGFR polypeptide can be assessed by any standard methods. In addition, these methods can be combined with methods to detect the polypeptide having ROS 1 kinase activity or ALK kinase activity or mutant EGFR polypeptide as described above.

### Nucleotide Assays.

Full length ROS1 polynucleotide or ROS1 fusion polynucleotide-specific binding reagents, full length ALK polynucleotide or ALK fusion polynucleotide-specific binding reagents, and mutant EGFR polynucleotide-specific binding reagents useful in practicing the methods disclosed herein may also be mRNA, oligonucleotide or DNA probes that can directly hybridize to, and detect, fusion or truncated polypeptide expression transcripts in a biological sample. Such probes are discussed in detail herein. Briefly, and by way of example, formalin-fixed, paraffin-embedded (PPFE) patient samples may be probed with a fluorescein-labeled RNA probe followed by washes with formamide, SSC and PBS and analysis with a fluorescent microscope.

Polynucleotides encoding a polypeptide with ROS1 or ALK kinase activity or a mutant EGFR polypeptide may also be used for diagnostic purposes. The polynucleotides that may be used include oligonucleotide sequences, antisense RNA and DNA molecules, and PNAs. The polynucleotides may be used to detect and quantitate gene expression in biopsied tissues in which expression of a polypeptide with ROS1 or ALK kinase activity (e.g., a ROS1 or ALK fusion polypeptide or full length ROS1 or ALK) or a mutant EGFR polypeptide may be correlated with disease. The diagnostic assay may be used to distinguish between absence, presence, and excess expression of a polypeptide with ROS1 or ALK kinase activity or a mutant EGFR polypeptide, and to monitor regulation of levels of a polypeptide with ROS 1 or ALK kinase activity or a mutant EGFR polypeptide during therapeutic intervention.

In one embodiment, hybridization with PCR primers that are capable of detecting polynucleotide sequences, including genomic sequences, encoding a polypeptide with ROS1 or ALK kinase activity or a mutant EGFR polypeptide may be used to identify nucleic acid sequences that encode such polypeptides with ROS1 or ALK kinase activity or mutant EGFR polypeptides. The specificity of the probe, whether it is made from a highly specific region, e.g., 10 unique nucleotides in the fusion junction, or a less specific region, *e.g.,* the 3' coding region, and the stringency of the hybridization or amplification (maximal, high, intermediate, or low) will determine whether the probe identifies only naturally occurring sequences encoding ROS1 or ALK kinase polypeptides (e.g., full length ROS1 or ALK or a ROS1 or ALK fusion protein) or mutant EGFR polypeptides, alleles, or related sequences.

Probes may also be used for the detection of related sequences. The hybridization probes (e.g., FISH probes or Southern or Northern blotting probes) of the subject methods may be DNA or RNA and derived from the nucleotide sequences of encoding polypeptides with ROS1 kinase activity, polypeptides with ALK kinase activity, or mutant EGFR polypeptides. In some embodiments, where the polypeptide having ROS1 or ALK kinase activity is a fusion protein, the hybridization probes encompassing the fusion junction, or from genomic sequence including promoter, enhancer elements, and introns of the naturally occurring ROS1 or ALK gene and the fusion partner gene (e.g., for ROS1, SLC34A2, FIG, or CD74; for ALK, NPM, EML4, TFG, etc.).

A ROS1 fusion polynucleotide (i.e., a polynucleotide encoding a ROS1 fusion polypeptide such as FIG-ROS1(S) or CD74-ROS1), full length ROS1 polynucleotide, ALK fusion polynucleotide (i.e., a polynucleotide encoding an ALK fusion polynucleotide such as EML4-ALK(796 aa variant), full length ALK polynucleotide, or mutant EGFR polynucleotide may be used in Southern or northern analysis, dot blot, or other membrane-based technologies; in PCR technologies; or in dip stick, pin, ELISA or chip assays utilizing fluids or tissues from patient biopsies to detect altered expression of a polypeptide with ROS1 kinase activity or expression of a mutant EGFR polypeptide. Such qualitative or quantitative methods are well known in the art. In a particular aspect, the nucleotide sequences encoding a polypeptide with ROS1 or ALK kinase activity or a mutant EGFR polypeptide may be useful in assays that detect activation or induction of various cancers, including lung cancer (e.g., non-small cell lung carcinoma (NSCLC) and small cell lung carcinoma) and colon cancer. Polynucleotides encoding a polypeptide with ROS1 kinase activity or a mutant EGFR polypeptide may be detectably labeled by standard methods, and added to a fluid or tissue sample from a patient under conditions suitable for the formation of hybridization complexes. After a suitable incubation period, the sample is washed and the signal is quantitated and compared with a standard value. If the amount of signal in the biopsied or extracted sample is significantly altered from that of a comparable control sample, the nucleotide sequences have hybridized with nucleotide sequences in the sample, and the presence of altered levels of nucleotide sequences encoding a polypeptide with ROS1 or ALK kinase activity (e.g., a ROS1 or ALK fusion polypeptide or full length ROS1 or ALK polypeptide) or a mutant EGFR polypeptide in the sample indicates the presence of the associated disease. Such assays may also be used to evaluate the efficacy of a particular therapeutic treatment regimen in animal studies, in clinical trials, or in monitoring the treatment of an individual patient.

In some embodiments, the methods disclosed herein are carried out using a nucleic acid amplification (e.g., PCR) assay format. Polymerase chain reaction (PCR) is standard to those of skill in the art. *See, e.g.,* MOLECULAR CLONING, A LABORATORY MANUAL, 2nd. edition, Sambrook, J., Fritsch, E. F. and Maniatis, T., eds., Cold Spring Harbor Laboratory Press, Cold Spring Harbor, N.Y. (1989). PCR primers (also called oligomers) may be chemically synthesized, generated enzymatically, or produced from a recombinant source. Oligomers will preferably consist of two nucleotide sequences, one with sense orientation (5' to 3') and another with antisense (3' to 5'), employed under optimized conditions for identification of a specific gene or condition. The same two oligomers, nested sets of oligomers, or even a degenerate pool of oligomers may be employed under less stringent conditions for detection and/or quantitation of closely related DNA or RNA sequences.

Methods which may also be used to quantitate the expression of a nucleotide encoding a polypeptide with ROS1 or ALK kinase activity (e.g., ROS1 or ALK fusion polypeptide or full ROS1 or ALK polypeptide) or a mutant EGFR polypeptide include radiolabeling or biotinylating nucleotides, co-amplification of a control nucleic acid, and standard curves onto which the experimental results are interpolated (Melby et al., J. Immunol. Methods, 159: 235-244 (1993); Duplaa et al. Anal. Biochem. 229-236 (1993)). The speed of quantitation of multiple samples may be accelerated by running the assay in an ELISA format where the oligomer of interest is presented in various dilutions and a spectrophotometric or colorimetric response gives rapid quantitation.

In another embodiment, the polynucelotides encoding a polypeptide with ROS1 or ALK kinase activity or a mutant EGFR polypeptide may be used to generate hybridization probes which are useful for mapping the naturally occurring genomic sequence. The sequences may be mapped to a particular chromosome or to a specific region of the chromosome using well-known techniques. Such techniques include fluorescence in-situ hybridization (FISH), FACS, or artificial chromosome constructions, such as yeast artificial chromosomes, bacterial artificial chromosomes, bacterial P1 constructions or single chromosome cDNA libraries, as reviewed in Price, C. M., Blood Rev. 7: 127-134 (1993), and Trask, B. J., Trends Genet. 7: 149-154 (1991).

In further embodiments, fluorescence in-situ hybridization (FISH) is employed in the methods disclosed herein (as described in Verma et al. HUMAN CHROMOSOMES: A MANUAL OF BASIC TECHNIQUES, Pergamon Press, New York, N.Y. (1988)). In some embodiments, the FISH assay may be correlated with other physical chromosome mapping techniques and genetic map data. The FISH technique is well known (see, e.g., US Patent Nos. 5,756,696; 5,447,841; 5,776,688; and 5,663,319). Examples of genetic map data can be found in the 1994 Genome Issue of Science (265: 1981f). Correlation between the location of the gene encoding ROS1 or ALK protein and/or, in the case of fusion polypeptides, the gene encoding the fusion partner of a ROS1 or ALK fusion protein (e.g., for ROS1, the FIG gene, the SLC34A2 gene, or the CD74 gene; for ALK, the EML4 gene, the NPM gene, the ATIC gene, the CARS gene, etc.) on a physical chromosomal map and a specific disease, or predisposition to a specific disease, may help delimit the region of DNA associated with that genetic disease. The nucleotide sequences may be used to detect differences in gene sequences between normal, carrier, or affected individuals.

*In situ* hybridization of chromosomal preparations and physical mapping techniques such as linkage analysis using established chromosomal markers may be used for extending genetic maps. Often the placement of a gene on the chromosome of another mammalian species, such as mouse, may reveal associated markers even if the number or arm of a particular human chromosome is not known. New sequences can be assigned to chromosomal arms, or parts thereof, by physical mapping. This provides valuable information to investigators searching for disease genes using positional cloning or other gene discovery techniques. Once the disease or syndrome has been crudely localized by genetic linkage to a particular genomic region, for example, AT to 11q22-23 (Gatti et al., Nature 336: 577-580 (1988)), any sequences mapping to that area may represent associated or regulatory genes for further investigation. The nucleotide sequence may also be used to detect differences in the chromosomal location due to translocation, inversion, etc., among normal, carrier, or affected individuals.

Polynucleotides encoding a polypeptide with ROS1 or ALK kinase activity or a mutant EGFR may be detected by nucleotide sequencing, e.g., of chromosomal or expressed (e.g., mRNA, cDNA) nucleic acids. Methods of nucleic acid sequencing are well known and include chain termination sequencing and other sequencing techniques, such as single-molecule real-time sequencing, ion semiconductor sequencing, pyrosequencing, sequencing by synthesis (e.g., offered by Illumina), and sequencing by ligation (SOLiD sequencing).

It shall be understood that all of the methods (e.g., PCR, FISH, sequencing) that detect polynucleotides encoding a polypeptide with ROS1 or ALK kinase activity or a mutant EGFR polypeptide, may be combined with other methods that detect polypeptides with ROS1 or ALK kinase activity or a mutant EGFR polypeptide or polynucleotides encoding a polypeptide with ROS1 or ALK kinase activity or a mutant EGFR polypeptide. For example, detection of a FIG-ROS1(S) fusion polynucleotide in the genetic material of a biological sample (e.g., FIG-ROS1(S) in a circulating tumor cell) may be followed by western blotting analysis or immuno-histochemistry (IHC) analysis of the proteins of the sample to determine if the FIG-ROS1(S) polynucleotide was actually expressed as a FIG-ROS1(S) fusion polypeptide in the biological sample. Such western blotting or IHC analyses may be performed using an antibody that specifically binds to the polypeptide encoded by the detected FIG-ROS1(S) polynucleotide, or the analyses may be performed using antibodies that specifically bind either to full length FIG (e.g., bind to the N-terminus of the protein) or to full length ROS1 (e.g., bind an epitope in the kinase domain of ROS1). Such assays are known in the art (see, e.g., US Patent 7,468,252).

In another example, the CISH technology of Dako allows chromatogenic *in situ* hybridization with immuno-histochemistry on the same tissue section. See Elliot et al., Br J Biomed Sci 2008; 65: 167- 171, 2008 for a comparison of CISH and FISH.

Another aspect of the disclosure provides methods for diagnosing a patient as having a cancer or a suspected cancer driven by an ROS1 kinase, an ALK kinase, or a mutant EGFR polypeptide. The methods include contacting a biological sample of said cancer or a suspected cancer (where the biological sample contains at least one nucleic acid molecule) with a probe that hybridizes under stringent conditions to a nucleic acid molecule encoding a polypeptide with ROS1 or ALK kinase activity or a mutant EGFR polypeptide, and wherein hybridization of said probe to at least one nucleic acid molecule in said biological sample identifies said patient as having a cancer or a suspected cancer driven by a ROS1 kinase or a mutant EGFR polypeptide.

Yet another aspect of the disclosure provides a method for diagnosing a patient as having a cancer or a suspected cancer driven by a ROS1 kinase or ALK kinase or a mutant EGFR polypeptide. The method includes contacting a biological sample of said cancer or suspected cancer (where said biological sample contains at least one polypeptide) with a reagent that specifically binds to a polypeptide with ROS1 or ALK kinase activity or a mutant EGFR polypeptide, wherein specific binding of said reagent to at least one polypeptide in said biological sample identifies said patient as having a lung cancer or a suspected lung cancer driven by a ROS1 kinase or an ALK kinase or a mutant EGFR polypeptide.

In various embodiments, the identification of a lung cancer or suspected lung cancer as being driven by a ROS1 kinase or a mutant EGFR polypeptide will identify that patient having that lung cancer or suspected lung cancer as being likely to respond to a ROS1-inhibiting therapeutic, an EGFR-inhibiting therapeutic, or both.

In order to provide a basis for the diagnosis of disease *(e.g.,* a lung cancer) characterized by expression of a polypeptide with ROS1 or ALK kinase activity or a mutant EGFR polypeptide, a normal or standard profile for expression may be established. This may be accomplished by combining body fluids or cell extracts taken from normal subjects, either animal or human, with a polynucleotide sequence, or a fragment thereof, which encodes a polypeptide with ROS1 or ALK kinase activity or a mutant EGFR polypeptide, under conditions suitable for hybridization or amplification. Standard hybridization may be quantified by comparing the values obtained from normal subjects with those from an experiment where a known amount of a substantially purified polynucleotide is used. Standard values obtained from normal samples may be compared with values obtained from samples from patients who are symptomatic for disease. Deviation between standard and subject values is used to establish the presence of disease.

Once disease is established and a treatment protocol is initiated, hybridization assays may be repeated on a regular basis to evaluate whether the level of expression in the patient begins to approximate that which is observed in the normal patient. The results obtained from successive assays may be used to show the efficacy of treatment over a period ranging from several days to months.

A similar normal or standard profile for expression or activity level of a polypeptide having ROS1 or ALK kinase activity or a mutant EGFR polypeptide can be established. For example, for protein expression, the profile can be established using a reagent that specifically binds to the polypeptide can also be established using, e.g., an antibody that specifically binds to the polypeptide (e.g., binds to full length ROS1 or binds to the fusion junction of a ROS1 fusion polypeptide) and comparing levels of binding in normal subject with levels of binding in patients symptomatic for lung cancer. Similarly, for ROS1, ALK, or EGFR kinase activity levels, a standard in vitro kinase assay (see Ausubel et al., supra; Sambrook et al., supra) can be performed on a samples taken from normal patients as compared to samples taken from patients symptomatic for lung cancer.

In various embodiments, the inhibition of ROS1 or ALK expression or kinase activity is determined using a reagent that specifically binds to a ROS1 or ALK fusion polynucleotide, a reagent that specifically binds to ROS1 or ALK fusion polypeptide, a reagent that specifically binds to a full length ROS1 or ALK polynucleotide, or a reagent that specifically binds to a full length ROS1 or ALK polypeptide. In some additional embodiments, the inhibition of ROS1 or ALK expression or kinase activity is determined using a reagent that specifically binds to the full length protein of a fusion partner of a ROS1 fusion polypeptide or a ALK fusion protein. For example, for ROS1, the reagent may specifically bind a FIG or CD74 or SLC34A2 polynucleotide or specifically binds to a full length FIG or CD74 or SLC34A2 polypeptide. For ROS1, the reagent may specifically binds to a full length NPM or EML4 or ATIC or CARS or TFG or KIF5B or RANBP2 or TPM3, or ALO17 or MSN or TPM4 or ATIC or MYH9 or CLTC or SEC31L1 polynucleotide, or may specifically binds to a full length NPM or EML4 or ATIC or CARS or TFG or KIF5B or RANBP2 or TPM3, or ALO17 or MSN or TPM4 or ATIC or MYH9 or CLTC or SEC31L1 polypeptide.

In various embodiments, the expression and/or activity of said ALK or ROS1 polypeptide is inhibited with a composition that includes a therapeutic selected from the group consisting of crizotinib (also known as PF-02341066), ASP3026, NVP TAE-684, AP26113, CEP-14083, CEP-14513, CEP11988, WHI-P131 and WHI-P154.

As used herein, a "ROS1 inhibitor" or a "ROS1-inhibiting compound" means any composition that includes one or more compounds, chemical or biological, that inhibit, either directly or indirectly, the expression and/or activity of a polypeptide with ROS1 kinase activity. Such inhibition may be in vitro or in vivo. "ROS1 inhibitor therapeutic" or "ROS1-inhibiting therapeutic" means a ROS1-inhibiting compound used as a therapeutic to treat a patient harboring a cancer *(e.g.,* a lung cancer such as NSCLC or SCLC) characterized by the presence of a polypeptide with ROS1 kinase activity such as aberrantly expressed full length ROS1 protein or a ROS1 fusion polypeptide (e.g., one of the FIG-ROS1 fusion proteins) described herein.

In some embodiments of the disclosure, the ROS1 inhibitor is a reagent that specifically binds to a ROS1 fusion polypeptide (e.g., FIG-ROS1(S), FIG-ROS1(L), FIG-ROS1(XL), SLC34A2-ROS1(VS), SLC34A2-ROS1(S), SLC34A2-ROS1(L), or CD74-ROS1), a reagent that specifically binds to a full length ROS1 polypeptide, an siRNA targeting a ROS1 fusion polynucleotide (e.g., an SLC34A2-ROS1(S) fusion polynucleotide) or an siRNA targeting a full length ROS1 polynucleotide. Non-limiting siRNAs for inhibiting ROS1 protein expression are as follows:
5'AAGCCCGGAUGGCAACGUUTT3' (ROS1(6318-6340) (SEQ ID NO: 16)); or
5'AAGCCUGAAGGCCUGAACUTT3' (ROS1(7181-7203) (SEQ ID NO: 17)).

The ability of these two siRNAs to inhibit ROS1 kinase activity has been described (see U.S. Patent Publication No. 20100221737, incorporated by reference.

In some embodiments, a ROS1 inhibitor is selected from the group consisting of crizotinib (also known as PF-02341066), ASP3026 (ClinicalTrials.gov Identifier: NCT01284192), NVP TAE-684 (Gu et al., 2011, PLos ONE, 6:el5640), CH5424802 (Sakamoto et al., 2011, Cancer Cell, 19:679-690), and AP26113 (ClinicalTrials.gov Identifier: NCT01449461; Katayama et al., 2011, Proc. Natl. Acad. Sci. USA, 108:7535-40). Additional ROS1 inhibitors are disclosed, e.g., in WO 2012/016133, US 2012/0065233, and El-Deeb et al., 2009, Bioorg. Med. Chem Lett., 19:5622-26.

As used herein, a "ALK inhibitor" or a "ALK-inhibiting compound" means any composition that includes one or more compounds, chemical or biological, that inhibits, either directly or indirectly, the expression and/or activity of a polypeptide with ALK kinase activity. Such inhibition may be in vitro or in vivo. "ALK inhibitor therapeutic" or "ALK -inhibiting therapeutic" means a ALK -inhibiting compound used as a therapeutic to treat a patient harboring a cancer *(e.g.,* a lung cancer such as NSCLC or SCLC) characterized by the presence of a polypeptide with ALK kinase activity such as aberrantly expressed full length ALK protein or a ALK fusion polypeptide (e.g., one of the EM4-ALK fusion proteins) described herein.

The ALK and/or ROS1-inhibiting therapeutic may be, for example, a kinase inhibitor, such as a small molecule or antibody inhibitor. It may be a pan-kinase inhibitor with activity against several different kinases, or a kinase-specific inhibitor. Since ROS1, ALK, LTK, InsR, and IGF1R belong to the same family of tyrosine kinases, they may share similar structure in the kinase domain. Thus, in some embodiments, an ALK and/or ROS1 inhibitor also inhibits the activity of an ALK kinase, an LTK kinase, an insulin receptor, or an IGF1 receptor. ROS1-inhibiting compounds are discussed in further detail below. Patient biological samples may be taken before and after treatment with the inhibitor and then analyzed, using methods described above, for the biological effect of the inhibitor on ALK or ROS1 kinase activity, including the phosphorylation of downstream substrate protein. Such a pharmacodynamic assay may be useful in determining the biologically active dose of the drug that may be preferable to a maximal tolerable dose. Such information would also be useful in submissions for drug approval by demonstrating the mechanism of drug action.

In another embodiment, the expression and/or activity of said polypeptide is inhibited with a composition that includes a ROS1 or ALK inhibiting therapeutic selected from the group consisting of PF-02341066, NVP TAE-684, AP26113, CEP-14083, CEP-14513, CEP11988, WHI-P131 and WHI-P154.

Various EGFR inhibitors are known and can be used in the methods disclosed herein, including gefitinib, erlotinib, cetuximab, afatinib, necitumumab, nimotuzumab, PF299804 (Jänne et al., 2011, Clin. Cancer Res., 17:1131-39), RO5083945 (glycoengineered anti-EGFR monoclonal antibody; Hoffmann-La Roche; Markman et al., 2010, J. Clin. Oncol., 28:15s, abstr 2522), ABT-806 (humanized anti-EGFR monoclonal antibody; Abbott), NVP-TAE684 (Katayama et al., 2011, Proc. Natl. Acad. Sci. USA, 108:7535-40), and AP26113 (ibid.).

In accordance with the present disclosure, the polypeptide with ROS1 or ALK kinase activity or mutant EGFR polypeptide may occur in at least one subgroup of human cancer. Accordingly, the progression of a mammalian cancer in which a polypeptide with ROS1 or ALK kinase activity or mutant EGFR polypeptide is expressed may be inhibited, *in vivo,* by inhibiting the activity of ROS1 or ALK kinase or mutant EGFR polypeptide in such cancer. ROS1 or ALK activity in cancers characterized by expression of a polypeptide with ROS1 or ALK kinase activity may be inhibited by contacting the cancer with a therapeutically effective amount of a ROS1-inhibiting and/or ALK-inhibiting therapeutic. Additionally, mutant EGFR activity may be inhibited by contacting the cancer with a therapeutically effective amount of an EGFR inhibiting therapeutic. Accordingly, the disclosure provides, in part, a method for inhibiting the progression of cancers (e.g., lung cancers) that express a polypeptide with ROS1 or ALK kinase activity and a mutant EGFR polypeptide by inhibiting the expression and/or activity of ROS1 or ALK kinase and the mutant EGFR polypeptide in the cancer by contacting the cancer *(e.g.,* a lung cancer) with a therapeutically effective amount of an ROS1-inhibiting therapeutic and/or an EGFR-inhibiting therapeutic.

As used herein, by "therapeutically effective amount" or "pharmaceutically effective amount" is mean an amount of an ROS1-inhibiting therapeutic, ALK-inhibiting therapeutic, and/or EGFR-inhibiting therapeutic that is adequate to inhibit the cancer (or cell thereof) or suspected cancer (or cells thereof), as compared to an untreated cancer or suspected cancer, by either slowing the growth of the cancer or suspected cancer, reducing the mass of the cancer or suspected cancer, reducing the number of cells of the cancer or suspected cancer, or killing the cancer. When two or more therapeutics are administered to a patient in combination, the effective amount of each therapeutic may be less than if the therapeutic were to be administered alone.

A ROS1-inhibiting therapeutic and/or ALK-inhibiting therapeutic may be any composition that includes at least one ROS1 or ALK inhibitor. Such compositions also include compositions including only a single ROS1- or ALK-inhibiting compound, as well as compositions that include multiple therapeutics (including those against other RTKs), which may also include a non-specific therapeutic agent like a chemotherapeutic agent or general transcription inhibitor.

In some embodiments, a ROS1-inhibiting therapeutic and/or ALK-inhibiting therapeutic useful in the practice of the methods disclosed herein is a targeted, small molecule inhibitor. Small molecule targeted inhibitors are a class of molecules that typically inhibit the activity of their target enzyme by specifically, and often irreversibly, binding to the catalytic site of the enzyme, and/or binding to an ATP-binding cleft or other binding site within the enzyme that prevents the enzyme from adopting a conformation necessary for its activity. Because of the close similarity in structure and function between the ROS1 kinase and the ALK kinase, any ALK kinase inhibitor is predicted to also inhibit ROS1 kinase.

Accordingly, in another aspect, the disclosure provides methods of treating a patient for lung cancer, that include: detecting the presence in a biological sample from a lung of a patient having or suspected of having lung cancer of one or more polypeptides selected from the group consisting of a polypeptide having ROS1 kinase activity, a polypeptide having ALK kinase activity, and a mutant EGFR polypeptide (in any combination); and administering an effective amount of an ALK/ROS1-inhibiting therapeutic and/or an EGFR-inhibiting therapeutic to the patient, thereby treating the subject for lung cancer.

It should be noted that when a ROS1 or ALK inhibitor and an EGFR inhibitor are administered to a patient, that the inhibitory molecule may be one that inhibits both ROS1 or ALK and EGFR. For example, NVP TAE-684 inhibits ROS1 (Gu et al., 2011, PLos ONE, 6:e15640), ALK (Galkin et al., 2007, Proc. Natl. Acad. Sci. USA, 104:270-275) and EGFR (Katayama et al., 2011, Proc. Natl. Acad. Sci. USA, 108:7535-40), as does AP26113 (Katayama et al., 2011, Proc. Natl. Acad. Sci. USA, 108:7535-40). Additional molecules may be identified that inhibit both ROS1 and/or ALK and EGFR.

As used herein, by "protein having ALK kinase activity" is meant any polypeptide that retains the full kinase domain of ALK and thus, has ALK kinase activity. Non-limiting polypeptides with ALK kinase activity include full length ALK (see U.S. Patent No. 5,770,421), NPM-ALK, ALO17-ALK, TFG-ALK, MSN-ALK, TPM3-ALK, TPM4-ALK, ATIC-ALK, MYH9-ALK, CLTC-ALK, SEC31L1-ALK, RANBP2-ALK, CARS-ALK, EML4-ALK, KIF5B-ALK, and TFG-ALK (see, e.g., Palmer et al., Biochem. J. 420:345-361, 2009 (and the articles cited therein), Rikova et al., Cell 131:1190-1203, 2007; Soda et al., Nature 448:561-566, 2007; Morris et al., Science 263:1281-84, 1994; Du et al., J. Mol. Med 84:863-875, 2007; Panagopoulos et al., Int. J. Cancer 118:1181-86, 2006; Cools et al., Genes Chromosomes Cancer 34:354-362, 2002; Debelenko et al., Lab. Invest. 83:1255-65, 2003; Ma et al., Genes Chromosomes Cancer 37:98-105, 2003; Lawrence et al., Am. J. Pathol. 157:377-384, 1995; Hernandez et al., Blood 94:3265-68, 1999; Takeuchi K., Clin Cancer Res. 15:3143-49, 2009; Tort et al., Lab. Invest. 81:419-426, 2001; Trinei et al., Cancer Res. 60:793-798, 2000; and Touriol et al., Blood 95:3204-07, 2000. See also Pulford et al., J. Cell. Physiol., 199:330-358, 2004.

In various embodiments, the patient is a human. In various embodiments, the lung cancer is non-small cell lung cancer or is small cell lung cancer.

One useful small-molecule kinase inhibitor is Pfizer, Inc.'s compound crizotinib (also known as PF-02341066), which inhibits ALK, ROS1, and MET kinase activity, and its properties have been well described. *See* You et al., Cancer Res 67: 4408 (2007) and U.S. Patent Pub. No. 2008/0300273. Additional small molecule kinase inhibitors that target ROS1 include TAE-684 (from Novartis), CH5424802 (Chugai; see Sakamoto, H. et al., Cancer Cell 19: 679-690, 2011), AP26113 (Ariad Pharmaceuticals, Inc.), and CEP-14083, CEP-14513, and CEP-11988 (Cephalon; see Wan et al., Blood 107: 1617-23, 2006). TAE-684, a 5-chloro-2,4-diaminophenylpyrimidine, has also been shown to inhibit the ALK kinase. Galkin, et al., Proc. National Acad. Sci 104:270-275, 2007.

Additional small molecule inhibitors and other inhibitors (e.g., indirect inhibitors) of ROS1 kinase activity may be rationally designed using X-ray crystallographic or computer modeling of ROS1 three dimensional structure, or may found by high throughput screening of compound libraries for inhibition of key upstream regulatory enzymes and/or necessary binding molecules, which results in inhibition of ROS1 or ALK kinase activity. Such approaches are well known in the art, and have been described. ROS1 inhibition or ALK inhibition by such therapeutics may be confirmed, for example, by examining the ability of the compound to inhibit ROS1 or ALK kinase activity, but not other kinase activity, in a panel of kinases, and/or by examining the inhibition of ROS1 or ALK activity in a biological sample that includes cancer cells (e*.g.*, lung cancer cells). Methods for identifying compounds that inhibit a cancer characterized by the expression/presence of polypeptide with ROS1 or ALK kinase activity are further described below.

ROS1-inhibiting therapeutics, ALK-inhibiting therapeutics, and/or EGFR-inhibiting therapeutics useful in the methods disclosed herein may also be targeted antibodies that specifically bind to critical catalytic or binding sites or domains required for ROS1 or ALK activity, and inhibit the kinase by blocking access of ligands, substrates or secondary molecules to α and/or preventing the enzyme from adopting a conformation necessary for its activity. The production, screening, and therapeutic use of humanized target-specific antibodies has been well-described. *See* Merluzzi et al., Adv Clin Path. 4(2): 77-85 (2000). Commercial technologies and systems, such as Morphosys, Inc.'s Human Combinatorial Antibody Library (HuCAL^{®}), for the high-throughput generation and screening of humanized target-specific inhibiting antibodies are available.

The production of various anti-receptor kinase targeted antibodies and their use to inhibit activity of the targeted receptor has been described. *See, e.g.* U.S. Patent Publication No. 20040202655, U.S. Patent Publication No. 20040086503, U.S. Patent Publication No. 20040033543. Standardized methods for producing, and using, receptor tyrosine kinase activity-inhibiting antibodies are known in the art. *See, e.g.,* European Patent No. EP1423428,

Phage display approaches may also be employed to generate ROS1-specific, ALK-specific, or EGFR-specific antibody inhibitors, and protocols for bacteriophage library construction and selection of recombinant antibodies are provided in the well-known reference text CURRENT PROTOCOLS IN IMMUNOLOGY, Colligan et al. (Eds.), John Wiley & Sons, Inc. (1992-2000), Chapter 17, Section 17.1. *See also* U.S. Patent No. 6,319,690, U.S. Patent No. 6,300,064, U.S. Patent No. 5,840,479, and U.S. Patent Publication No. 20030219839.

A library of antibody fragments displayed on the surface of bacteriophages may be produced (*see, e.g.* U. S. Patent 6,300,064) and screened for binding to a polypeptide with ROS1 kinase activity or ALK kinase activity or an EGFR polypeptide (e.g., a mutant EGFR polypeptide). *See* European Patent No. EP1423428.

Antibodies identified in screening of antibody libraries as described above may then be further screened for their ability to block the activity of ROS1, ALK, or EGFR (e.g., a mutant EGFR), both *in vitro* kinase assay and *in vivo* in cell lines and/or tumors. ROS1, ALK, or EGFR inhibition may be confirmed, for example, by examining the ability of such antibody therapeutic to inhibit ROS1, ALK, or EGFR kinase activity in a panel of kinases, and/or by examining the inhibition of ROS1, ALK, or EGFR activity in a biological sample that includes cancer cells, as described above. In some embodiments, a ROS1-inhibiting compound reduces ROS1 kinase activity, but reduces the kinase activity of other kinases to a lesser extent (or not at all). Likewise, in some embodiments, an ALK-inhibiting compound reduces ALK kinase activity, but reduces the kinase activity of other kinases to a lesser extent (or not at all). Similarly, in some embodiments, an EGFR-inhibiting compound reduces EGFR kinase activity, but reduces the kinase activity of other kinases to a lesser extent (or not at all). Methods for screening such compounds for ROS1, ALK, and/or EGFR kinase inhibition are further described above.

ROS1-inhibiting, ALK-inhibiting, or EGFR-inhibiting compounds that useful in the practice of the disclosed methods may also be compounds that indirectly inhibit ROS1, ALK, or EGFR activity by inhibiting the activity of proteins or molecules other than ROS1, ALK, or EGFR kinase itself. Such inhibiting therapeutics may be targeted inhibitors that modulate the activity of key regulatory kinases that phosphorylate or de-phosphorylate (and hence activate or deactivate) ROS1, ALK, or EGFR itself, or interfere with binding of ligands. As with other receptor tyrosine kinases, ROS1, ALK, and EGFR regulate downstream signaling through a network of adaptor proteins and downstream kinases. As a result, induction of cell growth and survival by ROS1, ALK, or EGFR activity may be inhibited by targeting these interacting or downstream proteins.

ROS1, ALK, or EGFR kinase activity may also be indirectly inhibited by using a compound that inhibits the binding of an activating molecule necessary for these full length and fusion polypeptide (e.g., an CD74-ROS1 or an EML4-ALK fusion polypeptide) to adopt its active conformation (i.e., such that the kinase domain is able to be activated). For example, the production and use of anti-PDGF antibodies has been described. *See* U.S. Patent Publication No. 20030219839, "Anti-PDGF Antibodies and Methods for Producing Engineered Antibodies," Bowdish et al. Inhibition of ligand (PDGF) binding to the receptor directly down-regulates the receptor activity.

ROS1, ALK, and/or EGFR inhibiting compounds or therapeutics may also include antisense and/or transcription inhibiting compounds that inhibit ROS1, ALK, or EGFR kinase activity by blocking transcription of the gene encoding polypeptides with ROS1, ALK, or EGFR kinase activity. The inhibition of various receptor kinases, including VEGFR, EGFR, and IGFR, and FGFR, by antisense therapeutics for the treatment of cancer has been described. *See, e.g.,* U.S. Patent Nos. 6,734,017; 6, 710,174, 6,617,162; 6,340,674; 5,783,683; 5,610,288.

Antisense oligonucleotides may be designed, constructed, and employed as therapeutic agents against target genes in accordance with known techniques. *See, e.g.* Cohen, J., Trends in Pharmacol. Sci. 10(11): 435-437 (1989); Marcus-Sekura, Anal. Biochem. 172: 289-295 (1988); Weintraub, H., Sci. AM. Pp. 40-46 (1990); Van Der Krol et al., BioTechniques 6: 958-976 (1988); Skorski et al., Proc. Natl. Acad. Sci. USA (1994) 91: 4504-4508. Inhibition of human carcinoma growth *in vivo* using an antisense RNA inhibitor of EGFR has recently been described. See U.S. Patent Publication No. 20040047847. Similarly, a ROS1-inhibiting or ALK-inhibiting therapeutic that includes at least one antisense oligonucleotide against a mammalian ROS1 or ALK gene or a mammalian ROS1 or ALK fusion protein-encoding polynucleotide may be prepared according to standard methods. Pharmaceutical compositions that include ROS1-inhibiting antisense compounds may be prepared and administered as further described below.

Small interfering RNA molecule (siRNA) compositions, which inhibit translation, and hence activity, of ROS1, ALK, or EGFR through the process of RNA interference, may also be desirably employed in the methods disclosed herein. RNA interference, and the selective silencing of target protein expression by introduction of exogenous small double-stranded RNA molecules having sequence complimentary to mRNA encoding the target protein, has been well described. *See, e.g.* U.S. Patent Publication No. 20040038921, U.S. Patent Publication No. 20020086356, and U.S. Patent Publication 20040229266.

Double-stranded RNA molecules (dsRNA) have been shown to block gene expression in a highly conserved regulatory mechanism known as RNA interference (RNAi). Briefly, the RNAse III Dicer processes dsRNA into small interfering RNAs (siRNA) of approximately 22 nucleotides, which serve as guide sequences to induce target-specific mRNA cleavage by an RNA-induced silencing complex RISC (*see* Hammond et al., Nature (2000) 404: 293-296). RNAi involves a catalytic-type reaction whereby new siRNAs are generated through successive cleavage of longer dsRNA. Thus, unlike antisense, RNAi degrades target RNA in a non-stoichiometric manner. When administered to a cell or organism, exogenous dsRNA has been shown to direct the sequence-specific degradation of endogenous messenger RNA (mRNA) through RNAi.

A wide variety of target-specific siRNA products, including vectors and systems for their expression and use in mammalian cells, are now commercially available. *See, e.g.,* Promega, Inc. (www.promega.com); Dharmacon, Inc. (www.dharmacon.com). Detailed technical manuals on the design, construction, and use of dsRNA for RNAi are available. *See, e.g.,* Dharmacon's "RNAi Technical Reference & Application Guide"; Promega's "RNAi: A Guide to Gene Silencing." ROS1-inhibiting siRNA products are also commercially available, and may be suitably employed in the methods disclosed herein. *See, e.g.,* Dharmacon, Inc., Lafayette, CO (Cat Nos. M-003162-03, MU-003162-03, D-003162-07 thru -10 (siGENOME^{™} SMARTselection and SMARTpool^{®} siRNAs).

It has recently been established that small dsRNA less than 49 nucleotides in length, and preferably 19-25 nucleotides, that include at least one sequence that is substantially identical to part of a target mRNA sequence, and which dsRNA optimally has at least one overhang of 1-4 nucleotides at an end, are most effective in mediating RNAi in mammals. *See* U.S. Patent Publication Nos. 20040038921 and 20040229266. The construction of such dsRNA, and their use in pharmaceutical preparations to silence expression of a target protein, *in vivo,* are described in detail in such publications.

If the sequence of the gene to be targeted in a mammal is known, 21-23 nt RNAs, for example, can be produced and tested for their ability to mediate RNAi in a mammalian cell, such as a human or other primate cell. Those 21-23 nt RNA molecules shown to mediate RNAi can be tested, if desired, in an appropriate animal model to further assess their *in vivo* effectiveness. Target sites that are known, for example target sites determined to be effective target sites based on studies with other nucleic acid molecules, for example ribozymes or antisense, or those targets known to be associated with a disease or condition such as those sites containing mutations or deletions, can be used to design siRNA molecules targeting those sites as well.

Alternatively, the sequences of effective dsRNA can be rationally designed/predicted screening the target mRNA of interest for target sites, for example by using a computer folding algorithm. The target sequence can be parsed *in silico* into a list of all fragments or subsequences of a particular length, for example 23 nucleotide fragments, using a custom Perl script or commercial sequence analysis programs such as Oligo, MacVector, or the GCG Wisconsin Package.

Various parameters can be used to determine which sites are the most suitable target sites within the target RNA sequence. These parameters include but are not limited to secondary or tertiary RNA structure, the nucleotide base composition of the target sequence, the degree of homology between various regions of the target sequence, or the relative position of the target sequence within the RNA transcript. Based on these determinations, any number of target sites within the RNA transcript can be chosen to screen siRNA molecules for efficacy, for example by using in vitro RNA cleavage assays, cell culture, or animal models. *See, e.g.,* U.S. Patent Publication No. 20030170891. An algorithm for identifying and selecting RNAi target sites has also recently been described. *See* U.S. Patent Publication No. 20040236517.

Commonly used gene transfer techniques include calcium phosphate, DEAE-dextran, electroporation and microinjection and viral methods (Graham et al. (1973) Virol. 52: 456; McCutchan et al., (1968), J. Natl. Cancer Inst. 41: 351; Chu et al. (1987), Nucl. Acids Res. 15: 1311; Fraley et al. (1980), J. Biol. Chem. 255: 10431; Capecchi (1980), Cell 22: 479). DNA may also be introduced into cells using cationic liposomes (Feigner et al. (1987), Proc. Natl. Acad. Sci USA 84: 7413). Commercially available cationic lipid formulations include Tfx 50 (Promega Corp., Fitchburg, WI) or Lipofectamin 200 (Life Technologies, Carlsbad, CA). Alternatively, viral vectors may be employed to deliver dsRNA to a cell and mediate RNAi. *See* U.S Patent Publication No. 20040023390.

Transfection and vector/expression systems for RNAi in mammalian cells are commercially available and have been well described. See, e.g., Dharmacon, Inc. (Lafayette, CO), DharmaFECT^{™} system; Promega, Inc., siSTRIKE^{™} U6 Hairpin system; see also Gou et al. (2003) FEBS. 548, 113-118; Sui, G. et al. A DNA vector-based RNAi technology to suppress gene expression in mammalian cells (2002) Proc. Natl. Acad. Sci. 99, 5515-5520; Yu et al. (2002) Proc. Natl. Acad. Sci. 99, 6047-6052; Paul, C. et al. (2002) Nature Biotechnology 19, 505-508; McManus et al. (2002) RNA 8, 842-850.

siRNA interference in a mammal using prepared dsRNA molecules may then be effected by administering a pharmaceutical preparation that includes the dsRNA to the mammal. The pharmaceutical composition is administered in a dosage sufficient to inhibit expression of the target gene. dsRNA can typically be administered at a dosage of less than 5 mg dsRNA per kilogram body weight per day, and is sufficient to inhibit or completely suppress expression of the target gene. In general a suitable dose of dsRNA will be in the range of 0.01 to 2.5 milligrams per kilogram body weight of the recipient per day, preferably in the range of 0.1 to 200 micrograms per kilogram body weight per day, more preferably in the range of 0.1 to 100 micrograms per kilogram body weight per day, even more preferably in the range of 1.0 to 50 micrograms per kilogram body weight per day, and most preferably in the range of 1.0 to 25 micrograms per kilogram body weight per day. A pharmaceutical composition including the dsRNA is administered once daily, or in multiple sub-doses, for example, using sustained release formulations well known in the art. The preparation and administration of such pharmaceutical compositions may be carried out accordingly to standard techniques, as further described below.

Such dsRNA may then be used to inhibit ROS1 expression and activity in a cancer, by preparing a pharmaceutical preparation that includes a therapeutically-effective amount of such dsRNA, as described above, and administering the preparation to a human subject having a lung cancer or suspected lung cancer (*e.g.,* a NSCLC or SCLC) expressing a polypeptide with ROS1 or ALK kinase activity (such as, for example, aberrant expression of full length ROS1 or ALK protein or expression of a ROS1 or ALK fusion protein), for example, via direct injection to the tumor. The similar inhibition of other receptor tyrosine kinases, such as VEGFR and EGFR using siRNA inhibitors has recently been described. See U.S. Patent Publication No. 20040209832, U.S. Patent Publication No. 20030170891, and U.S. Patent Publication No. 20040175703.

ROS1-inhibiting and/or EGFR-inhibiting therapeutics useful in the practice of the methods disclosed herein may be administered to a mammal by any means known in the art including, but not limited to oral or peritoneal routes, including intravenous, intramuscular, intraperitoneal, subcutaneous, transdermal, airway (aerosol), rectal, vaginal and topical (including buccal and sublingual) administration.

For oral administration, a ROS1-inhibiting and/or EGFR-inhibiting therapeutic will generally be provided in the form of tablets or capsules, as a powder or granules, or as an aqueous solution or suspension. Tablets for oral use may include the active ingredients mixed with pharmaceutically acceptable carriers and excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredients is mixed with water or an oil such as peanut oil, liquid paraffin or olive oil. For intramuscular, intraperitoneal, subcutaneous and intravenous use, the pharmaceutical compositions will generally be provided in sterile aqueous solutions or suspensions, buffered to an appropriate pH and isotonicity. Suitable aqueous vehicles include Ringer's solution and isotonic sodium chloride. The carrier may consist exclusively of an aqueous buffer ("exclusively" means no auxiliary agents or encapsulating substances are present which might affect or mediate uptake of the ROS1- and/or EGFR-inhibiting therapeutic). Such substances include, for example, micellar structures, such as liposomes or capsids, as described below. Aqueous suspensions may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

ROS1-inhibiting and/or EGFR-inhibiting therapeutic compositions may also include encapsulated formulations to protect the therapeutic (e.g., a dsRNA compound or an antibody that specifically binds a ROS1 fusion polypeptide or mutant EGFR polypeptide) against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811; PCT publication WO 91/06309; and European patent publication EP-A-43075. An encapsulated formulation may include a viral coat protein. The viral coat protein may be derived from or associated with a virus, such as a polyoma virus, or it may be partially or entirely artificial. For example, the coat protein may be a Virus Protein 1 and/or Virus Protein 2 of the polyoma virus, or a derivative thereof.

ROS1-inhibiting and/or EGFR-inhibiting therapeutics can also include a delivery vehicle, including liposomes, for administration to a subject, carriers and diluents and their salts, and/or can be present in pharmaceutically acceptable formulations. For example, methods for the delivery of nucleic acid molecules are described in Akhtar et al., 1992, Trends Cell Bio., 2, 139; DELIVERY STRATEGIES FOR ANTISENSE OLIGONUCLEOTIDE THERAPEUTICS, ed. Akbtar, 1995, Maurer et al., 1999, Mol. Membr. Biol., 16, 129-140; Hofland and Huang, 1999, Handb. Exp. Pharmacol., 137, 165-192; and Lee et al., 2000, ACS Symp. Ser., 752, 184-192. U.S. Pat. No. 6,395,713 and PCT Publication No. WO 94/02595 further describe the general methods for delivery of nucleic acid molecules. These protocols can be utilized for the delivery of virtually any nucleic acid molecule.

ROS1-inhibiting and/or EGFR-inhibiting therapeutics (*i.e.,* a ROS1- or EGFR-inhibiting compound being administered as a therapeutic) can be administered to a mammalian tumor by a variety of methods known to those of skill in the art, including, but not restricted to, encapsulation in liposomes, by iontophoresis, or by incorporation into other vehicles, such as hydrogels, cyclodextrins, biodegradable nanocapsules, and bioadhesive microspheres, or by proteinaceous vectors (see PCT Publication No. WO 00/53722). Alternatively, the therapeutic/vehicle combination is locally delivered by direct injection or by use of an infusion pump. Direct injection of the composition, whether subcutaneous, intramuscular, or intradermal, can take place using standard needle and syringe methodologies, or by needle-free technologies such as those described in Conry et al., 1999, Clin. Cancer Res., 5, 2330-2337 and PCT Publication No. WO 99/3 1262.

Pharmaceutically acceptable formulations of ROS1-inhibiting and/or EGFR-inhibiting therapeutics include salts of the above-described compounds, *e.g.*, acid addition salts, for example, salts of hydrochloric, hydrobromic, acetic acid, and benzene sulfonic acid. A pharmacological composition or formulation refers to a composition or formulation in a form suitable for administration, *e.g*., systemic administration, into a cell or patient, including for example a human. Suitable forms, in part, depend upon the use or the route of entry, for example oral, transdermal, or by injection. Such forms should not prevent the composition or formulation from reaching a target cell. For example, pharmacological compositions injected into the blood stream should be soluble. Other factors are known in the art, and include considerations such as toxicity and forms that prevent the composition or formulation from exerting its effect.

Administration routes that lead to systemic absorption (*e.g.*, systemic absorption or accumulation of drugs in the blood stream followed by distribution throughout the entire body) are desirable and include, without limitation: intravenous, subcutaneous, intraperitoneal, inhalation, oral, intrapulmonary and intramuscular. Each of these administration routes exposes the ROS1-inhibiting therapeutic to an accessible diseased tissue or tumor. The rate of entry of a drug into the circulation has been shown to be a function of molecular weight or size. The use of a liposome or other drug carrier containing the compounds can potentially localize the drug, for example, in certain tissue types, such as the tissues of the reticular endothelial system (RES). A liposome formulation that can facilitate the association of drug with the surface of cells, such as, lymphocytes and macrophages is also useful. This approach can provide enhanced delivery of the drug to target cells by taking advantage of the specificity of macrophage and lymphocyte immune recognition of abnormal cells, such as cancer cells.

By "pharmaceutically acceptable formulation" is meant, a composition or formulation that allows for the effective distribution of the nucleic acid molecules in the physical location most suitable for their desired activity. Non-limiting examples of agents suitable for formulation with the nucleic acid molecules include: P-glycoprotein inhibitors (such as Pluronic P85), which can enhance entry of drugs into the CNS (Jolliet-Riant and Tillement, 1999, Fundam. Clin. Pharmacol., 13, 16-26); biodegradable polymers, such as poly (DL-lactide-coglycolide) microspheres for sustained release delivery after intracerebral implantation (Emerich et al, 1999, Cell Transplant, 8, 47-58) (Rosermes, Inc. Cambridge, Mass.); and loaded nanoparticles, such as those made of polybutylcyanoacrylate, which can deliver drugs across the blood brain barrier and can alter neuronal uptake mechanisms (Prog Neuro-psychopharmacol Biol Psychiatry, 23, 941-949, 1999). Other non-limiting examples of delivery strategies for the ROS1-inhibiting compounds useful in the methods disclosed herein include material described in Boado et al., 1998, J. Pharm. Sci., 87, 1308-15; Tyler et al., 1999, FEBS Lett., 421, 280-284; Pardridge et al., 1995, PNAS USA., 92, 5592-96; Boado, 1995, Adv. Drug Delivery Rev., 15, 73-107; Aldrian-Herrada et al., 1998, Nucleic Acids Res., 26, 4910-4916; and Tyler et al., 1999, PNAS USA., 96, 7053-7058.

Therapeutic compositions that include surface-modified liposomes containing poly (ethylene glycol) lipids (PEG-modified, or long-circulating liposomes or stealth liposomes) may also be suitably employed in the methods disclosed herein. These formulations offer a method for increasing the accumulation of drugs in target tissues. This class of drug carriers resists opsonization and elimination by the mononuclear phagocytic system (MPS or RES), thereby enabling longer blood circulation times and enhanced tissue exposure for the encapsulated drug (Lasic et al. Chem. Rev. 1995, 95, 2601-2627; Ishiwata et al., Chem. Pharm. Bull. 1995, 43, 1005-1011). Such liposomes have been shown to accumulate selectively in tumors, presumably by extravasation and capture in the neovascularized target tissues (Lasic et al., Science 1995, 267, 1275-1276; Oku et al., 1995, Biochim. Biophys. Acta, 1238, 86-90). The long-circulating liposomes enhance the pharmacokinetics and pharmacodynamics of DNA and RNA, particularly compared to conventional cationic liposomes which are known to accumulate in tissues of the MPS (Liu et al., J. Biol. Chem. 1995, 42, 24864-24870; PCT Publication No. WO 96/10391; PCT Publication No. WO 96/10390; and PCT Publication No. WO 96/10392). Long-circulating liposomes are also likely to protect drugs from nuclease degradation to a greater extent compared to cationic liposomes, based on their ability to avoid accumulation in metabolically aggressive MPS tissues such as the liver and spleen.

Therapeutic compositions may include a pharmaceutically effective amount of the desired compounds in a pharmaceutically acceptable carrier or diluent. Acceptable carriers or diluents for therapeutic use are well known in the pharmaceutical art, and are described, for example, in REMINGTON'S PHARMACEUTICAL SCIENCES, Mack Publishing Co. (A. R. Gennaro edit. 1985). For example, preservatives, stabilizers, dyes and flavoring agents can be provided. These include sodium benzoate, sorbic acid and esters of p-hydroxybenzoic acid. In addition, antioxidants and suspending agents can be used.

In some embodiments, the ROS1-inhibiting therapeutic and/or the EGFR-inhibiting therapeutic is administered in an effective amount. By "effective amount" or "effective dose" is meant the amount of the therapeutic required to prevent, inhibit the occurrence, or treat (alleviate a symptom to some extent, preferably all of the symptoms) of a disease state (e.g., lung cancer). The effective dose depends on the type of disease, the therapeutic used, the route of administration, the type of mammal being treated, the physical characteristics of the specific mammal under consideration, concurrent medication, and other factors that those skilled in the medical arts will recognize. Generally, an effective amount is an amount between 0.1 mg/kg and 100 mg/kg body weight/day of active ingredients is administered dependent upon potency of the negatively charged polymer.

Dosage levels of the order of from about 0.1 mg to about 140 mg per kilogram of body weight per day are useful in the treatment of the above-indicated conditions (about 0.5 mg to about 7 g per patient per day). The amount of active ingredient that can be combined with the carrier materials to produce a single dosage form varies depending upon the host treated and the particular mode of administration. Dosage unit forms generally contain between from about 1 mg to about 500 mg of an active ingredient. It is understood that the specific dose level for any particular patient depends upon a variety of factors including the activity of the specific compound employed, the age, body weight, general health, sex, diet, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

A ROS1-inhibiting and/or EGFR-inhibiting therapeutic useful in the practice of the disclosure may include a single compound as described above, or a combination of multiple compounds, whether in the same class of inhibitor (*e.g*., antibody inhibitor), or in different classes (*e.g.*, antibody inhibitors and small-molecule inhibitors). Such combination of compounds may increase the overall therapeutic effect in inhibiting the progression of a fusion protein-expressing cancer. For example, the therapeutic composition may a small molecule inhibitor, such as Crizotinib (also known as PF-02341066) produced by Pfizer, Inc. (see U.S. Pub. No. 2008/0300273) alone, or in combination with other Crizotinib analogues targeting ROS1 activity and/or small molecule inhibitors of ROS1, such as NVP-TAE684 produced by Novartis, Inc., or the CH5424802 compound described in Sakamoto et al., Cancer Cell 19: 679-690, 2011. The therapeutic composition may also include one or more non-specific chemotherapeutic agent in addition to one or more targeted inhibitors. Such combinations have recently been shown to provide a synergistic tumor killing effect in many cancers. The effectiveness of such combinations in inhibiting ROS1 and/or EGFR activity and tumor growth *in vivo* can be assessed as described below.

The disclosure also provides, in part, methods for determining whether a compound inhibits the progression of a cancer (*e.g.,* a lung cancer) characterized by a polypeptide with ROS1 or ALK kinase activity, a mutant EGFR polypeptide or polynucleotide encoding the same by determining whether the compound inhibits the ROS1, ALK, or EGFR kinase activity of the polypeptide in the cancer. In some embodiments, inhibition of activity of ROS1 or ALK or a mutant EGFR polypeptide is determined by examining a biological sample that includes cells from bone marrow, blood, or a tumor. In another embodiment, inhibition of activity of ROS1 or ALK or mutant EGFR kinase is determined using at least reagent that specifically binds to a ROS1 or ALK polypeptide (e.g., a ROS1-specific antibody or an ALK-specific antibody) or a mutant EGFR polypeptide, or a reagent that specifically binds to a ROS1 or ALK polypeptide- or mutant EGFR polypeptide-encoding polynucleotide (e.g., an siRNA or an antisense).

The tested compound may be any type of therapeutic or composition as described above. Methods for assessing the efficacy of a compound, both *in vitro* and *in vivo,* are well established and known in the art. For example, a composition may be tested for ability to inhibit ROS1 or a mutant EGFR polypeptide *in vitro* using a cell or cell extract in which ROS1 kinase is activated or that expresses a mutant EGFR polypeptide. A panel of compounds may be employed to test the specificity of the compound for ROS1 or EGFR (as opposed to other targets, such as PDGFR).

Another technique for drug screening which may be used provides for high throughput screening of compounds having suitable binding affinity to a protein of interest, as described in PCT Publication No. WO 84/03564. In this method, as applied to polypeptides having ROS1 or ALK activity or mutant EGFR polypeptides, large numbers of different small test compounds are synthesized on a solid substrate, such as plastic pins or some other surface. The test compounds are reacted with a polypeptide disclosed herein, or fragments thereof, and washed. Bound polypeptide is then detected by methods well known in the art. A purified polypeptide can also be coated directly onto plates for use in the aforementioned drug screening techniques. Alternatively, non-neutralizing antibodies can be used to capture the peptide and immobilize it on a solid support.

A compound found to be an effective inhibitor of ROS1 or mutant EGFR activity *in vitro* may then be examined for its ability to inhibit the progression of a cancer expressing a polypeptide with kinase activity (such as lung cancer or other cancer such as a liver cancer, lung cancer, colon cancer, kidney cancer, or a pancreatic cancer), *in vivo,* using, for example, mammalian xenografts harboring human lung, liver, pancreatic, kidney, lung, or colon tumors that express a polypeptide with ROS1 or ALK kinase activity or a mutant EGFR polypeptide. In this procedure, cancer cell lines known to express a protein having ROS1 or ALK kinase activity (e.g., full length ROS1 or ALK or one of the ROS1 or ALK fusion proteins) or a mutant EGFR polypeptide may be placed subcutaneously in an animal (e.g., into a nude or SCID mouse, or other immune-compromised animal). The cells then grow into a tumor mass that may be visually monitored. The animal may then be treated with the drug. The effect of the drug treatment on tumor size may be externally observed. The animal is then sacrificed and the tumor removed for analysis by IHC and western blot. Similarly, mammalian bone marrow transplants may be prepared, by standard methods, to examine drug response in hematological tumors expressing a protein with ROS1 or ALK kinase activity or a mutant EGFR polypeptide. In this way, the effects of the drug may be observed in a biological setting most closely resembling a patient. The drug's ability to alter signaling in the tumor cells or surrounding stromal cells may be determined by analysis with phosphorylation-specific antibodies. The drug's effectiveness in inducing cell death or inhibition of cell proliferation may also be observed by analysis with apoptosis specific markers such as cleaved caspase 3 and cleaved PARP.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. In some embodiments, the compounds exhibit high therapeutic indices.

In practicing the disclosed method for determining whether a compound inhibits progression of a tumor characterized by the presence of a polypeptide with ROS1 kinase activity (or polynucleotide encoding the same), biological samples that include cells from mammalian xenografts (or bone marrow transplants) may also be advantageously employed. Non-limiting xenografts (or transplant recipients) are small mammals, such as mice, harboring human tumors (or leukemias) that express a polypeptide with ROS1 or ALK kinase activity (e.g., a ROS1 or ALK fusion polypeptide or full length ROS1 or ALK) or a mutant EGFR polypeptide. Xenografts harboring human tumors are well known in the art (*see* Kal, Cancer Treat Res. 72: 155-69 (1995)) and the production of mammalian xenografts harboring human tumors is well described (*see* Winograd et al., In Vivo. 1(1): 1-13 (1987)). Similarly the generation and use of bone marrow transplant models is well described (*see, e.g.,* Schwaller, et al., EMBO J. 17: 5321-333 (1998); Kelly et al., Blood 99: 310-318 (2002)).

The following Examples are provided only to further illustrate, and are not intended to limit its scope, except as provided in the claims appended hereto. The present disclosure encompasses modifications and variations of the methods taught herein which would be obvious to one of ordinary skill in the art. Materials, reagents and the like to which reference is made are obtainable from commercial sources, unless otherwise noted.

### EXAMPLE 1

### Detection of ROS1 Kinase Protein by Immunohistochemistry (IHC)

ROS1 fusion proteins have previously been described in NSCLC cell lines and NSCLC human tumor samples (as well as in other tissues such as liver cancer and brain cancer). To determine whether or not the ROS1 fusion proteins discovered in NSCLC could be detected by immunohistochemistry, a ROS1-specific rabbit monoclonal antibody was used. The ROS1-specific antibody (namely rabbit monoclonal antibody ROS1 D4D6) that was used in these studies has been described previously (see PCT Publication No. WO2010/093928), and specifically binds a region on the human ROS1 kinase protein that is C-terminal to the kinase domain of the ROS1 protein. While the D4D6 antibody is not yet commercially available, similar ROS1-specific antibodies are commercially available from a variety of suppliers including, without limitation, the Ros (C-20) antibody, Catalog No. sc-6347 from Santa Cruz Biotechnology, Inc., (Santa Cruz, CA) and the ROS1 (69D6) antibody, Catalog No #3266 from Cell Signaling Technology, Inc. (Danvers, MA).

For these studies, a cohort of 556 human samples of NSCLC tumors were prepared as paraffin blocks. All tumor samples were evaluated by a pathologist, and were found to comprise 246 adenocarcinoma, 64 bronchioaveolar carcinoma, 226 squamous and 20 large cell carcinoma cases. The identifications of selected samples, including ROS1 and mutant-EGFR positive samples, were confirmed by an independent pathologist.

Immunohistochemistry: 4-6 µm tissue sections were deparaffinized and rehydrated through xylene and graded ethanol, respectively (e.g., through three changes of xylene for 5 minutes each, then rehydrated through two changes of 100% ethanol and 2 changes of 95% ethanol, each for 5 minutes). Slides were rinsed in diH₂O, then subjected to antigen retrieval in a Decloaking Chamber (Biocare Medical, Concord, CA) using 1.0 mM EDTA, pH 8.0 and manufacturer's settings: SP1 125°C for 30 seconds and SP2 90°C for 10 seconds. Slides were quenched in 3% H₂O₂ for 10 minutes, then washed in diH₂O. After blocking in Tris buffered saline positive 0.5% Tween-20 (TBST)/5% goat serum in a humidified chamber, slides were incubated overnight at 4°C with ROS1 (D4D6) XP^{™} Rabbit mAb at 0.19 µg/ml diluted in SignalStain^{®} Antibody Diluent (#8112 Cell Signaling Technology, Danvers, MA). After washing with TBST, detection was performed with either ENVISION+ (Dako, Carpinteria, CA) or SIGNALSTAIN^{®} Boost IHC Detection Reagent (HRP, Rabbit) (catalog #8114 Cell Signaling Technology, Danvers, MA) with a 30 minute incubation at room temperature in a humidified chamber. After washing the slides (e.g., three times in TBST) the slides were next exposed to NovaRed (Vector Laboratories, Burlingame, CA) prepared per the manufacturer's instructions.

Slides were developed for 1 minute and then rinsed in diH₂O. Slides were counterstained by incubating in hematoxylin (ready to use commercially available from Invitrogen (Carlsbad, CA) Catalog #00-8011) for 1 minute, rinsed for 30 seconds in diH₂O, incubated for 20 seconds in bluing reagent (Richard Allan Scientific, Kalamazoo, MI (a Thermo Scientific company), Catalog #7301), and then finally washed for 30 seconds in diH₂O. Slides were dehydrated in 2 changes of 95% ethanol for 20 seconds each and 2 changes of 100% ethanol for 2 minutes each. Slides were cleared in 2 changes of xylene for 20 seconds each, then air dried. Coverslips were mounted using VectaMount (Vector Laboratories, Burlingame, CA). Slides were air dried, then evaluated under the microscope. Images (20x) were acquired using an Olympus CX41 microscope equipped with an Olympus DP70 camera and DP Controller software.

Out of the 556 NSCLC tumors screened by immunohistochemistry with the ROS1-specific Rmab ROS1 D4D6, 9 ROS1-postive tumors were identified. The breakdown was as follows:
Of the 246 adenocarcinomas, 8 (or 3.3%) were positive for ROS1 kinase.
Of the 20 large cell carcinomas, 1 (or 5.0%) were positive for ROS1 kinase.

A variety of ROS1 IHC staining patterns ranging from weak cytoplasmic to strong perinuclear aggregates were observed (see Figs. 1A-F). In 5/9 (55%) cases ROS1 localized diffusely in the cytoplasm (Fig. 1A). Strong cytoplasmic staining was observed in 1 large cell carcinoma (Fig. 1C). Two cases had unique phenotypes distinct from each other with one being diffuse cytoplasmic with areas of punctate plasma membrane staining (Fig. 1D) and the other vesicular staining throughout (Fig. 1F). It should also be noted that in rare cases non-neoplastic cells such as macrophages and bronchial epithelial cells stained with ROS1 D4D6. ROS1 expression was absent in the surrounding stromal tissue.

### EXAMPLE 2

### Detection of a ROS1 Fusion in Human Cancer Samples Using FISH Assay

The presence of either the SLC34A2-ROS1 fusion protein and/or the CD74-ROS1 protein (or another ROS1 fusion protein) in human NSCLC tumor samples was detected using a fluorescence in situ hybridization (FISH) assay, as previously described. See, e.g., Verma et al. HUMAN CHROMOSOMES: A MANUAL OF BASIC TECHNIQUES, Pergamon Press, New York, N.Y. (1988). Over 200 paraffin-embedded human NSCLC tumor samples were examined.

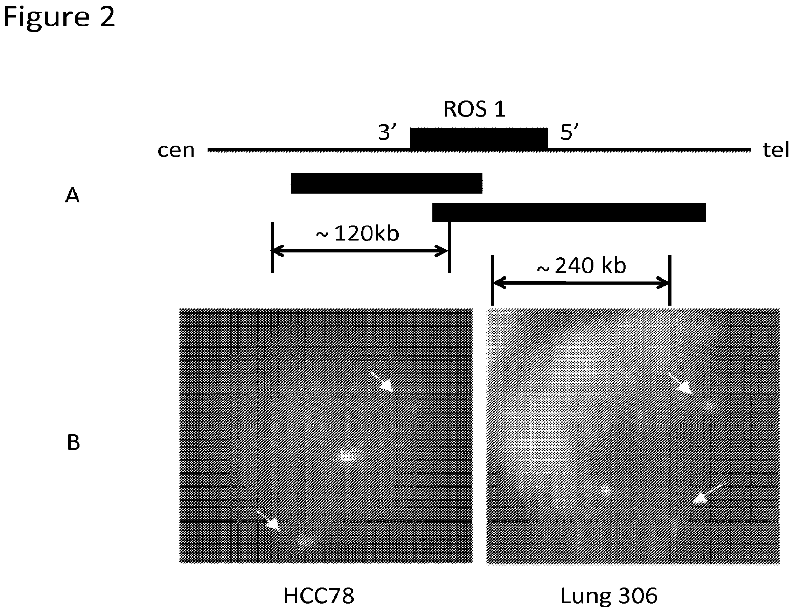

For analyzing rearrangements involving ROS1, a dual color break-apart probe was designed. A proximal probe (BAC clone RP1-179P9) and two distal probes (BAC clone RP11-323017, RP1-94G16) (all of which are commercially available, for example, from Invitrogen Inc., Carlsbad, CA, as Catalog Nos. RPCI1.C and RPCI11.C ) were obtained. The locations at which these probes bind to the ROS1 gene are shown schematically in Fig. 2. As shown in Fig. 2A, the proximal probe was labeled with Spectrum Orange dUTP, and the distal probes were labeled with Spectrum Green dUTP. Labeling of the probes was done with the Nick Translation DNA Labeling Kit according to manufacturer's instructions (Enzo Life Sciences, Farmingdale, NY). FISH was performed on 4-µm thick FFPE tissue sections according to standard methods. For example, the paraffin embedded tissue sections were re-hydrated and subjected to microwave antigen retrieval in 0.01M Citrate buffer (pH 6.0) for 11 minutes. Sections were digested with Protease (4mg/ml Pepsin, 2000-3000U/mg) for 25 minutes at 37°C, dehydrated and hybridized with the FISH probe set at 37°C for 18 hours. After washing, 4',6-diamidino-2-phenylindole (DAPI; mg/ml) in Vectashield mounting medium (Vector Laboratories, Burlingame, CA) was applied for nuclear counterstaining.

FISH-positive cases for ROS1 were defined as >15% split signals in tumor cells. The Nikon C1 Confocal microscope, 60 X objective and trifilter (dapi, TRITC, FITC) was used for scoring each case. For image acquisition the Olympus BX-51 widefield fluorescence microscope with 40 X objective and Metamorph software was used to generate tricolor images.

Thus, the ROS1 rearrangement probe contains two differently labeled probes on opposite sides of the breakpoint of the ROS1 gene in the wild type (WT) sequence (see Figure 15A). When hybridized, the native ROS1 region will appear as an orange/green fusion signal, while rearrangement at this locus (as occurs in the SLC34A2-ROS1 fusion protein) will result in separate orange and green signals.

As shown in Figure 2B, a rearranged ROS1 gene was found in HCC78 (Fig. 2B, left panel) which, as described above, contains a gene rearrangement resulting in the SLC34A2-ROS1 fusion. In one of the human lung samples, namely lung 306, a similar ROS1 gene rearrangement was found which may be SLC34A2-ROS1 or CD74-ROS1.

The FISH analysis revealed a low incidence of this ROS1 mutation in the sample population studied. Of the initial 123 tumors screened, two out of 123 tumors or 1.6% of tumors contained the ROS1 fusion mutations. However, given the high incidence of NSCLC worldwide (over 151,00 new cases in the U.S. annually, alone), there are expected to be a significant number of patients that harbor this mutant ROS1, which patients may benefit from a ROS1-inhibiting therapeutic regime.

### Example 3

### Discovery of FIG-ROS1 positive NSCLC tumor

From Example 1, one of the tumor samples, namely Tumor 749, showed ROS1 staining that was localized to vesicular compartments (see Fig. IF). This staining pattern is distinct from all other ROS1 positive tumors, which pointed to the possibility of a different ROS1 fusion partner.

To determine what the FISH pattern of this Tumor 749 was, a third distal probe RP11-213A17, was obtained from Invitrogen to further investigate whether the ROS1 mutation in this tumor might be due to a FIG-ROS1 fusion. Fusions between the FIG gene and the ROS1 gene have been described in glioblastoma, cholangiocarcinoma, and liver cancer (see Charest et al., Genes Chromosomes Cancer 37: 58-71, 2003; Charest et al., Proc. Natl. Acad. Sci. USA 100: 916-921, 2003; and PCT Publica NO. WO2010/093928), but this fusion has never been described in lung before. Since the fusion between the FIG gene and the ROS1 gene results not a translocation or inversion but, rather, results from an intrachromosomal deletion on chromosome 6 of 240 kilobases, a new set of FISH probes was designed.

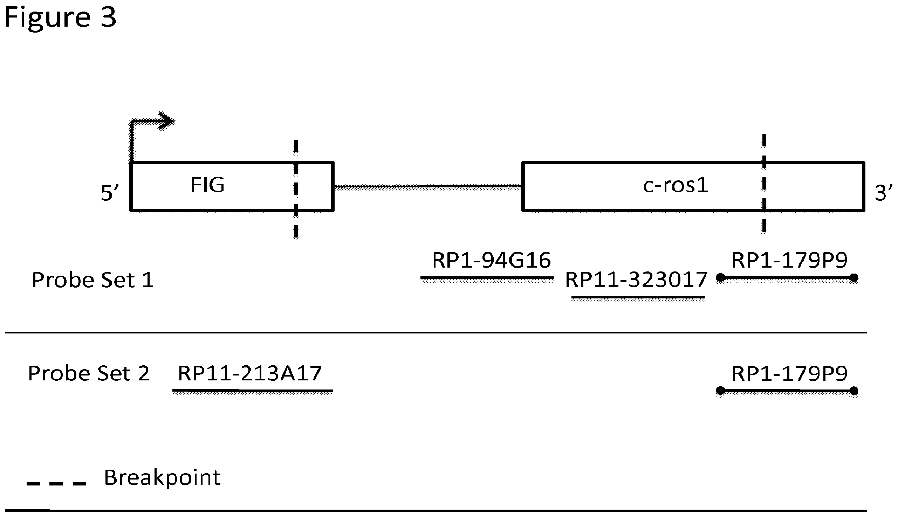

The FISH probes used in the IHC confirmation testing described previously (see Example 2 above) identified those tumors and cells with ROS1 balanced translocations that could be due to the presence of one of the SLC34A2-ROS1 fusion protein or the CD74-ROS1 fusion protein. The FISH pattern in lung 749 suggested that the rearrangement was not one of these two fusions but potentially that of FIG-ROS1. To determine if lung ID 749 was indeed FIG-ROS1 positive, another FISH probe set was designed (Fig. 3). As described above in Example 2, Probe set 1 containing 179P9 and 323017 BACs flanked either side of the ROS1 breakpoint in the ROS1 fusion proteins described herein (e.g., after exon 34, 35, or 36 of ROS1) (see Fig. 3 and Fig. 2A). In SLC34A-ROS1 positive HCC78 cells (see Fig. 2B, left panel and Fig. 4A), probe set 1 results in a balanced translocation. In the FIG-ROS1 positive human U118MG glioblastoma cell line, the 323017 BAC did not hybridize, since this section of chromosome 6 is deleted, resulting in only orange signals (Fig. 4C). Probe set 2 contained 179P9 located on ROS1 and 213A7 located on the FIG gene, thus U118MG shows both orange and green signals with this probe set (see Fig. 4D). HCC78 cells showed 1 chromosome with a balanced translocation (e.g., from a SLC34A2-ROS1 fusion; see the two yellow arrows in Fig. 4B) and the white arrow in Fig. 4B points to a normal chromosome with the green and orange signals close together since the FIG gene and the ROS1 gene are, in fact, close together on the same chromosome (see Fig. 4B). The wild-type chromosome displayed a separated signal due to the distance between the probes. Lung ID 749, when probed with either probe set 1 (Fig. 4E) or probe set 2 (see Fig. 4F), mimicked that of U118MG cells (Figs. 4C and D). These data were the first to shown the FIG-ROS1 fusion as an intrachromosomal deletion on chromosome 6 in NSCLC.

### Example 4

### Isolation & Sequencing of the FIG-ROS1(S) Fusion Gene from Lung tumor 749

To isolate and sequence the ROS1 fusion from tumor 749 (which was a_Formalin-Fixed, Paraffin-Embedded Tumor), the following protocol was used.

RT-PCR from FFPE tumor samples: RNA from 3 × 10 µm sections was extracted following standard protocols (RNeasy FFPE Kit, Qiagen). First strand cDNA was synthesized from 500 ng of total RNA with the use of SuperScript III first strand synthesis system (Invitrogen) with gene specific primers. Then the FIG-ROS1 fusion cDNA was amplified with the use of PCR primer pairs FIG-F3 and ROS1-GSP3.1 for the short isoform and FIG-F7 and ROS1-GSP3.2 for the long isoforms. GAPDH primers were purchased from Qiagen (Valencia, CA).

### Primers

ROS1-GSP3.1: CAGCAAGAGACGCAGAGTCAGTTT (SEQ ID NO: 18)
ROS1-GSP3.2: GCAGCTCAGCCAACTCTTTGTCTT (SEQ ID NO: 10)
FIG-F3: GCTGTTCTCCAGGCTGAAGTATATGG (SEQ ID NO: 19)
FIG-F7: GTAACCCTGGTGCTAGTTGCAAAG (SEQ ID NO: 20)

The primers for FIG were selected because based on the FISH patterns observed in tumor 749 and the published information on the FIG-ROS1 fusion, tumor 749 was expected to be a FIG-ROS1 fusion.

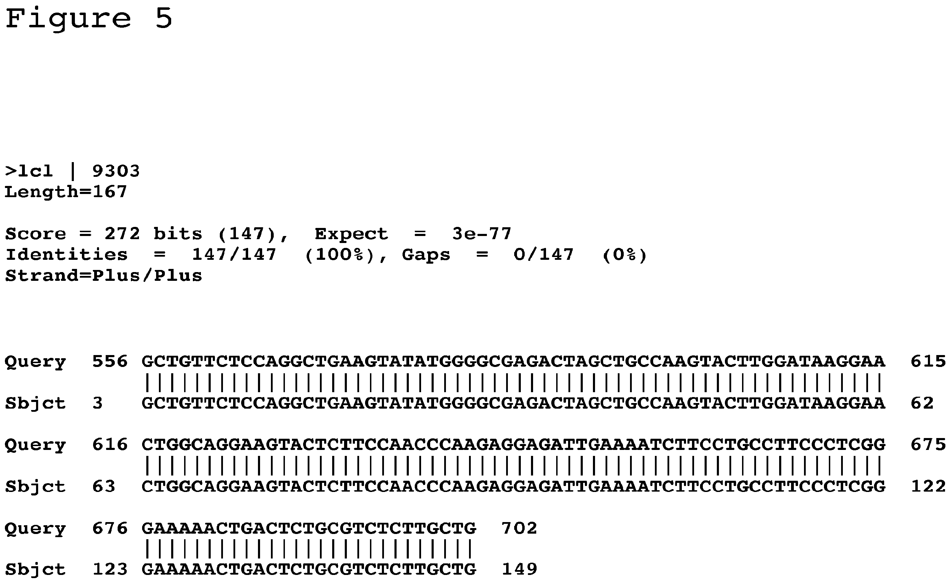

As predicted, the ROS1 fusion protein in tumor 749 was indeed a FIG-ROS1 fusion, specifically the FIG-ROS1 (S) fusion previously described (see PCT Publication No. WO2010/0923828). Figure 5 shows an alignment of the sequence from the FFPE block from tumor 749 (in the "sbjct" line) with the sequence from the FIG-ROS1(S) described in PCT Publication No. WO2010/0923828 (in "query" line). As shown in Figure 5, the identity was 100% with 0 gaps. Since FIG-ROS1(S) contains the entire kinase domain of ROS1 kinase, this FIG-ROS1(S) is expected to retain kinase activity and, thus, is a protein with ROS1 kinase activity as described herein.

The amino acid sequence of FIG-ROS1(S) is set forth in SEQ ID NO: 24 and the nucleotide sequence of FIG-ROS1(S) is set forth in SEQ ID NO: 23.

FIG-ROS1(L) in liver cancer has also been described (see PCT Publication No. WO2010/0923828). The amino acid and nucleotide sequence of FIG-ROS1(L) is set forth in SEQ ID NOs 22 and 21, respectively. In addition, based on analysis of the gene structure of the FIG and the ROS1 genes, a third FIG-ROS1 variant (namely FIG-ROS1(XL) has been proposed (see PCT Publication No. WO2010/0923828). The amino acid and nucleotide sequence of FIG-ROS1(XL) is set forth in SEQ ID NOs 26 and 25, respectively. Given this finding of FIG-ROS1(S) in NSCLC, other variants of FIG-ROS1 fusion protein may also be found in NSCLC.

### EXAMPLE 5

### Detection of ROS1 Kinase Expression in a Human Lung Cancer Sample Using PCR Assay

The presence of aberrantly expressed full length ROS1 protein or a ROS1 fusion protein (e.g., one of the SLC34A2-ROS1 fusion proteins, CD74-ROS1 fusion protein, or one of the FIG-ROS1 fusion proteins) in a human lung cancer sample may be detected using either genomic or reverse transcriptase (RT) polymerase chain reaction (PCR), previously described. See, e.g., Cools et al., N. Engl. J. Med. 348: 1201-1214 (2003).

Briefly and by way of example, tumor or pleural effusion samples may be obtained from a patient having NSCLC using standard techniques. PCR probes against truncated ROS1 kinase, SLC34A2-ROS1 fusion protein, CD74-ROS1, or FIG-ROS1 are constructed. RNeasy Mini Kit (Qiagen) may be used to extract RNA from the tumor or pleural effusion samples. DNA may be extracted with the use of DNeasy Tissue Kit (Qiagen). For RT-PCR, first-strand cDNA is synthesized from, e.g., 2.5 mg of total RNA with the use, for example, of SuperScript^{™} III first-strand synthesis system (Invitrogen) with oligo (dT)₂₀. Then, the ROS1 gene or ROS1 fusion gene (e.g., SLC34A2-ROS1, CD74-ROS1, or FIG-ROS1) is amplified with the use of primer pairs, e.g. SLC34A2-F1 and ROS1-P3 (see Example 5 above). For genomic PCR, amplification of the fusion gene may be performed with the use of Platinum Taq DNA polymerase high fidelity (Invitrogen) with primer pairs, e.g. SLC34A2-F1 and ROS1-R1, or SLC34A2-F1 and ROS1-R2.

Such an analysis will identify a patient having a cancer characterized by expression of the truncated ROS1 kinase (and/or ROS1 fusion protein such as FIG-ROS1, SLC34A2-ROS1, or CD74-ROS1), which patient is a candidate for treatment using a ROS1-inhibiting therapeutic.

### Example 6

### Sensitivity of ROS1 Kinase Fusions to TAE-684 and Crizotinib

The small molecule, TAE-684, a 5-chloro-2,4-diaminophenylpyrimidine, inhibits the ALK kinase. The structure of TAE-684 is provided in Galkin, et al., Proc. National Acad. Sci 104(1) 270-275, 2007, incorporated by reference. Another small molecule, namely crizotinib, also inhibits the ALK kinase, as well as the MET kinase. The structure of crizotinib (also called PF-02341066) is provided in Zou HY et al., Cancer Research 67: 4408-4417, 2007 and U.S. Patent Publication No. 20080300273, incorporated by reference.

Whether TAE-684 and/or crizotinib also inhibits kinase activity of ROS1 fusion polypeptides was determined.

BaF3 and Karpas 299 cells were obtained from DSMZ (Deutsche Sammlung von Mikroorganismen und Zellkulturen GmbH, Germany). BaF3 cells, which need interleukin-3 to survive, were maintained at 37°C in RPMI-1640 medium (Invitrogen) with 10% fetal bovine serum (FBS) (Sigma) and 1.0 ng/ml murine IL-3 (R&D Systems). Karpas 299 cells (a lymphoma cell line) were grown in RPMI- 1640 with 10% FBS.

BaF3 cells were transduced with retrovirus encoding FIG-ROS1(S), FIG-ROS1(L), or FLT- 3ITD (the Internal tandem duplication mutation in FLT3 causes AML leukemia), and selected for IL3 independent growth. Karpas 299 cells, which express NPM-ALK, was used as a positive control. Retroviruses were generated as previously described (see PCT Publication No. WO 2010/093928, incorporated by reference).

A MTS assay was performed using the CellTiter 96 Aqueous One Solution Reagent (Promega, Catalog No G3582). Briefly, 1 × 10⁵ cells/well in 24 well plates were grown in 1 mL medium that included 0 nM, 3 nM, 10 nM, 30 nM, 100 nM, 300 nM or 1000 nM TAE-684. After 72 hours, 20 µl of the CellTiter 96 Aqueous One Solution Reagent was added into each well of a 96 well assay plate (flat bottom), and then 100 µl of cells grown with or without treatment. Media-only wells were used as controls. The 96 well plate was incubated for 1-4 hours at 37 °C, and then viable cells were counted by reading the absorbance at 490 nm using a 96 well plate reader.

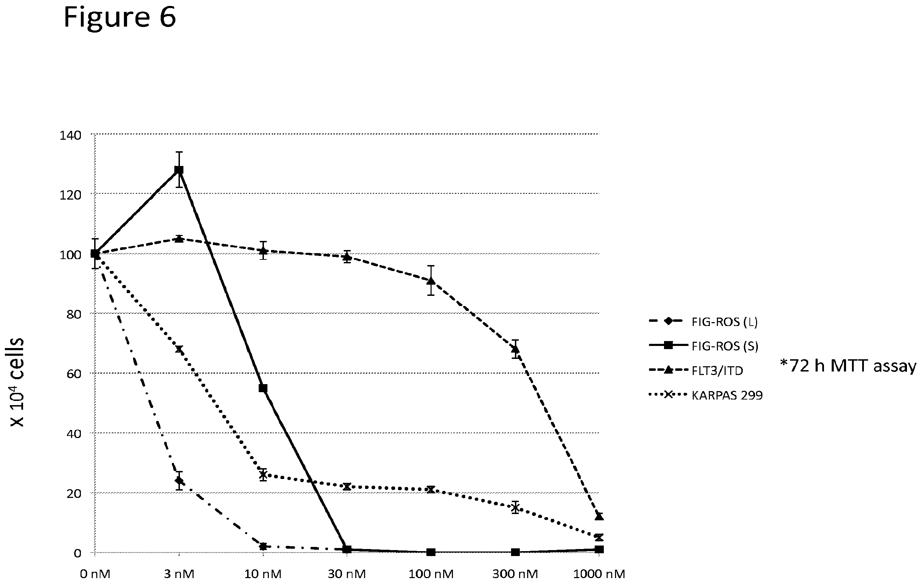

As shown in Fig. 6, the BaF3 cells transduced with retrovirus expressing one of the FIG-ROS1 polypeptides stopped growing in the presence of TAE-684. FIG-ROS1(S) was less susceptible to TAE-684 than FIG-ROS1(L). Karpas 299 cells also responded (i.e., stopped growing) in the presence of TAE-684. The BaF3 cells transduced with FLT3/ITD were not susceptible to TAE-684. The IC50 values from two experiments are as follows in Table 4, with data from a final cell line, namely BaF3 cells expressing myc-tagged neomycin, available only in the second experiment.

**Table 4**

| TAE-684 | IC50 | IC50 |
|---|---|---|
| FIG-ROS1 (L) | 1.78 nM | 2.84 nM |
| FIG-ROS1 (S) | 10.16 nM | 15.01 nM |
| FLT3/ITD | 419.35 nM | 316.44 nM |
| Neo-Myc | NA | 1641.84 nM |
| Karpas-299 | 4.85 nM | 4.36 nM |

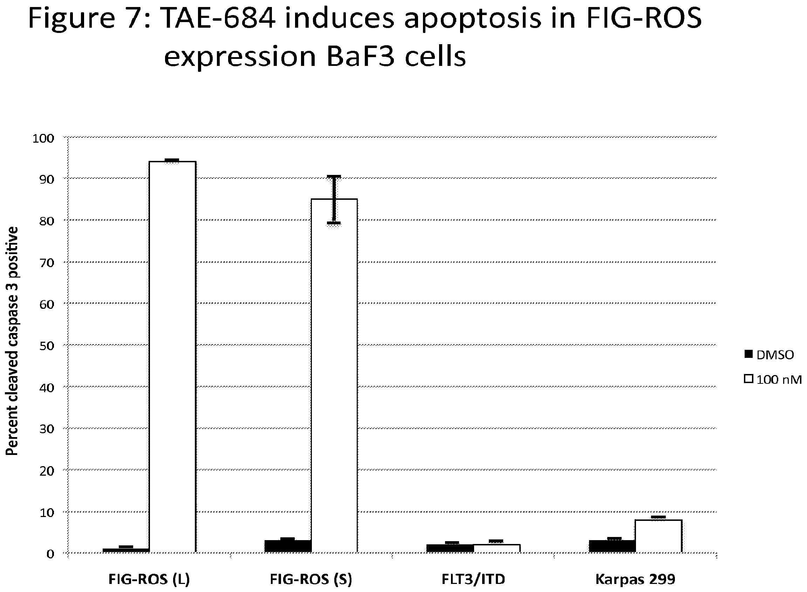

The mechanism of death of the BaF3 and Karpas 299 cells was next assessed by measuring the percentage of cleaved-caspase 3 positive cells by flow cytometry assay using cleaved caspase-3 as a marker for apoptosis. These results were obtained using the protocol publicly available from Cell Signaling Technology, Inc. (Danvers, MA). As shown in Fig. 7, the presence of TAE-684 caused the BaF3 cells expressing FIG-ROS1(S) or FIG-ROS1(L) to die by apoptosis. Karpas 299 cells, which stopped growing in the presence of TAE-684, did not die by apoptosis - they simply underwent cell cycle arrest. Thus, the mechanism by which TAE-684 inhibits FIG-ROS1 fusion polypeptides is different from the mechanism by which TAE-684 inhibits the ALK kinase.

To further identify the mechanism of action of TAE-684 on the FIG-ROS1 fusion polypeptides, all four cell lines (i.e., Karpas 299 cells and BaF3 cells transduced with retrovirus encoding FIG-ROS1(S), FIG-ROS1(L), and FLT-3ITD) were subjected to western blotting analysis following treatment with 0, 10, 50, or 100 nM TAE-684 for three hours. All antibodies were from Cell Signaling Technology, Inc. (Danvers, MA)

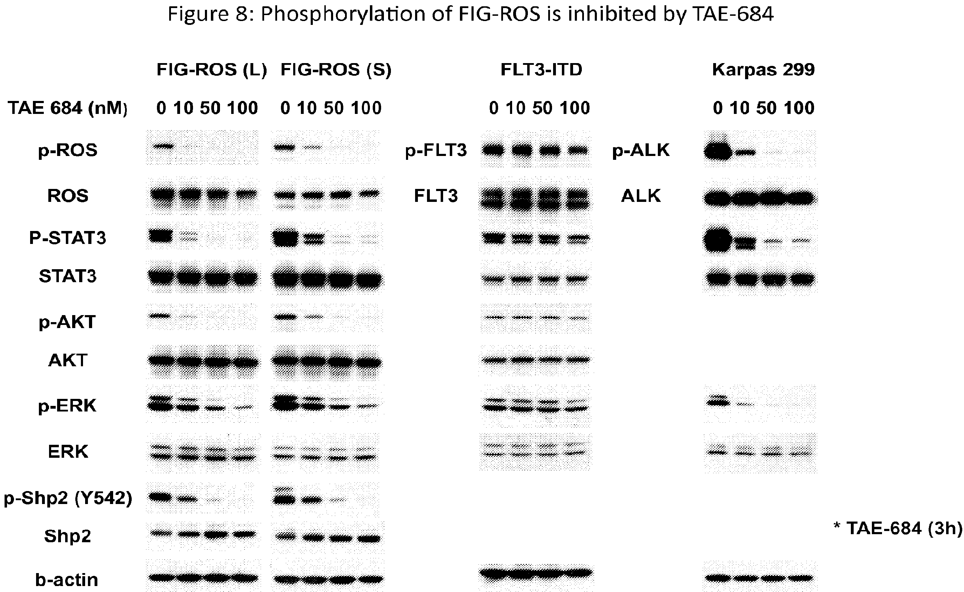

As shown in Fig. 8, phosphorylation of both FIG-ROS1(S) and FIG-ROS1(L) in FIG-ROS1(S) and FIG-ROS1(L) expressing BaF3 cells was inhibited by TAE-684. In addition, phosphorylation of STAT3, AKT, and ERK, and Shp2 were inhibited in FIG-ROS1(S) and FIG-ROS1(L) expressing BaF3 cells. The phosphorylation of STAT3, AKT, and ERK, and Shp2 was not affected in the BaF3 cells transduced with the FLT-3ITD retrovirus. TAE-684 also inhibited ALK and ERK phosphorylation in Karpas 299 cells. Since ROS1, ALK, LTK, InsR, and IGF1R belong to the same family of tyrosine kinases, they may share similar structure in the kinase domain. Kinase inhibitors or antibodies designed against ALK, LTK, InsR, and IGF1R may have therapeutic effects against ROS1 kinase.

A parallel set of experiments was next done on the same cells using the same protocols with the addition of another negative control, namely BaF3 cells transduced with the neo-myc tag, to compare two ALK therapeutics, namely TAE-684 and crizotinib.

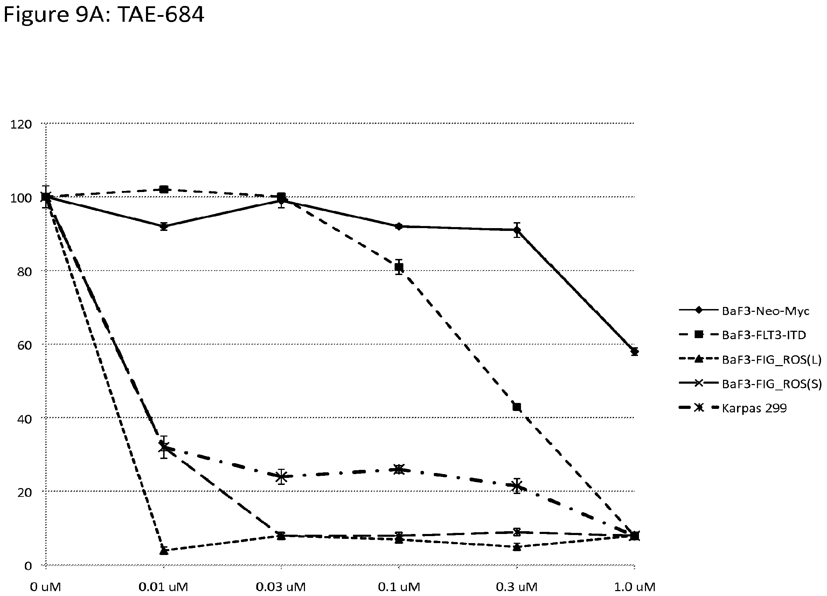

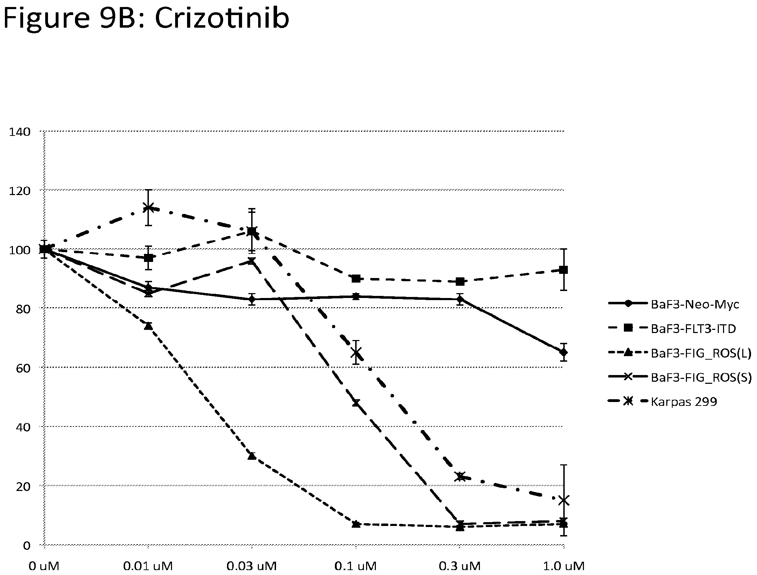

As shown in Fig. 9A (TAE-684) and Fig. 9B (crizotinib), the FIG-ROS1 fusion protein-containing BaF3 cells were more sensitive to TAE-684 than to crizotinib at the same concentration of each therapeutic. It may be that crizotinib is not as effective as a similar dose of TAE-684, since even the positive control, namely the NPM-ALK fusion protein-expressing Karpas 299 cells, were not sensitive to crizotinib as compared to TAE-684 at the same concentrations. Both of the negative controls (i.e., BaF3 transduced with FLT3-ITD or BaF3 transduced with neo-myc) were less sensitive to crizotinib and to TAE-684 than the FIG-ROS1 protein-expressing BaF3 cells and the NPM-ALK protein-expressing Karpas 299.

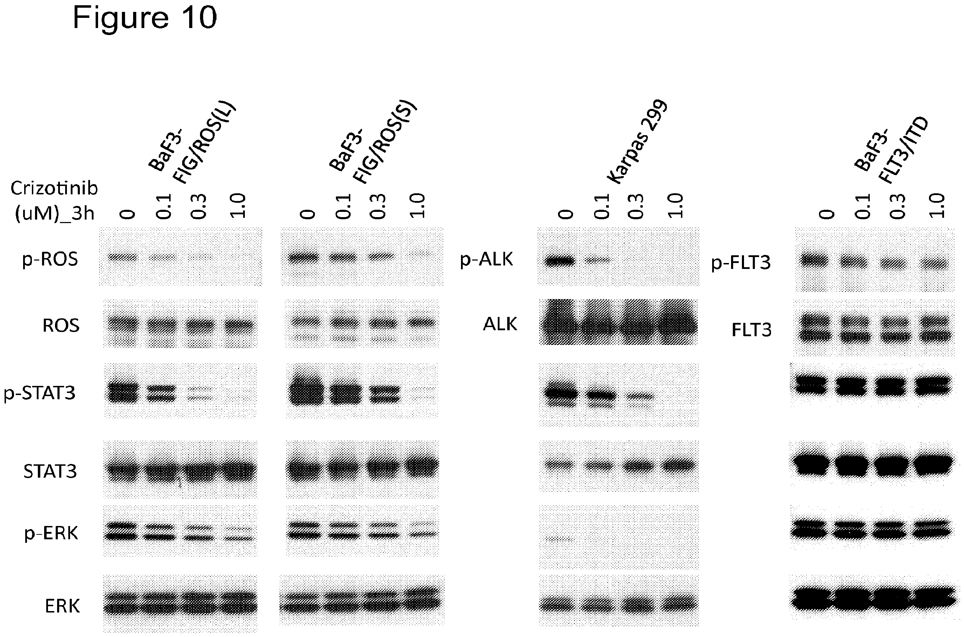

Western blotting analysis following treatment with 0, 0.1, 0.3, or 1.0 uM crizotinib for three hours was next performed using antibodies available from Cell Signaling Technology, Inc. As shown in Figure 10, phosphorylation of both FIG-ROS1(S) and FIG-ROS1(L) in FIG-ROS1(S) and FIG-ROS1(L) expressing BaF3 cells was inhibited by crizotinib. In addition, phosphorylation of STAT3 and ERK, were inhibited by crizotinib in FIG-ROS1(S) and FIG-ROS1(L) expressing BaF3 cells. The phosphorylation of STAT3 and ERK was not affected in the BaF3 cells transduced with the FLT-3ITD retrovirus following crizotinib treatment. Crizotinib also inhibited ALK, STAT3 and ERK phosphorylation in Karpas 299 cells. Since ROS1, ALK, LTK, InsR, and IGF1R belong to the same family of tyrosine kinases, they may share similar structure in the kinase domain. Kinase inhibitors or antibodies designed against ALK, LTK, InsR, and IGF1R may have therapeutic effects against ROS1 kinase.

### Example 15

### Survey of NSCLC expressing ALK and/or ROS1.

In addition to ROS1 kinase, NSCLC have also been described which contain proteins having ALK activity (see, e.g., US Patent Nos. 7,700,339; 7,605,131; 7,728,120). Using the IHC methods described above in Example 1, numerous FFPE samples of human NSCLC tumors were screened for specific binding by anti-ROSl or anti-ALK antibodies. Such antibodies are commercially available from numerous sources.

The same samples were also screened with FISH for the ROS1 gene or for the ALK gene using standard methods. For example, a FISH protocol for the ROS1 gene is described in the Examples above. A FISH protocol for the ALK is described in U.S. Patent No. 7,700,339, herein incorporated by reference. Likewise, another FISH assay is described in US Patent Publication No. 20110110923, incorporated herein by reference). The results of the screening are shown below in Tables 5 (ROS1 positive samples) and 6 (ALK positive samples).

**Table 5 Histopathology of ROS1 positive samples**

| **Patient No.** | **Tumor ID** | **Diagnosis** | **Histologic pattern (%)** | **IHC Score** | ***ROS1* FISH** |
|---|---|---|---|---|---|
| 1 | 147 | Adenocarcinoma | BAC (40), papillary (30), Acinar (20), Solid (10) | 3+ | + |
| 2 | 306 | Adenocarcinoma | Acinar (70), papillary (20), and solid (10) | 3+ | + |
| 3 | 570 | Adenocarcinoma | Acinar (90), BAC (5), micropapillary (5) | 3+ | + |
| 4 | 400037 | Adenocarcinoma | Acinar | 2+ | + |
| 5 | 668 | Adenocarcinoma | Solid (80), Acinar (10), BAC (10) | 1+ | + |
| 6 | 702 | Adenocarcinoma | Papillary (40), Acinar (30), Solid (30) | 1+ | + |
| 7 | 749 | Adenocarcinoma | Solid (80), Acinar (20) | 1+ | +, green deletion |
| 8 | 760 | Adenocarcinoma | Signet cells | 3+ | + |
| 9 | 575 | Large Cell | | 2+ | Not scoreable |

**Table 6: Histopathology of ALK positive cases.**

| **Patient No.** | **Tumor ID** | **Diagnosis** | **Histologic Pattern (%)** | ***ALK* FISH** |
|---|---|---|---|---|
| 1 | 187 | Adenocarcinoma | Solid Focal signet cell ring features | + |
| 2 | 307 | Adenocarcinoma | BAC (30), Acinar (10), papillary (10), solid (50) clear cell and mucinous features | + |
| 3 | 587 | Adenocarcinoma | Acinar (85), solid (10), papillary (5) | Not scoreable |
| 4 | 618 | Adenocarcinoma | Solid | + |
| 5 | 645 | Adenocarcinoma | Solid (70), BAC (30) | + |
| 6 | 652 | Adenocarcinoma | Papillary (60), Micropapillary (40) | + |
| 7 | 663 | Adenocarcinoma | Papillary (50) BAC (50) | + |
| 8 | 664 | Adenocarcinoma | Acinar | + |
| 9 | 666 | Adenocarcinoma | Solid (90), Papillary (10) | + |
| 10 | 670 | Adenocarcinoma | Solid (60), Papillary (40) | + |
| 11 | 680 | Adenocarcinoma | Solid (70) and acinar (30) with signet ring cell features | + |
| 12 | 759 | Adenocarcinoma | Solid with signet ring cells | + |
| 13 | 580 | Adenocarcinoma (uncertain) | | + |
| 14 | 70 | Adenocarcinoma | Solid | + |
| 15 | 383 | Adenocarcinoma | BAC (40), papillary (30), Acinar (30) | + |
| 16 | 395 | Adenocarcinoma | Solid | + |
| 17 | 278 | Squamous; large cell carcinoma (uncertain) | | + |
| 18 | 330 | Large cell neuroendocrine carcinoma | | + |
| 19 | 503 | Squamous | | + |
| 20 | 615 | Squamous | | + |
| 21 | 644 | Squamous | | + |
| 22 | 691 | Squamous | | + |

Based on this screening of human NSCLC by both IHC and by FISH, it was found that ALK and ROS1 expression in these tumors is mutually exclusive. In other words, if an NSCLC tumor is driven by ALK, it will not express ROS1. Likewise, if an NSCLC tumor is driven by ROS1, it will not express ALK. Thus, a therapeutic such as crizotinib or TAE-684 that inhibits both ROS1 activity and ALK activity will be particularly effective in treating NSCLC.

### Example 16

### Analysis of EGFR mutations in ROS1 and ALK positive NSCLC tumors.

The mutational status of all ROS1 and ALK positive tumors in the patient cohorts was examined using IHC with mutation specific EGFR antibodies (EGFR L858R and EGFR E746-A750del (Yu et al., 2009, Clin. Cancer Res., 15:3023-28)). The slides were stained with EGF Receptor (L858R Mutant Specific) (43B2) Rabbit mAb (1.2 µg/ml), EGF Receptor (E746-A750del Specific) (6B6) XP^{®} Rabbit mAb (8.5 µg/ml) or EGF Receptor (D38B1) XP^{®} Rabbit mAb (0.28 µg/ml), (#3197, #2085 and #4267, respectively, all Cell Signaling Technology, Danvers, MA) all diluted in SignalStain^{®} Antibody Diluent (#8112 Cell Signaling Technology, Danvers, MA), incubated for 1 hour at room temperature, washed, then incubated with SignalStain^{®} Boost IHC Detection Reagent (HRP, Rabbit) (Cell Signaling Technology, #8114) for 30 minutes. All slides were exposed to NOVARED substrate (Vector Laboratories, Burlingame, CA), and coverslips were then mounted. Images (20x) were acquired using an Olympus CX41 microscope equipped with an Olympus DP70 camera and DP Controller software.

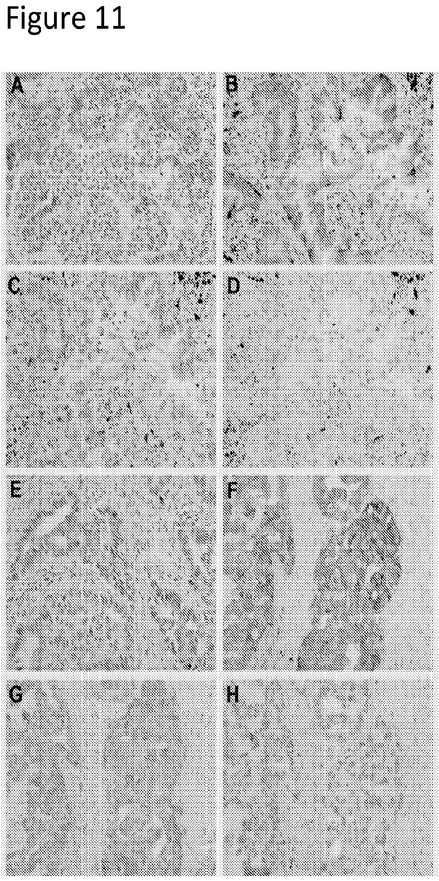

As expected, all ROS1 and ALK positive tumors expressed total EGFR. Unexpectedly, two EGFR L858R/ALK positive (patients 3 and 8), one EGFR L858R/ROS1 positive (patient 1) and one EGFR E746-A750del/ROS1 positive (patient 6) tumors were identified (see Figs. 11A-H). Sequencing confirmed the presence of EGFR mutations in the two ROS1 positive tumors.

### EQUIVALENTS

It is to be understood that while the disclosure has been described in conjunction with the detailed description thereof, the foregoing description is intended to illustrate and not limit the scope of the invention, which is defined by the scope of the appended claims.

## Claims

1. A method comprising detecting in a biological sample from a human patient having or suspected of having cancer the presence of a polypeptide having ROS1 kinase activity and detecting in the biological sample the presence of a mutant EGFR polypeptide that has increased kinase activity and/or renders a tumor cell sensitive to one or more EGFR inhibitors.

2. The method of claim 1, wherein the sample is from a lung cancer, optionally a non-small cell lung cancer.

3. The method of claim 1 or 2, wherein the polypeptide having ROS1 kinase activity is a ROS1 fusion.

4. The method of any of claims 1-3, wherein the mutant EGFR polypeptide comprises a mutation in the kinase domain.

5. The method of any of claims 1-4, wherein detecting the presence of a polypeptide having ROS1 kinase activity comprises the use of an antibody.

6. The method of any of claims 1-5, wherein detecting the presence of a mutant EGFR polypeptide comprises the use of a mutant-specific antibody.

7. A therapeutically effective amount of a ROS1 inhibitor and an EGFR inhibitor for use in treating a human cancer patient, wherein the presence of a polypeptide having ROS1 kinase activity and the presence of a mutant EGFR polypeptide that has increased kinase activity and/or renders a tumor cell sensitive to one or more EGFR inhibitors have been detected in the patient.

8. The therapeutically effective amount of the ROS1 inhibitor and the EGFR inhibitor for use according to claim 7, wherein the cancer is a lung cancer, optionally a non-small cell lung cancer.

9. The therapeutically effective amount of the ROS1 inhibitor and the EGFR inhibitor for use according to claim 7 or 8, wherein the polypeptide having ROS1 kinase activity is a ROS1 fusion.

10. The therapeutically effective amount of the ROS1 inhibitor and the EGFR inhibitor for use according to any of claims 7-9, wherein the mutant EGFR polypeptide comprises a mutation in the kinase domain.

11. The therapeutically effective amount of the ROS1 inhibitor and the EGFR inhibitor for use according to any of claims 7-10, wherein the ROS1 inhibitor is selected from the group consisting of crizotinib, ASP3026, NVP TAE-684, CH5424802, and AP26113.

12. The therapeutically effective amount of the ROS1 inhibitor and the EGFR inhibitor for use according to any of claims 7-11, wherein the EGFR inhibitor is selected from the group consisting of gefitinib, erlotinib, cetuximab, afatinib, necitumumab, nimotuzumab, PF299804, RO5083945, ABT-806, and AP26113.

## Patentansprüche

1. Verfahren umfassend Nachweisen der Anwesenheit eines Polypeptids mit ROS1-Kinaseaktivität in einer biologischen Probe eines menschlichen Patienten, der an Krebs leidet oder bei dem Verdacht auf Krebs besteht; und Nachweisen der Anwesenheit eines mutierten EGFR-Polypeptids in der biologischen Probe, das erhöhte Kinaseaktivität aufweist und/oder eine Tumorzelle empfindlich gegenüber einem oder mehreren EGFR-Inhibitoren macht.

2. Verfahren gemäß Anspruch 1, wobei die Probe aus einem Lungenkrebs, optional einem nicht-kleinzelligen Lungenkrebs stammt.

3. Verfahren gemäß Anspruch 1 oder 2, wobei das Polypeptid mit ROS1-Kinaseaktivität eine ROS1-Fusion ist.

4. Verfahren gemäß irgendeinem der Ansprüche 1-3, wobei das mutierte EGFR-Polypeptid eine Mutation in der Kinase-Domäne umfasst.

5. Verfahren gemäß irgendeinem der Ansprüche 1-4, wobei das Nachweisen der Anwesenheit eines Polypeptids mit ROS1-Kinaseaktivität die Verwendung eines Antikörpers umfasst.

6. Verfahren gemäß irgendeinem der Ansprüche 1-5, wobei das Nachweisen der Anwesenheit eines mutierten EGFR-Polypeptids die Verwendung eines Mutanten-spezifischen Antikörpers umfasst.

7. Therapeutisch wirksame Menge eines ROS1-Inhibitors und eines EGFR-Inhibitors zur Verwendung bei der Behandlung eines menschlichen Krebspatienten, wobei die Anwesenheit eines Polypeptids mit ROS1-Kinaseaktivität und die Anwesenheit eines mutierten EGFR-Polypeptids, das erhöhte Kinaseaktivität aufweist und/oder eine Tumorzelle empfindlich gegenüber einem oder mehreren EGFR-Inhibitoren macht, in dem Patienten nachgewiesen wurden.

8. Therapeutisch wirksame Menge des ROS1-Inhibitors und des EGFR-Inhibitors zur Verwendung gemäß Anspruch 7, wobei der Krebs ein Lungenkrebs, optional ein nichtkleinzelliger Lungenkrebs, ist.

9. Therapeutisch wirksame Menge des ROS1-Inhibitors und des EGFR-Inhibitors zur Verwendung gemäß Anspruch 7 oder 8, wobei das Polypeptid mit ROS1-Kinaseaktivität eine ROS1-Fusion ist.

10. Therapeutisch wirksame Menge des ROS1-Inhibitors und des EGFR-Inhibitors zur Verwendung gemäß irgendeinem der Ansprüche 7-9, wobei das mutierte EGFR-Polypeptid eine Mutation in der Kinase-Domäne umfasst.

11. Therapeutisch wirksame Menge des ROS1-Inhibitors und des EGFR-Inhibitors zur Verwendung gemäß irgendeinem der Ansprüche 7-10, wobei der ROS1-Inhibitor ausgewählt ist aus der Gruppe bestehend aus Crizotinib, ASP3026, NVP TAE-684, CH5424802 und AP26113.

12. Therapeutisch wirksame Menge des ROS1-Inhibitors und des EGFR-Inhibitors zur Verwendung gemäß irgendeinem der Ansprüche 7-11, wobei der EGFR-Inhibitor ausgewählt ist aus der Gruppe bestehend aus Gefitinib, Erlotinib, Cetuximab, Afatinib, Necitumumab, Nimotuzumab, PF299804, RO5083945, ABT-806 und AP26113.

## Revendications

1. Procédé comprenant la détection, dans un échantillon biologique provenant d'un patient humain présentant ou suspecté de présenter un cancer, de la présence d'un polypeptide à activité kinase ROS1 et la détection, dans l'échantillon biologique, de la présence d'un polypeptide EGFR mutant qui présente une activité de kinase accrue et/ou rend une cellule tumorale sensible à un ou plusieurs inhibiteurs d'EGFR.

2. Procédé selon la revendication 1, dans lequel l'échantillon provient d'un cancer du poumon, optionnellement un cancer du poumon non à petites cellules.

3. Procédé selon la revendication 1 ou 2, dans lequel le polypeptide à activité kinase ROS1 est une fusion de ROS1.

4. Procédé selon l'une quelconque des revendications 1-3, dans lequel le polypeptide EGFR mutant comprend une mutation dans le domaine kinase.

5. Procédé selon l'une quelconque des revendications 1-4, dans lequel la détection de la présence d'un polypeptide à activité kinase ROS1 comprend l'utilisation d'un anticorps.

6. Procédé selon l'une quelconque des revendications 1-5, dans lequel la détection de la présence d'un polypeptide EGFR mutant comprend l'utilisation d'un anticorps spécifique d'un mutant.

7. Quantité thérapeutiquement efficace d'un inhibiteur de ROS1 et d'un inhibiteur d'EGFR pour une utilisation dans le traitement d'un patient humain pour un cancer, dans laquelle la présence d'un polypeptide à activité kinase ROS1 et la présence d'un polypeptide EGFR mutant qui présente une activité de kinase accrue et/ou rend une cellule tumorale sensible à un ou plusieurs inhibiteurs d'EGFR ont été détectées chez le patient.

8. Quantité thérapeutiquement efficace de l'inhibiteur de ROS1 et de l'inhibiteur d'EGFR pour une utilisation selon la revendication 7, dans laquelle le cancer est un cancer du poumon, optionnellement un cancer du poumon non à petites cellules.

9. Quantité thérapeutiquement efficace de l'inhibiteur de ROS1 et de l'inhibiteur d'EGFR pour une utilisation selon la revendication 7 ou 8, dans laquelle le polypeptide à activité kinase ROS1 est une fusion de ROS1.

10. Quantité thérapeutiquement efficace de l'inhibiteur de ROS1 et de l'inhibiteur d'EGFR pour une utilisation selon l'une quelconque des revendications 7-9, dans laquelle le polypeptide EGFR mutant comprend une mutation dans le domaine kinase.

11. Quantité thérapeutiquement efficace de l'inhibiteur de ROS1 et de l'inhibiteur d'EGFR pour une utilisation selon l'une quelconque des revendications 7-10, dans laquelle l'inhibiteur de ROS1 est choisi dans le groupe consistant en le crizotinib, l'ASP3026, le NVP TAE-684, le CH5424802, et l'AP26113.

12. Quantité thérapeutiquement efficace de l'inhibiteur de ROS1 et de l'inhibiteur d'EGFR pour une utilisation selon l'une quelconque des revendications 7-11, dans laquelle l'inhibiteur d'EGFR est choisi dans le groupe consistant en le géfitinib, l'erlotinib, le cétuximab, l'afatinib, le nécitumumab, le nimotuzumab, le PF299804, le RO5083945, l'ABT-806, et l'AP26113.


### SEQUENCE LISTING

<110> Cell Signaling Technology, Inc.
<120> EGFR And ROS1 Kinase In Cancer
<130> CST-321
<150> 61/635,057 <151> 2012-04-18
<150> 61/787,033 <151> 2013-03-15
<160> 36
<170> PatentIn version 3.5
<210> 1
   <211> 2347
   <212> PRT
   <213> Artificial Sequence
<220>
   <223> Synthetic Polypeptide
<400> 1
   Met Lys Asn Ile Tyr Cys Leu Ile Pro Lys Leu Val Asn Phe Ala Thr
   1               5                   10                  15
   Leu Gly Cys Leu Trp Ile Ser Val Val Gln Cys Thr Val Leu Asn Ser
               20                  25                  30
   Cys Leu Lys Ser Cys Val Thr Asn Leu Gly Gln Gln Leu Asp Leu Gly
           35                  40                  45
   Thr Pro His Asn Leu Ser Glu Pro Cys Ile Gln Gly Cys His Phe Trp
       50                  55                  60
   Asn Ser Val Asp Gln Lys Asn Cys Ala Leu Lys Cys Arg Glu Ser Cys
   65                  70                  75                  80
   Glu Val Gly Cys Ser Ser Ala Glu Gly Ala Tyr Glu Glu Glu Val Leu
                   85                  90                  95
   Glu Asn Ala Asp Leu Pro Thr Ala Pro Phe Ala Ser Ser Ile Gly Ser
               100                 105                 110
   His Asn Met Thr Leu Arg Trp Lys Ser Ala Asn Phe Ser Gly Val Lys
           115                 120                 125
   Tyr Ile Ile Gln Trp Lys Tyr Ala Gln Leu Leu Gly Ser Trp Thr Tyr
       130                 135                 140
   Thr Lys Thr Val Ser Arg Pro Ser Tyr Val Val Lys Pro Leu His Pro
   145 150 155 160
   Phe Thr Glu Tyr Ile Phe Arg Val Val Trp Ile Phe Thr Ala Gln Leu
   165 170 175
   Gln Leu Tyr Ser Pro Pro Ser Pro Ser Tyr Arg Thr His Pro His Gly
   180 185 190
   Val Pro Glu Thr Ala Pro Leu Ile Arg Asn Ile Glu Ser Ser Ser Pro
   195 200 205
   Asp Thr Val Glu Val Ser Trp Asp Pro Pro Gln Phe Pro Gly Gly Pro
   210 215 220
   Ile Leu Gly Tyr Asn Leu Arg Leu Ile Ser Lys Asn Gln Lys Leu Asp
   225 230 235 240
   Ala Gly Thr Gln Arg Thr Ser Phe Gln Phe Tyr Ser Thr Leu Pro Asn
   245 250 255
   Thr Ile Tyr Arg Phe Ser Ile Ala Ala Val Asn Glu Val Gly Glu Gly
   260 265 270
   Pro Glu Ala Glu Ser Ser Ile Thr Thr Ser Ser Ser Ala Val Gln Gln
   275 280 285
   Glu Glu Gln Trp Leu Phe Leu Ser Arg Lys Thr Ser Leu Arg Lys Arg
   290 295 300
   Ser Leu Lys His Leu Val Asp Glu Ala His Cys Leu Arg Leu Asp Ala
   305 310 315 320
   Ile Tyr His Asn Ile Thr Gly Ile Ser Val Asp Val His Gln Gln Ile
   325 330 335
   Val Tyr Phe Ser Glu Gly Thr Leu Ile Trp Ala Lys Lys Ala Ala Asn
   340 345 350
   Met Ser Asp Val Ser Asp Leu Arg Ile Phe Tyr Arg Gly Ser Gly Leu
   355 360 365
   Ile Ser Ser Ile Ser Ile Asp Trp Leu Tyr Gln Arg Met Tyr Phe Ile
   370 375 380
   Met Asp Glu Leu Val Cys Val Cys Asp Leu Glu Asn Cys Ser Asn Ile
   385 390 395 400
   Glu Glu Ile Thr Pro Pro Ser Ile Ser Ala Pro Gln Lys Ile Val Ala
   405 410 415
   Asp Ser Tyr Asn Gly Tyr Val Phe Tyr Leu Leu Arg Asp Gly Ile Tyr
   420 425 430
   Arg Ala Asp Leu Pro Val Pro Ser Gly Arg Cys Ala Glu Ala Val Arg
   435 440 445
   Ile Val Glu Ser Cys Thr Leu Lys Asp Phe Ala Ile Lys Pro Gln Ala
   450 455 460
   Lys Arg Ile Ile Tyr Phe Asn Asp Thr Ala Gln Val Phe Met Ser Thr
   465 470 475 480
   Phe Leu Asp Gly Ser Ala Ser His Leu Ile Leu Pro Arg Ile Pro Phe
   485 490 495
   Ala Asp Val Lys Ser Phe Ala Cys Glu Asn Asn Asp Phe Leu Val Thr
   500 505 510
   Asp Gly Lys Val Ile Phe Gln Gln Asp Ala Leu Ser Phe Asn Glu Phe
   515 520 525
   Ile Val Gly Cys Asp Leu Ser His Ile Glu Glu Phe Gly Phe Gly Asn
   530 535 540
   Leu Val Ile Phe Gly Ser Ser Ser Gln Leu His Pro Leu Pro Gly Arg
   545 550 555 560
   Pro Gln Glu Leu Ser Val Leu Phe Gly Ser His Gln Ala Leu Val Gln
   565 570 575
   Trp Lys Pro Pro Ala Leu Ala Ile Gly Ala Asn Val Ile Leu Ile Ser
   580 585 590
   Asp Ile Ile Glu Leu Phe Glu Leu Gly Pro Ser Ala Trp Gln Asn Trp
   595 600 605
   Thr Tyr Glu Val Lys Val Ser Thr Gln Asp Pro Pro Glu Val Thr His
   610 615 620
   Ile Phe Leu Asn Ile Ser Gly Thr Met Leu Asn Val Pro Glu Leu Gln
   625 630 635 640
   Ser Ala Met Lys Tyr Lys Val Ser Val Arg Ala Ser Ser Pro Lys Arg
   645 650 655
   Pro Gly Pro Trp Ser Glu Pro Ser Val Gly Thr Thr Leu Val Pro Ala
   660 665 670
   Ser Glu Pro Pro Phe Ile Met Ala Val Lys Glu Asp Gly Leu Trp Ser
   675 680 685
   Lys Pro Leu Asn Ser Phe Gly Pro Gly Glu Phe Leu Ser Ser Asp Ile
   690 695 700
   Gly Asn Val Ser Asp Met Asp Trp Tyr Asn Asn Ser Leu Tyr Tyr Ser
   705 710 715 720
   Asp Thr Lys Gly Asp Val Phe Val Trp Leu Leu Asn Gly Thr Asp Ile
   725 730 735
   Ser Glu Asn Tyr His Leu Pro Ser Ile Ala Gly Ala Gly Ala Leu Ala
   740 745 750
   Phe Glu Trp Leu Gly His Phe Leu Tyr Trp Ala Gly Lys Thr Tyr Val
   755 760 765
   Ile Gln Arg Gln Ser Val Leu Thr Gly His Thr Asp Ile Val Thr His
   770 775 780
   Val Lys Leu Leu Val Asn Asp Met Val Val Asp Ser Val Gly Gly Tyr
   785 790 795 800
   Leu Tyr Trp Thr Thr Leu Tyr Ser Val Glu Ser Thr Arg Leu Asn Gly
   805 810 815
   Glu Ser Ser Leu Val Leu Gln Thr Gln Pro Trp Phe Ser Gly Lys Lys
   820 825 830
   Val Ile Ala Leu Thr Leu Asp Leu Ser Asp Gly Leu Leu Tyr Trp Leu
   835 840 845
   Val Gln Asp Ser Gln Cys Ile His Leu Tyr Thr Ala Val Leu Arg Gly
   850 855 860
   Gln Ser Thr Gly Asp Thr Thr Ile Thr Glu Phe Ala Ala Trp Ser Thr
   865 870 875 880
   Ser Glu Ile Ser Gln Asn Ala Leu Met Tyr Tyr Ser Gly Arg Leu Phe
   885 890 895
   Trp Ile Asn Gly Phe Arg Ile Ile Thr Thr Gln Glu Ile Gly Gln Lys
   900 905 910
   Thr Ser Val Ser Val Leu Glu Pro Ala Arg Phe Asn Gln Phe Thr Ile
   915 920 925
   Ile Gln Thr Ser Leu Lys Pro Leu Pro Gly Asn Phe Ser Phe Thr Pro
   930 935 940
   Lys Val Ile Pro Asp Ser Val Gln Glu Ser Ser Phe Arg Ile Glu Gly
   945 950 955 960
   Asn Ala Ser Ser Phe Gln Ile Leu Trp Asn Gly Pro Pro Ala Val Asp
   965 970 975
   Trp Gly Val Val Phe Tyr Ser Val Glu Phe Ser Ala His Ser Lys Phe
   980 985 990
   Leu Ala Ser Glu Gln His Ser Leu Pro Val Phe Thr Val Glu Gly Leu
   995 1000 1005
   Glu Pro Tyr Ala Leu Phe Asn Leu Ser Val Thr Pro Tyr Thr Tyr
   1010 1015 1020
   Trp Gly Lys Gly Pro Lys Thr Ser Leu Ser Leu Arg Ala Pro Glu
   1025 1030 1035
   Thr Val Pro Ser Ala Pro Glu Asn Pro Arg Ile Phe Ile Leu Pro
   1040 1045 1050
   Ser Gly Lys Cys Cys Asn Lys Asn Glu Val Val Val Glu Phe Arg
   1055 1060 1065
   Trp Asn Lys Pro Lys His Glu Asn Gly Val Leu Thr Lys Phe Glu
   1070 1075 1080
   Ile Phe Tyr Asn Ile Ser Asn Gln Ser Ile Thr Asn Lys Thr Cys
   1085 1090 1095
   Glu Asp Trp Ile Ala Val Asn Val Thr Pro Ser Val Met Ser Phe
   1100 1105 1110
   Gln Leu Glu Gly Met Ser Pro Arg Cys Phe Ile Ala Phe Gln Val
   1115 1120 1125
   Arg Ala Phe Thr Ser Lys Gly Pro Gly Pro Tyr Ala Asp Val Val
   1130 1135 1140
   Lys Ser Thr Thr Ser Glu Ile Asn Pro Phe Pro His Leu Ile Thr
   1145 1150 1155
   Leu Leu Gly Asn Lys Ile Val Phe Leu Asp Met Asp Gln Asn Gln
   1160 1165 1170
   Val Val Trp Thr Phe Ser Ala Glu Arg Val Ile Ser Ala Val Cys
   1175 1180 1185
   Tyr Thr Ala Asp Asn Glu Met Gly Tyr Tyr Ala Glu Gly Asp Ser
   1190 1195 1200
   Leu Phe Leu Leu His Leu His Asn Arg Ser Ser Ser Glu Leu Phe
   1205 1210 1215
   Gln Asp Ser Leu Val Phe Asp Ile Thr Val Ile Thr Ile Asp Trp
   1220 1225 1230
   Ile Ser Arg His Leu Tyr Phe Ala Leu Lys Glu Ser Gln Asn Gly
   1235 1240 1245
   Met Gln Val Phe Asp Val Asp Leu Glu His Lys Val Lys Tyr Pro
   1250 1255 1260
   Arg Glu Val Lys Ile His Asn Arg Asn Ser Thr Ile Ile Ser Phe
   1265 1270 1275
   Ser Val Tyr Pro Leu Leu Ser Arg Leu Tyr Trp Thr Glu Val Ser
   1280 1285 1290
   Asn Phe Gly Tyr Gln Met Phe Tyr Tyr Ser Ile Ile Ser His Thr
   1295 1300 1305
   Leu His Arg Ile Leu Gln Pro Thr Ala Thr Asn Gln Gln Asn Lys
   1310 1315 1320
   Arg Asn Gln Cys Ser Cys Asn Val Thr Glu Phe Glu Leu Ser Gly
   1325 1330 1335
   Ala Met Ala Ile Asp Thr Ser Asn Leu Glu Lys Pro Leu Ile Tyr
   1340 1345 1350
   Phe Ala Lys Ala Gln Glu Ile Trp Ala Met Asp Leu Glu Gly Cys
   1355 1360 1365
   Gln Cys Trp Arg Val Ile Thr Val Pro Ala Met Leu Ala Gly Lys
   1370 1375 1380
   Thr Leu Val Ser Leu Thr Val Asp Gly Asp Leu Ile Tyr Trp Ile
   1385 1390 1395
   Ile Thr Ala Lys Asp Ser Thr Gln Ile Tyr Gln Ala Lys Lys Gly
   1400 1405 1410
   Asn Gly Ala Ile Val Ser Gln Val Lys Ala Leu Arg Ser Arg His
   1415 1420 1425
   Ile Leu Ala Tyr Ser Ser Val Met Gln Pro Phe Pro Asp Lys Ala
   1430 1435 1440
   Phe Leu Ser Leu Ala Ser Asp Thr Val Glu Pro Thr Ile Leu Asn
   1445 1450 1455
   Ala Thr Asn Thr Ser Leu Thr Ile Arg Leu Pro Leu Ala Lys Thr
   1460 1465 1470
   Asn Leu Thr Trp Tyr Gly Ile Thr Ser Pro Thr Pro Thr Tyr Leu
   1475 1480 1485
   Val Tyr Tyr Ala Glu Val Asn Asp Arg Lys Asn Ser Ser Asp Leu
   1490 1495 1500
   Lys Tyr Arg Ile Leu Glu Phe Gln Asp Ser Ile Ala Leu Ile Glu
   1505 1510 1515
   Asp Leu Gln Pro Phe Ser Thr Tyr Met Ile Gln Ile Ala Val Lys
   1520 1525 1530
   Asn Tyr Tyr Ser Asp Pro Leu Glu His Leu Pro Pro Gly Lys Glu
   1535 1540 1545
   Ile Trp Gly Lys Thr Lys Asn Gly Val Pro Glu Ala Val Gln Leu
   1550 1555 1560
   Ile Asn Thr Thr Val Arg Ser Asp Thr Ser Leu Ile Ile Ser Trp
   1565 1570 1575
   Arg Glu Ser His Lys Pro Asn Gly Pro Lys Glu Ser Val Arg Tyr
   1580 1585 1590
   Gln Leu Ala Ile Ser His Leu Ala Leu Ile Pro Glu Thr Pro Leu
   1595 1600 1605
   Arg Gln Ser Glu Phe Pro Asn Gly Arg Leu Thr Leu Leu Val Thr
   1610 1615 1620
   Arg Leu Ser Gly Gly Asn Ile Tyr Val Leu Lys Val Leu Ala Cys
   1625 1630 1635
   His Ser Glu Glu Met Trp Cys Thr Glu Ser His Pro Val Thr Val
   1640 1645 1650
   Glu Met Phe Asn Thr Pro Glu Lys Pro Tyr Ser Leu Val Pro Glu
   1655 1660 1665
   Asn Thr Ser Leu Gln Phe Asn Trp Lys Ala Pro Leu Asn Val Asn
   1670 1675 1680
   Leu Ile Arg Phe Trp Val Glu Leu Gln Lys Trp Lys Tyr Asn Glu
   1685 1690 1695
   Phe Tyr His Val Lys Thr Ser Cys Ser Gln Gly Pro Ala Tyr Val
   1700 1705 1710
   Cys Asn Ile Thr Asn Leu Gln Pro Tyr Thr Ser Tyr Asn Val Arg
   1715 1720 1725
   Val Val Val Val Tyr Lys Thr Gly Glu Asn Ser Thr Ser Leu Pro
   1730 1735 1740
   Glu Ser Phe Lys Thr Lys Ala Gly Val Pro Asn Lys Pro Gly Ile
   1745 1750 1755
   Pro Lys Leu Leu Glu Gly Ser Lys Asn Ser Ile Gln Trp Glu Lys
   1760 1765 1770
   Ala Glu Asp Asn Gly Cys Arg Ile Thr Tyr Tyr Ile Leu Glu Ile
   1775 1780 1785
   Arg Lys Ser Thr Ser Asn Asn Leu Gln Asn Gln Asn Leu Arg Trp
   1790 1795 1800
   Lys Met Thr Phe Asn Gly Ser Cys Ser Ser Val Cys Thr Trp Lys
   1805 1810 1815
   Ser Lys Asn Leu Lys Gly Ile Phe Gln Phe Arg Val Val Ala Ala
   1820 1825 1830
   Asn Asn Leu Gly Phe Gly Glu Tyr Ser Gly Ile Ser Glu Asn Ile
   1835 1840 1845
   Ile Leu Val Gly Asp Asp Phe Trp Ile Pro Glu Thr Ser Phe Ile
   1850 1855 1860
   Leu Thr Ile Ile Val Gly Ile Phe Leu Val Val Thr Ile Pro Leu
   1865 1870 1875
   Thr Phe Val Trp His Arg Arg Leu Lys Asn Gln Lys Ser Ala Lys
   1880 1885 1890
   Glu Gly Val Thr Val Leu Ile Asn Glu Asp Lys Glu Leu Ala Glu
   1895 1900 1905
   Leu Arg Gly Leu Ala Ala Gly Val Gly Leu Ala Asn Ala Cys Tyr
   1910 1915 1920
   Ala Ile His Thr Leu Pro Thr Gln Glu Glu Ile Glu Asn Leu Pro
   1925 1930 1935
   Ala Phe Pro Arg Glu Lys Leu Thr Leu Arg Leu Leu Leu Gly Ser
   1940 1945 1950
   Gly Ala Phe Gly Glu Val Tyr Glu Gly Thr Ala Val Asp Ile Leu
   1955 1960 1965
   Gly Val Gly Ser Gly Glu Ile Lys Val Ala Val Lys Thr Leu Lys
   1970 1975 1980
   Lys Gly Ser Thr Asp Gln Glu Lys Ile Glu Phe Leu Lys Glu Ala
   1985 1990 1995
   His Leu Met Ser Lys Phe Asn His Pro Asn Ile Leu Lys Gln Leu
   2000 2005 2010
   Gly Val Cys Leu Leu Asn Glu Pro Gln Tyr Ile Ile Leu Glu Leu
   2015 2020 2025
   Met Glu Gly Gly Asp Leu Leu Thr Tyr Leu Arg Lys Ala Arg Met
   2030 2035 2040
   Ala Thr Phe Tyr Gly Pro Leu Leu Thr Leu Val Asp Leu Val Asp
   2045 2050 2055
   Leu Cys Val Asp Ile Ser Lys Gly Cys Val Tyr Leu Glu Arg Met
   2060 2065 2070
   His Phe Ile His Arg Asp Leu Ala Ala Arg Asn Cys Leu Val Ser
   2075 2080 2085
   Val Lys Asp Tyr Thr Ser Pro Arg Ile Val Lys Ile Gly Asp Phe
       2090                2095                2100
   Gly Leu Ala Arg Asp Ile Tyr Lys Asn Asp Tyr Tyr Arg Lys Arg
       2105                2110                2115
   Gly Glu Gly Leu Leu Pro Val Arg Trp Met Ala Pro Glu Ser Leu
       2120                2125                2130
   Met Asp Gly Ile Phe Thr Thr Gln Ser Asp Val Trp Ser Phe Gly
       2135                2140                2145
   Ile Leu Ile Trp Glu Ile Leu Thr Leu Gly His Gln Pro Tyr Pro
       2150                2155                2160
   Ala His Ser Asn Leu Asp Val Leu Asn Tyr Val Gln Thr Gly Gly
       2165                2170                2175
   Arg Leu Glu Pro Pro Arg Asn Cys Pro Asp Asp Leu Trp Asn Leu
       2180                2185                2190
   Met Thr Gln Cys Trp Ala Gln Glu Pro Asp Gln Arg Pro Thr Phe
       2195                2200                2205
   His Arg Ile Gln Asp Gln Leu Gln Leu Phe Arg Asn Phe Phe Leu
       2210                2215                2220
   Asn Ser Ile Tyr Lys Ser Arg Asp Glu Ala Asn Asn Ser Gly Val
       2225                2230                2235
   Ile Asn Glu Ser Phe Glu Gly Glu Asp Gly Asp Val Ile Cys Leu
       2240                2245                2250
   Asn Ser Asp Asp Ile Met Pro Val Ala Leu Met Glu Thr Lys Asn
       2255                2260                2265
   Arg Glu Gly Leu Asn Tyr Met Val Leu Ala Thr Glu Cys Gly Gln
       2270                2275                2280
   Gly Glu Glu Lys Ser Glu Gly Pro Leu Gly Ser Gln Glu Ser Glu
       2285                2290                2295
   Ser Cys Gly Leu Arg Lys Glu Glu Lys Glu Pro His Ala Asp Lys
       2300                2305                2310
   Asp Phe Cys Gln Glu Lys Gln Val Ala Tyr Cys Pro Ser Gly Lys
       2315                2320                2325
   Pro Glu Gly Leu Asn Tyr Ala Cys Leu Thr His Ser Gly Tyr Gly
   2330 2335 2340
   Asp Gly Ser Asp
   2345
<210> 2
   <211> 7368
   <212> DNA
   <213> Artificial Sequence
<220>
   <223> Synthetic Polynucleotide
<400> 2
   caagctttca agcattcaaa ggtctaaatg aaaaaggcta agtattattt caaaaggcaa 60
   gtatatccta atatagcaaa acaaacaaag caaaatccat cagctactcc tccaattgaa 120
   gtgatgaagc ccaaataatt catatagcaa aatggagaaa attagaccgg ccatctaaaa 180
   atctgccatt ggtgaagtga tgaagaacat ttactgtctt attccgaagc ttgtcaattt 240
   tgcaactctt ggctgcctat ggatttctgt ggtgcagtgt acagttttaa atagctgcct 300
   aaagtcgtgt gtaactaatc tgggccagca gcttgacctt ggcacaccac ataatctgag 360
   tgaaccgtgt atccaaggat gtcacttttg gaactctgta gatcagaaaa actgtgcttt 420
   aaagtgtcgg gagtcgtgtg aggttggctg tagcagcgcg gaaggtgcat atgaagagga 480
   agtactggaa aatgcagacc taccaactgc tccctttgct tcttccattg gaagccacaa 540
   tatgacatta cgatggaaat ctgcaaactt ctctggagta aaatacatca ttcagtggaa 600
   atatgcacaa cttctgggaa gctggactta tactaagact gtgtccagac cgtcctatgt 660
   ggtcaagccc ctgcacccct tcactgagta cattttccga gtggtttgga tcttcacagc 720
   gcagctgcag ctctactccc ctccaagtcc cagttacagg actcatcctc atggagttcc 780
   tgaaactgca cctttgatta ggaatattga gagctcaagt cccgacactg tggaagtcag 840
   ctgggatcca cctcaattcc caggtggacc tattttgggt tataacttaa ggctgatcag 900
   caaaaatcaa aaattagatg cagggacaca gagaaccagt ttccagtttt actccacttt 960
   accaaatact atctacaggt tttctattgc agcagtaaat gaagttggtg agggtccaga 1020
   agcagaatct agtattacca cttcatcttc agcagttcaa caagaggaac agtggctctt 1080
   tttatccaga aaaacttctc taagaaagag atctttaaaa catttagtag atgaagcaca 1140
   ttgccttcgg ttggatgcta tataccataa tattacagga atatctgttg atgtccacca 1200
   gcaaattgtt tatttctctg aaggaactct catatgggcg aagaaggctg ccaacatgtc 1260
   tgatgtatct gacctgagaa ttttttacag aggttcagga ttaatttctt ctatctccat 1320
   agattggctt tatcaaagaa tgtatttcat catggatgaa ctggtatgtg tctgtgattt 1380
   agagaactgc tcaaacatcg aggaaattac tccaccctct attagtgcac ctcaaaaaat 1440
   tgtggctgat tcatacaatg ggtatgtctt ttacctcctg agagatggca tttatagagc 1500
   agaccttcct gtaccatctg gccggtgtgc agaagctgtg cgtattgtgg agagttgcac 1560
   gttaaaggac tttgcaatca agccacaagc caagcgaatc atttacttca atgacactgc 1620
   ccaagtcttc atgtcaacat ttctggatgg ctctgcttcc catctcatcc tacctcgcat 1680
   cccctttgct gatgtgaaaa gttttgcttg tgaaaacaat gactttcttg tcacagatgg 1740
   caaggtcatt ttccaacagg atgctttgtc ttttaatgaa ttcatcgtgg gatgtgacct 1800
   gagtcacata gaagaatttg ggtttggtaa cttggtcatc tttggctcat cctcccagct 1860
   gcaccctctg ccaggccgcc cgcaggagct ttcggtgctg tttggctctc accaggctct 1920
   tgttcaatgg aagcctcctg cccttgccat aggagccaat gtcatcctga tcagtgatat 1980
   tattgaactc tttgaattag gcccttctgc ctggcagaac tggacctatg aggtgaaagt 2040
   atccacccaa gaccctcctg aagtcactca tattttcttg aacataagtg gaaccatgct 2100
   gaatgtacct gagctgcaga gtgctatgaa atacaaggtt tctgtgagag caagttctcc 2160
   aaagaggcca ggcccctggt cagagccctc agtgggtact accctggtgc cagctagtga 2220
   accaccattt atcatggctg tgaaagaaga tgggctttgg agtaaaccat taaatagctt 2280
   tggcccagga gagttcttat cctctgatat aggaaatgtg tcagacatgg attggtataa 2340
   caacagcctc tactacagtg acacgaaagg cgacgttttt gtgtggctgc tgaatgggac 2400
   ggatatctca gagaattatc acctacccag cattgcagga gcaggggctt tagcttttga 2460
   gtggctgggt cactttctct actgggctgg aaagacatat gtgatacaaa ggcagtctgt 2520
   gttgacggga cacacagaca ttgttaccca cgtgaagcta ttggtgaatg acatggtggt 2580
   ggattcagtt ggtggatatc tctactggac cacactctat tcagtggaaa gcaccagact 2640
   aaatggggaa agttcccttg tactacagac acagccttgg ttttctggga aaaaggtaat 2700
   tgctctaact ttagacctca gtgatgggct cctgtattgg ttggttcaag acagtcaatg 2760
   tattcacctg tacacagctg ttcttcgggg acagagcact ggggatacca ccatcacaga 2820
   atttgcagcc tggagtactt ctgaaatttc ccagaatgca ctgatgtact atagtggtcg 2880
   gctgttctgg atcaatggct ttaggattat cacaactcaa gaaataggtc agaaaaccag 2940
   tgtctctgtt ttggaaccag ccagatttaa tcagttcaca attattcaga catcccttaa 3000
   gcccctgcca gggaactttt cctttacccc taaggttatt ccagattctg ttcaagagtc 3060
   ttcatttagg attgaaggaa atgcttcaag ttttcaaatc ctgtggaatg gtccccctgc 3120
   ggtagactgg ggtgtagttt tctacagtgt agaatttagt gctcattcta agttcttggc 3180
   tagtgaacaa cactctttac ctgtatttac tgtggaagga ctggaacctt atgccttatt 3240
   taatctttct gtcactcctt atacctactg gggaaagggc cccaaaacat ctctgtcact 3300
   tcgagcacct gaaacagttc catcagcacc agagaacccc agaatattta tattaccaag 3360
   tggaaaatgc tgcaacaaga atgaagttgt ggtggaattt aggtggaaca aacctaagca 3420
   tgaaaatggg gtgttaacaa aatttgaaat tttctacaat atatccaatc aaagtattac 3480
   aaacaaaaca tgtgaagact ggattgctgt caatgtcact ccctcagtga tgtcttttca 3540
   acttgaaggc atgagtccca gatgctttat tgccttccag gttagggcct ttacatctaa 3600
   ggggccagga ccatatgctg acgttgtaaa gtctacaaca tcagaaatca acccatttcc 3660
   tcacctcata actcttcttg gtaacaagat agttttttta gatatggatc aaaatcaagt 3720
   tgtgtggacg ttttcagcag aaagagttat cagtgccgtt tgctacacag ctgataatga 3780
   gatgggatat tatgctgaag gggactcact ctttcttctg cacttgcaca atcgctctag 3840
   ctctgagctt ttccaagatt cactggtttt tgatatcaca gttattacaa ttgactggat 3900
   ttcaaggcac ctctactttg cactgaaaga atcacaaaat ggaatgcaag tatttgatgt 3960
   tgatcttgaa cacaaggtga aatatcccag agaggtgaag attcacaata ggaattcaac 4020
   aataatttct ttttctgtat atcctctttt aagtcgcttg tattggacag aagtttccaa 4080
   ttttggctac cagatgttct actacagtat tatcagtcac accttgcacc gaattctgca 4140
   acccacagct acaaaccaac aaaacaaaag gaatcaatgt tcttgtaatg tgactgaatt 4200
   tgagttaagt ggagcaatgg ctattgatac ctctaaccta gagaaaccat tgatatactt 4260
   tgccaaagca caagagatct gggcaatgga tctggaaggc tgtcagtgtt ggagagttat 4320
   cacagtacct gctatgctcg caggaaaaac ccttgttagc ttaactgtgg atggagatct 4380
   tatatactgg atcatcacag caaaggacag cacacagatt tatcaggcaa agaaaggaaa 4440
   tggggccatc gtttcccagg tgaaggccct aaggagtagg catatcttgg cttacagttc 4500
   agttatgcag ccttttccag ataaagcgtt tctgtctcta gcttcagaca ctgtggaacc 4560
   aactatactt aatgccacta acactagcct cacaatcaga ttacctctgg ccaagacaaa 4620
   cctcacatgg tatggcatca ccagccctac tccaacatac ctggtttatt atgcagaagt 4680
   taatgacagg aaaaacagct ctgacttgaa atatagaatt ctggaatttc aggacagtat 4740
   agctcttatt gaagatttac aaccattttc aacatacatg atacagatag ctgtaaaaaa 4800
   ttattattca gatcctttgg aacatttacc accaggaaaa gagatttggg gaaaaactaa 4860
   aaatggagta ccagaggcag tgcagctcat taatacaact gtgcggtcag acaccagcct 4920
   cattatatct tggagagaat ctcacaagcc aaatggacct aaagaatcag tccgttatca 4980
   gttggcaatc tcacacctgg ccctaattcc tgaaactcct ctaagacaaa gtgaatttcc 5040
   aaatggaagg ctcactctcc ttgttactag actgtctggt ggaaatattt atgtgttaaa 5100
   ggttcttgcc tgccactctg aggaaatgtg gtgtacagag agtcatcctg tcactgtgga 5160
   aatgtttaac acaccagaga aaccttattc cttggttcca gagaacacta gtttgcaatt 5220
   taattggaag gctccattga atgttaacct catcagattt tgggttgagc tacagaagtg 5280
   gaaatacaat gagttttacc atgttaaaac ttcatgcagc caaggtcctg cttatgtctg 5340
   taatatcaca aatctacaac cttatacttc atataatgtc agagtagtgg tggtttataa 5400
   gacgggagaa aatagcacct cacttccaga aagctttaag acaaaagctg gagtcccaaa 5460
   taaaccaggc attcccaaat tactagaagg gagtaaaaat tcaatacagt gggagaaagc 5520
   tgaagataat ggatgtagaa ttacatacta tatccttgag ataagaaaga gcacttcaaa 5580
   taatttacag aaccagaatt taaggtggaa gatgacattt aatggatcct gcagtagtgt 5640
   ttgcacatgg aagtccaaaa acctgaaagg aatatttcag ttcagagtag tagctgcaaa 5700
   taatctaggg tttggtgaat atagtggaat cagtgagaat attatattag ttggagatga 5760
   tttttggata ccagaaacaa gtttcatact tactattata gttggaatat ttctggttgt 5820
   tacaatccca ctgacctttg tctggcatag aagattaaag aatcaaaaaa gtgccaagga 5880
   aggggtgaca gtgcttataa acgaagacaa agagttggct gagctgcgag gtctggcagc 5940
   cggagtaggc ctggctaatg cctgctatgc aatacatact cttccaaccc aagaggagat 6000
   tgaaaatctt cctgccttcc ctcgggaaaa actgactctg cgtctcttgc tgggaagtgg 6060
   agcctttgga gaagtgtatg aaggaacagc agtggacatc ttaggagttg gaagtggaga 6120
   aatcaaagta gcagtgaaga ctttgaagaa gggttccaca gaccaggaga agattgaatt 6180
   cctgaaggag gcacatctga tgagcaaatt taatcatccc aacattctga agcagcttgg 6240
   agtttgtctg ctgaatgaac cccaatacat tatcctggaa ctgatggagg gaggagacct 6300
   tcttacttat ttgcgtaaag cccggatggc aacgttttat ggtcctttac tcaccttggt 6360
   tgaccttgta gacctgtgtg tagatatttc aaaaggctgt gtctacttgg aacggatgca 6420
   tttcattcac agggatctgg cagctagaaa ttgccttgtt tccgtgaaag actataccag 6480
   tccacggata gtgaagattg gagactttgg actcgccaga gacatctata aaaatgatta 6540
   ctatagaaag agaggggaag gcctgctccc agttcggtgg atggctccag aaagtttgat 6600
   ggatggaatc ttcactactc aatctgatgt atggtctttt ggaattctga tttgggagat 6660
   tttaactctt ggtcatcagc cttatccagc tcattccaac cttgatgtgt taaactatgt 6720
   gcaaacagga gggagactgg agccaccaag aaattgtcct gatgatctgt ggaatttaat 6780
   gacccagtgc tgggctcaag aacccgacca aagacctact tttcatagaa ttcaggacca 6840
   acttcagtta ttcagaaatt ttttcttaaa tagcatttat aagtccagag atgaagcaaa 6900
   caacagtgga gtcataaatg aaagctttga aggtgaagat ggcgatgtga tttgtttgaa 6960
   ttcagatgac attatgccag ttgctttaat ggaaacgaag aaccgagaag ggttaaacta 7020
   tatggtactt gctacagaat gtggccaagg tgaagaaaag tctgagggtc ctctaggctc 7080
   ccaggaatct gaatcttgtg gtctgaggaa agaagagaag gaaccacatg cagacaaaga 7140
   tttctgccaa gaaaaacaag tggcttactg cccttctggc aagcctgaag gcctgaacta 7200
   tgcctgtctc actcacagtg gatatggaga tgggtctgat taatagcgtt gtttgggaaa 7260
   tagagagttg agataaacac tctcattcag tagttactga aagaaaactc tgctagaatg 7320
   ataaatgtca tggtggtcta taactccaaa taaacaatgc aacgttcc 7368
<210> 3
   <211> 1210
   <212> PRT
   <213> Artificial Sequence
<220>
   <223> Synthetic Polypeptide
<400> 3
<210> 4
   <211> 1186
   <212> PRT
   <213> Artificial Sequence
<220>
   <223> Synthetic Polypeptide
<400> 4
   Leu Glu Glu Lys Lys Val Cys Gln Gly Thr Ser Asn Lys Leu Thr Gln
   1 5 10 15
   Leu Gly Thr Phe Glu Asp His Phe Leu Ser Leu Gln Arg Met Phe Asn
   20 25 30
   Asn Cys Glu Val Val Leu Gly Asn Leu Glu Ile Thr Tyr Val Gln Arg
   35 40 45
   Asn Tyr Asp Leu Ser Phe Leu Lys Thr Ile Gln Glu Val Ala Gly Tyr
   50 55 60
   Val Leu Ile Ala Leu Asn Thr Val Glu Arg Ile Pro Leu Glu Asn Leu
   65 70 75 80
   Gln Ile Ile Arg Gly Asn Met Tyr Tyr Glu Asn Ser Tyr Ala Leu Ala
   85 90 95
   Val Leu Ser Asn Tyr Asp Ala Asn Lys Thr Gly Leu Lys Glu Leu Pro
   100 105 110
   Met Arg Asn Leu Gln Glu Ile Leu His Gly Ala Val Arg Phe Ser Asn
   115 120 125
   Asn Pro Ala Leu Cys Asn Val Glu Ser Ile Gln Trp Arg Asp Ile Val
   130 135 140
   Ser Ser Asp Phe Leu Ser Asn Met Ser Met Asp Phe Gln Asn His Leu
   145                 150                 155                 160
   Gly Ser Cys Gln Lys Cys Asp Pro Ser Cys Pro Asn Gly Ser Cys Trp
                   165                 170                 175
   Gly Ala Gly Glu Glu Asn Cys Gln Lys Leu Thr Lys Ile Ile Cys Ala
               180                 185                 190
   Gln Gln Cys Ser Gly Arg Cys Arg Gly Lys Ser Pro Ser Asp Cys Cys
           195                 200                 205
   His Asn Gln Cys Ala Ala Gly Cys Thr Gly Pro Arg Glu Ser Asp Cys
       210                 215                 220
   Leu Val Cys Arg Lys Phe Arg Asp Glu Ala Thr Cys Lys Asp Thr Cys
   225                 230                 235                 240
   Pro Pro Leu Met Leu Tyr Asn Pro Thr Thr Tyr Gln Met Asp Val Asn
                   245                 250                 255
   Pro Glu Gly Lys Tyr Ser Phe Gly Ala Thr Cys Val Lys Lys Cys Pro
               260                 265                 270
   Arg Asn Tyr Val Val Thr Asp His Gly Ser Cys Val Arg Ala Cys Gly
           275                 280                 285
   Ala Asp Ser Tyr Glu Met Glu Glu Asp Gly Val Arg Lys Cys Lys Lys
       290                 295                 300
   Cys Glu Gly Pro Cys Arg Lys Val Cys Asn Gly Ile Gly Ile Gly Glu
   305                 310                 315                 320
   Phe Lys Asp Ser Leu Ser Ile Asn Ala Thr Asn Ile Lys His Phe Lys
                   325                 330                 335
   Asn Cys Thr Ser Ile Ser Gly Asp Leu His Ile Leu Pro Val Ala Phe
               340                 345                 350
   Arg Gly Asp Ser Phe Thr His Thr Pro Pro Leu Asp Pro Gln Glu Leu
           355                 360                 365
   Asp Ile Leu Lys Thr Val Lys Glu Ile Thr Gly Phe Leu Leu Ile Gln
       370                 375                 380
   Ala Trp Pro Glu Asn Arg Thr Asp Leu His Ala Phe Glu Asn Leu Glu
   385                 390                 395                 400
   Ile Ile Arg Gly Arg Thr Lys Gln His Gly Gln Phe Ser Leu Ala Val
                   405                 410                 415
   Val Ser Leu Asn Ile Thr Ser Leu Gly Leu Arg Ser Leu Lys Glu Ile
               420                 425                 430
   Ser Asp Gly Asp Val Ile Ile Ser Gly Asn Lys Asn Leu Cys Tyr Ala
           435                 440                 445
   Asn Thr Ile Asn Trp Lys Lys Leu Phe Gly Thr Ser Gly Gln Lys Thr
       450                 455                 460
   Lys Ile Ile Ser Asn Arg Gly Glu Asn Ser Cys Lys Ala Thr Gly Gln
   465                 470                 475                 480
   Val Cys His Ala Leu Cys Ser Pro Glu Gly Cys Trp Gly Pro Glu Pro
                   485                 490                 495
   Arg Asp Cys Val Ser Cys Arg Asn Val Ser Arg Gly Arg Glu Cys Val
               500                 505                 510
   Asp Lys Cys Asn Leu Leu Glu Gly Glu Pro Arg Glu Phe Val Glu Asn
           515                 520                 525
   Ser Glu Cys Ile Gln Cys His Pro Glu Cys Leu Pro Gln Ala Met Asn
       530                 535                 540
   Ile Thr Cys Thr Gly Arg Gly Pro Asp Asn Cys Ile Gln Cys Ala His
   545                 550                 555                 560
   Tyr Ile Asp Gly Pro His Cys Val Lys Thr Cys Pro Ala Gly Val Met
                   565                 570                 575
   Gly Glu Asn Asn Thr Leu Val Trp Lys Tyr Ala Asp Ala Gly His Val
               580                 585                 590
   Cys His Leu Cys His Pro Asn Cys Thr Tyr Gly Cys Thr Gly Pro Gly
           595                 600                 605
   Leu Glu Gly Cys Pro Thr Asn Gly Pro Lys Ile Pro Ser Ile Ala Thr
       610                 615                 620
   Gly Met Val Gly Ala Leu Leu Leu Leu Leu Val Val Ala Leu Gly Ile
   625                 630                 635                 640
   Gly Leu Phe Met Arg Arg Arg His Ile Val Arg Lys Arg Thr Leu Arg
                   645                 650                 655
   Arg Leu Leu Gln Glu Arg Glu Leu Val Glu Pro Leu Thr Pro Ser Gly
               660                 665                 670
   Glu Ala Pro Asn Gln Ala Leu Leu Arg Ile Leu Lys Glu Thr Glu Phe
           675                 680                 685
   Lys Lys Ile Lys Val Leu Gly Ser Gly Ala Phe Gly Thr Val Tyr Lys
       690                 695                 700
   Gly Leu Trp Ile Pro Glu Gly Glu Lys Val Lys Ile Pro Val Ala Ile
   705                 710                 715                 720
   Lys Glu Leu Arg Glu Ala Thr Ser Pro Lys Ala Asn Lys Glu Ile Leu
                   725                 730                 735
   Asp Glu Ala Tyr Val Met Ala Ser Val Asp Asn Pro His Val Cys Arg
               740                 745                 750
   Leu Leu Gly Ile Cys Leu Thr Ser Thr Val Gln Leu Ile Thr Gln Leu
           755                 760                 765
   Met Pro Phe Gly Cys Leu Leu Asp Tyr Val Arg Glu His Lys Asp Asn
       770                 775                 780
   Ile Gly Ser Gln Tyr Leu Leu Asn Trp Cys Val Gln Ile Ala Lys Gly
   785                 790                 795                 800
   Met Asn Tyr Leu Glu Asp Arg Arg Leu Val His Arg Asp Leu Ala Ala
                   805                 810                 815
   Arg Asn Val Leu Val Lys Thr Pro Gln His Val Lys Ile Thr Asp Phe
               820                 825                 830
   Gly Leu Ala Lys Leu Leu Gly Ala Glu Glu Lys Glu Tyr His Ala Glu
           835                 840                 845
   Gly Gly Lys Val Pro Ile Lys Trp Met Ala Leu Glu Ser Ile Leu His
       850                 855                 860
   Arg Ile Tyr Thr His Gln Ser Asp Val Trp Ser Tyr Gly Val Thr Val
   865                 870                 875                 880
   Trp Glu Leu Met Thr Phe Gly Ser Lys Pro Tyr Asp Gly Ile Pro Ala
                   885                 890                 895
   Ser Glu Ile Ser Ser Ile Leu Glu Lys Gly Glu Arg Leu Pro Gln Pro
   900 905 910
   Pro Ile Cys Thr Ile Asp Val Tyr Met Ile Met Val Lys Cys Trp Met
   915 920 925
   Ile Asp Ala Asp Ser Arg Pro Lys Phe Arg Glu Leu Ile Ile Glu Phe
   930 935 940
   Ser Lys Met Ala Arg Asp Pro Gln Arg Tyr Leu Val Ile Gln Gly Asp
   945 950 955 960
   Glu Arg Met His Leu Pro Ser Pro Thr Asp Ser Asn Phe Tyr Arg Ala
   965 970 975
   Leu Met Asp Glu Glu Asp Met Asp Asp Val Val Asp Ala Asp Glu Tyr
   980 985 990
   Leu Ile Pro Gln Gln Gly Phe Phe Ser Ser Pro Ser Thr Ser Arg Thr
   995 1000 1005
   Pro Leu Leu Ser Ser Leu Ser Ala Thr Ser Asn Asn Ser Thr Val
   1010 1015 1020
   Ala Cys Ile Asp Arg Asn Gly Leu Gln Ser Cys Pro Ile Lys Glu
   1025 1030 1035
   Asp Ser Phe Leu Gln Arg Tyr Ser Ser Asp Pro Thr Gly Ala Leu
   1040 1045 1050
   Thr Glu Asp Ser Ile Asp Asp Thr Phe Leu Pro Val Pro Glu Tyr
   1055 1060 1065
   Ile Asn Gln Ser Val Pro Lys Arg Pro Ala Gly Ser Val Gln Asn
   1070 1075 1080
   Pro Val Tyr His Asn Gln Pro Leu Asn Pro Ala Pro Ser Arg Asp
   1085 1090 1095
   Pro His Tyr Gln Asp Pro His Ser Thr Ala Val Gly Asn Pro Glu
   1100 1105 1110
   Tyr Leu Asn Thr Val Gln Pro Thr Cys Val Asn Ser Thr Phe Asp
   1115 1120 1125
   Ser Pro Ala His Trp Ala Gln Lys Gly Ser His Gln Ile Ser Leu
   1130 1135 1140
   Asp Asn  Pro Asp Tyr Gln Gln  Asp Phe Phe Pro Lys  Glu Ala Lys
       1145                 1150                 1155
   Pro Asn  Gly Ile Phe Lys Gly  Ser Thr Ala Glu Asn  Ala Glu Tyr
       1160                 1165                 1170
   Leu Arg  Val Ala Pro Gln Ser  Ser Glu Phe Ile Gly  Ala
       1175                 1180                 1185
<210> 5
   <211> 724
   <212> PRT
   <213> Artificial Sequence
<220>
   <223> Synthetic Polypeptide
<400> 5
   Met Ala Pro Trp Pro Glu Leu Gly Asp Ala Gln Pro Asn Pro Asp Lys
   1               5                   10                  15
   Tyr Leu Glu Gly Ala Ala Gly Gln Gln Pro Thr Ala Pro Asp Lys Ser
               20                  25                  30
   Lys Glu Thr Asn Lys Thr Asp Asn Thr Glu Ala Pro Val Thr Lys Ile
           35                  40                  45
   Glu Leu Leu Pro Ser Tyr Ser Thr Ala Thr Leu Ile Asp Glu Pro Thr
       50                  55                  60
   Glu Val Asp Asp Pro Trp Asn Leu Pro Thr Leu Gln Asp Ser Gly Ile
   65                  70                  75                  80
   Lys Trp Ser Glu Arg Asp Thr Lys Gly Lys Ile Leu Cys Phe Phe Gln
                   85                  90                  95
   Gly Ile Gly Arg Leu Ile Leu Leu Leu Gly Phe Leu Tyr Phe Phe Val
               100                 105                 110
   Cys Ser Leu Asp Ile Leu Ser Ser Ala Phe Gln Leu Val Gly Ala Gly
           115                 120                 125
   Val Pro Asn Lys Pro Gly Ile Pro Lys Leu Leu Glu Gly Ser Lys Asn
       130                 135                 140
   Ser Ile Gln Trp Glu Lys Ala Glu Asp Asn Gly Cys Arg Ile Thr Tyr
   145                 150                 155                 160
   Tyr Ile Leu Glu Ile Arg Lys Ser Thr Ser Asn Asn Leu Gln Asn Gln
   165 170 175
   Asn Leu Arg Trp Lys Met Thr Phe Asn Gly Ser Cys Ser Ser Val Cys
   180 185 190
   Thr Trp Lys Ser Lys Asn Leu Lys Gly Ile Phe Gln Phe Arg Val Val
   195 200 205
   Ala Ala Asn Asn Leu Gly Phe Gly Glu Tyr Ser Gly Ile Ser Glu Asn
   210 215 220
   Ile Ile Leu Val Gly Asp Asp Phe Trp Ile Pro Glu Thr Ser Phe Ile
   225 230 235 240
   Leu Thr Ile Ile Val Gly Ile Phe Leu Val Val Thr Ile Pro Leu Thr
   245 250 255
   Phe Val Trp His Arg Arg Leu Lys Asn Gln Lys Ser Ala Lys Glu Gly
   260 265 270
   Val Thr Val Leu Ile Asn Glu Asp Lys Glu Leu Ala Glu Leu Arg Gly
   275 280 285
   Leu Ala Ala Gly Val Gly Leu Ala Asn Ala Cys Tyr Ala Ile His Thr
   290 295 300
   Leu Pro Thr Gln Glu Glu Ile Glu Asn Leu Pro Ala Phe Pro Arg Glu
   305 310 315 320
   Lys Leu Thr Leu Arg Leu Leu Leu Gly Ser Gly Ala Phe Gly Glu Val
   325 330 335
   Tyr Glu Gly Thr Ala Val Asp Ile Leu Gly Val Gly Ser Gly Glu Ile
   340 345 350
   Lys Val Ala Val Lys Thr Leu Lys Lys Gly Ser Thr Asp Gln Glu Lys
   355 360 365
   Ile Glu Phe Leu Lys Glu Ala His Leu Met Ser Lys Phe Asn His Pro
   370 375 380
   Asn Ile Leu Lys Gln Leu Gly Val Cys Leu Leu Asn Glu Pro Gln Tyr
   385 390 395 400
   Ile Ile Leu Glu Leu Met Glu Gly Gly Asp Leu Leu Thr Tyr Leu Arg
   405 410 415
   Lys Ala Arg Met Ala Thr Phe Tyr Gly Pro Leu Leu Thr Leu Val Asp
               420                 425                 430
   Leu Val Asp Leu Cys Val Asp Ile Ser Lys Gly Cys Val Tyr Leu Glu
           435                 440                 445
   Arg Met His Phe Ile His Arg Asp Leu Ala Ala Arg Asn Cys Leu Val
       450                 455                 460
   Ser Val Lys Asp Tyr Thr Ser Pro Arg Ile Val Lys Ile Gly Asp Phe
   465                 470                 475                 480
   Gly Leu Ala Arg Asp Ile Tyr Lys Asn Asp Tyr Tyr Arg Lys Arg Gly
                   485                 490                 495
   Glu Gly Leu Leu Pro Val Arg Trp Met Ala Pro Glu Ser Leu Met Asp
               500                 505                 510
   Gly Ile Phe Thr Thr Gln Ser Asp Val Trp Ser Phe Gly Ile Leu Ile
           515                 520                 525
   Trp Glu Ile Leu Thr Leu Gly His Gln Pro Tyr Pro Ala His Ser Asn
       530                 535                 540
   Leu Asp Val Leu Asn Tyr Val Gln Thr Gly Gly Arg Leu Glu Pro Pro
   545                 550                 555                 560
   Arg Asn Cys Pro Asp Asp Leu Trp Asn Leu Met Thr Gln Cys Trp Ala
                   565                 570                 575
   Gln Glu Pro Asp Gln Arg Pro Thr Phe His Arg Ile Gln Asp Gln Leu
               580                 585                 590
   Gln Leu Phe Arg Asn Phe Phe Leu Asn Ser Ile Tyr Lys Ser Arg Asp
           595                 600                 605
   Glu Ala Asn Asn Ser Gly Val Ile Asn Glu Ser Phe Glu Gly Glu Asp
       610                 615                 620
   Gly Asp Val Ile Cys Leu Asn Ser Asp Asp Ile Met Pro Val Ala Leu
   625                 630                 635                 640
   Met Glu Thr Lys Asn Arg Glu Gly Leu Asn Tyr Met Val Leu Ala Thr
                   645                 650                 655
   Glu Cys Gly Gln Gly Glu Glu Lys Ser Glu Gly Pro Leu Gly Ser Gln
               660                 665                 670
   Glu Ser Glu Ser Cys Gly Leu Arg Lys Glu Glu Lys Glu Pro His Ala
   675 680 685
   Asp Lys Asp Phe Cys Gln Glu Lys Gln Val Ala Tyr Cys Pro Ser Gly
   690 695 700
   Lys Pro Glu Gly Leu Asn Tyr Ala Cys Leu Thr His Ser Gly Tyr Gly
   705 710 715 720
   Asp Gly Ser Asp
<210> 6
   <211> 2175
   <212> DNA
   <213> Artificial Sequence
<220>
   <223> Synthetic Polynucleotide
<400> 6
   atggctccct ggcctgaatt gggagatgcc cagcccaacc ccgataagta cctcgaaggg 60
   gccgcaggtc agcagcccac tgcccctgat aaaagcaaag agaccaacaa aacagataac 120
   actgaggcac ctgtaaccaa gattgaactt ctgccgtcct actccacggc tacactgata 180
   gatgagccca ctgaggtgga tgacccctgg aacctaccca ctcttcagga ctcggggatc 240
   aagtggtcag agagagacac caaagggaag attctctgtt tcttccaagg gattgggaga 300
   ttgattttac ttctcggatt tctctacttt ttcgtgtgct ccctggatat tcttagtagc 360
   gccttccagc tggttggagc tggagtccca aataaaccag gcattcccaa attactagaa 420
   gggagtaaaa attcaataca gtgggagaaa gctgaagata atggatgtag aattacatac 480
   tatatccttg agataagaaa gagcacttca aataatttac agaaccagaa tttaaggtgg 540
   aagatgacat ttaatggatc ctgcagtagt gtttgcacat ggaagtccaa aaacctgaaa 600
   ggaatatttc agttcagagt agtagctgca aataatctag ggtttggtga atatagtgga 660
   atcagtgaga atattatatt agttggagat gatttttgga taccagaaac aagtttcata 720
   cttactatta tagttggaat atttctggtt gttacaatcc cactgacctt tgtctggcat 780
   agaagattaa agaatcaaaa aagtgccaag gaaggggtga cagtgcttat aaacgaagac 840
   aaagagttgg ctgagctgcg aggtctggca gccggagtag gcctggctaa tgcctgctat 900
   gcaatacata ctcttccaac ccaagaggag attgaaaatc ttcctgcctt ccctcgggaa 960
   aaactgactc tgcgtctctt gctgggaagt ggagcctttg gagaagtgta tgaaggaaca 1020
   gcagtggaca tcttaggagt tggaagtgga gaaatcaaag tagcagtgaa gactttgaag 1080
   aagggttcca cagaccagga gaagattgaa ttcctgaagg aggcacatct gatgagcaaa 1140
   tttaatcatc ccaacattct gaagcagctt ggagtttgtc tgctgaatga accccaatac     1200
   attatcctgg aactgatgga gggaggagac cttcttactt atttgcgtaa agcccggatg     1260
   gcaacgtttt atggtccttt actcaccttg gttgaccttg tagacctgtg tgtagatatt     1320
   tcaaaaggct gtgtctactt ggaacggatg catttcattc acagggatct ggcagctaga     1380
   aattgccttg tttccgtgaa agactatacc agtccacgga tagtgaagat tggagacttt     1440
   ggactcgcca gagacatcta taaaaatgat tactatagaa agagagggga aggcctgctc     1500
   ccagttcggt ggatggctcc agaaagtttg atggatggaa tcttcactac tcaatctgat     1560
   gtatggtctt ttggaattct gatttgggag attttaactc ttggtcatca gccttatcca     1620
   gctcattcca accttgatgt gttaaactat gtgcaaacag gagggagact ggagccacca     1680
   agaaattgtc ctgatgatct gtggaattta atgacccagt gctgggctca agaacccgac     1740
   caaagaccta cttttcatag aattcaggac caacttcagt tattcagaaa ttttttctta     1800
   aatagcattt ataagtccag agatgaagca aacaacagtg gagtcataaa tgaaagcttt     1860
   gaaggtgaag atggcgatgt gatttgtttg aattcagatg acattatgcc agttgcttta     1920
   atggaaacga agaaccgaga agggttaaac tatatggtac ttgctacaga atgtggccaa     1980
   ggtgaagaaa agtctgaggg tcctctaggc tcccaggaat ctgaatcttg tggtctgagg     2040
   aaagaagaga aggaaccaca tgcagacaaa gatttctgcc aagaaaaaca agtggcttac     2100
   tgcccttctg gcaagcctga aggcctgaac tatgcctgtc tcactcacag tggatatgga     2160
   gatgggtctg attaa                                                      2175
<210> 7
   <211> 621
   <212> PRT
   <213> Artificial Sequence
<220>
   <223> Synthetic Polypeptide
<400> 7
   Met Ala Pro Trp Pro Glu Leu Gly Asp Ala Gln Pro Asn Pro Asp Lys
   1               5                   10                  15
   Tyr Leu Glu Gly Ala Ala Gly Gln Gln Pro Thr Ala Pro Asp Lys Ser
               20                  25                  30
   Lys Glu Thr Asn Lys Thr Asp Asn Thr Glu Ala Pro Val Thr Lys Ile
           35                  40                  45
   Glu Leu Leu Pro Ser Tyr Ser Thr Ala Thr Leu Ile Asp Glu Pro Thr
       50                  55                  60
   Glu Val Asp Asp Pro Trp Asn Leu Pro Thr Leu Gln Asp Ser Gly Ile
   65                  70                  75                  80
   Lys Trp Ser Glu Arg Asp Thr Lys Gly Lys Ile Leu Cys Phe Phe Gln
                   85                  90                  95
   Gly Ile Gly Arg Leu Ile Leu Leu Leu Gly Phe Leu Tyr Phe Phe Val
               100                 105                 110
   Cys Ser Leu Asp Ile Leu Ser Ser Ala Phe Gln Leu Val Gly Asp Asp
           115                 120                 125
   Phe Trp Ile Pro Glu Thr Ser Phe Ile Leu Thr Ile Ile Val Gly Ile
       130                 135                 140
   Phe Leu Val Val Thr Ile Pro Leu Thr Phe Val Trp His Arg Arg Leu
   145                 150                 155                 160
   Lys Asn Gln Lys Ser Ala Lys Glu Gly Val Thr Val Leu Ile Asn Glu
                   165                 170                 175
   Asp Lys Glu Leu Ala Glu Leu Arg Gly Leu Ala Ala Gly Val Gly Leu
               180                 185                 190
   Ala Asn Ala Cys Tyr Ala Ile His Thr Leu Pro Thr Gln Glu Glu Ile
           195                 200                 205
   Glu Asn Leu Pro Ala Phe Pro Arg Glu Lys Leu Thr Leu Arg Leu Leu
       210                 215                 220
   Leu Gly Ser Gly Ala Phe Gly Glu Val Tyr Glu Gly Thr Ala Val Asp
   225                 230                 235                 240
   Ile Leu Gly Val Gly Ser Gly Glu Ile Lys Val Ala Val Lys Thr Leu
                   245                 250                 255
   Lys Lys Gly Ser Thr Asp Gln Glu Lys Ile Glu Phe Leu Lys Glu Ala
               260                 265                 270
   His Leu Met Ser Lys Phe Asn His Pro Asn Ile Leu Lys Gln Leu Gly
           275                 280                 285
   Val Cys Leu Leu Asn Glu Pro Gln Tyr Ile Ile Leu Glu Leu Met Glu
       290                 295                 300
   Gly Gly Asp Leu Leu Thr Tyr Leu Arg Lys Ala Arg Met Ala Thr Phe
   305                 310                 315                 320
   Tyr Gly Pro Leu Leu Thr Leu Val Asp Leu Val Asp Leu Cys Val Asp
                   325                 330                 335
   Ile Ser Lys Gly Cys Val Tyr Leu Glu Arg Met His Phe Ile His Arg
               340                 345                 350
   Asp Leu Ala Ala Arg Asn Cys Leu Val Ser Val Lys Asp Tyr Thr Ser
           355                 360                 365
   Pro Arg Ile Val Lys Ile Gly Asp Phe Gly Leu Ala Arg Asp Ile Tyr
       370                 375                 380
   Lys Asn Asp Tyr Tyr Arg Lys Arg Gly Glu Gly Leu Leu Pro Val Arg
   385                 390                 395                 400
   Trp Met Ala Pro Glu Ser Leu Met Asp Gly Ile Phe Thr Thr Gln Ser
                   405                 410                 415
   Asp Val Trp Ser Phe Gly Ile Leu Ile Trp Glu Ile Leu Thr Leu Gly
               420                 425                 430
   His Gln Pro Tyr Pro Ala His Ser Asn Leu Asp Val Leu Asn Tyr Val
           435                 440                 445
   Gln Thr Gly Gly Arg Leu Glu Pro Pro Arg Asn Cys Pro Asp Asp Leu
       450                 455                 460
   Trp Asn Leu Met Thr Gln Cys Trp Ala Gln Glu Pro Asp Gln Arg Pro
   465                 470                 475                 480
   Thr Phe His Arg Ile Gln Asp Gln Leu Gln Leu Phe Arg Asn Phe Phe
                   485                 490                 495
   Leu Asn Ser Ile Tyr Lys Ser Arg Asp Glu Ala Asn Asn Ser Gly Val
               500                 505                 510
   Ile Asn Glu Ser Phe Glu Gly Glu Asp Gly Asp Val Ile Cys Leu Asn
           515                 520                 525
   Ser Asp Asp Ile Met Pro Val Ala Leu Met Glu Thr Lys Asn Arg Glu
       530                 535                 540
   Gly Leu Asn Tyr Met Val Leu Ala Thr Glu Cys Gly Gln Gly Glu Glu
   545                 550                 555                 560
   Lys Ser Glu Gly Pro Leu Gly Ser Gln Glu Ser Glu Ser Cys Gly Leu
   565 570 575
   Arg Lys Glu Glu Lys Glu Pro His Ala Asp Lys Asp Phe Cys Gln Glu
   580 585 590
   Lys Gln Val Ala Tyr Cys Pro Ser Gly Lys Pro Glu Gly Leu Asn Tyr
   595 600 605
   Ala Cys Leu Thr His Ser Gly Tyr Gly Asp Gly Ser Asp
   610 615 620
<210> 8
   <211> 1866
   <212> DNA
   <213> Artificial Sequence
<220>
   <223> Synthetic Polynucleotide
<400> 8
   atggctccct ggcctgaatt gggagatgcc cagcccaacc ccgataagta cctcgaaggg      60
   gccgcaggtc agcagcccac tgcccctgat aaaagcaaag agaccaacaa aacagataac     120
   actgaggcac ctgtaaccaa gattgaactt ctgccgtcct actccacggc tacactgata     180
   gatgagccca ctgaggtgga tgacccctgg aacctaccca ctcttcagga ctcggggatc     240
   aagtggtcag agagagacac caaagggaag attctctgtt tcttccaagg gattgggaga     300
   ttgattttac ttctcggatt tctctacttt ttcgtgtgct ccctggatat tcttagtagc     360
   gccttccagc tggttggaga tgatttttgg ataccagaaa caagtttcat acttactatt     420
   atagttggaa tatttctggt tgttacaatc ccactgacct ttgtctggca tagaagatta     480
   aagaatcaaa aaagtgccaa ggaaggggtg acagtgctta taaacgaaga caaagagttg     540
   gctgagctgc gaggtctggc agccggagta ggcctggcta atgcctgcta tgcaatacat     600
   actcttccaa cccaagagga gattgaaaat cttcctgcct tccctcggga aaaactgact     660
   ctgcgtctct tgctgggaag tggagccttt ggagaagtgt atgaaggaac agcagtggac     720
   atcttaggag ttggaagtgg agaaatcaaa gtagcagtga agactttgaa gaagggttcc     780
   acagaccagg agaagattga attcctgaag gaggcacatc tgatgagcaa atttaatcat     840
   cccaacattc tgaagcagct tggagtttgt ctgctgaatg aaccccaata cattatcctg     900
   gaactgatgg agggaggaga ccttcttact tatttgcgta aagcccggat ggcaacgttt     960
   tatggtcctt tactcacctt ggttgacctt gtagacctgt gtgtagatat ttcaaaaggc    1020
   tgtgtctact tggaacggat gcatttcatt cacagggatc tggcagctag aaattgcctt    1080
   gtttccgtga aagactatac cagtccacgg atagtgaaga ttggagactt tggactcgcc    1140
   agagacatct ataaaaatga ttactataga aagagagggg aaggcctgct cccagttcgg    1200
   tggatggctc cagaaagttt gatggatgga atcttcacta ctcaatctga tgtatggtct 1260
   tttggaattc tgatttggga gattttaact cttggtcatc agccttatcc agctcattcc 1320
   aaccttgatg tgttaaacta tgtgcaaaca ggagggagac tggagccacc aagaaattgt 1380
   cctgatgatc tgtggaattt aatgacccag tgctgggctc aagaacccga ccaaagacct 1440
   acttttcata gaattcagga ccaacttcag ttattcagaa attttttctt aaatagcatt 1500
   tataagtcca gagatgaagc aaacaacagt ggagtcataa atgaaagctt tgaaggtgaa 1560
   gatggcgatg tgatttgttt gaattcagat gacattatgc cagttgcttt aatggaaacg 1620
   aagaaccgag aagggttaaa ctatatggta cttgctacag aatgtggcca aggtgaagaa 1680
   aagtctgagg gtcctctagg ctcccaggaa tctgaatctt gtggtctgag gaaagaagag 1740
   aaggaaccac atgcagacaa agatttctgc caagaaaaac aagtggctta ctgcccttct 1800
   ggcaagcctg aaggcctgaa ctatgcctgt ctcactcaca gtggatatgg agatgggtct 1860
   gattaa 1866
<210> 9
   <211> 5616
   <212> DNA
   <213> Artificial Sequence
<220>
   <223> Synthetic Polynucleotide
<400> 9
   ccccggcgca gcgcggccgc agcagcctcc gccccccgca cggtgtgagc gcccgacgcg      60
   gccgaggcgg ccggagtccc gagctagccc cggcggccgc cgccgcccag accggacgac     120
   aggccacctc gtcggcgtcc gcccgagtcc ccgcctcgcc gccaacgcca caaccaccgc     180
   gcacggcccc ctgactccgt ccagtattga tcgggagagc cggagcgagc tcttcgggga     240
   gcagcgatgc gaccctccgg gacggccggg gcagcgctcc tggcgctgct ggctgcgctc     300
   tgcccggcga gtcgggctct ggaggaaaag aaagtttgcc aaggcacgag taacaagctc     360
   acgcagttgg gcacttttga agatcatttt ctcagcctcc agaggatgtt caataactgt     420
   gaggtggtcc ttgggaattt ggaaattacc tatgtgcaga ggaattatga tctttccttc     480
   ttaaagacca tccaggaggt ggctggttat gtcctcattg ccctcaacac agtggagcga     540
   attcctttgg aaaacctgca gatcatcaga ggaaatatgt actacgaaaa ttcctatgcc     600
   ttagcagtct tatctaacta tgatgcaaat aaaaccggac tgaaggagct gcccatgaga     660
   aatttacagg aaatcctgca tggcgccgtg cggttcagca acaaccctgc cctgtgcaac     720
   gtggagagca tccagtggcg ggacatagtc agcagtgact ttctcagcaa catgtcgatg     780
   gacttccaga accacctggg cagctgccaa aagtgtgatc caagctgtcc caatgggagc     840
   tgctggggtg caggagagga gaactgccag aaactgacca aaatcatctg tgcccagcag 900
   tgctccgggc gctgccgtgg caagtccccc agtgactgct gccacaacca gtgtgctgca 960
   ggctgcacag gcccccggga gagcgactgc ctggtctgcc gcaaattccg agacgaagcc 1020
   acgtgcaagg acacctgccc cccactcatg ctctacaacc ccaccacgta ccagatggat 1080
   gtgaaccccg agggcaaata cagctttggt gccacctgcg tgaagaagtg tccccgtaat 1140
   tatgtggtga cagatcacgg ctcgtgcgtc cgagcctgtg gggccgacag ctatgagatg 1200
   gaggaagacg gcgtccgcaa gtgtaagaag tgcgaagggc cttgccgcaa agtgtgtaac 1260
   ggaataggta ttggtgaatt taaagactca ctctccataa atgctacgaa tattaaacac 1320
   ttcaaaaact gcacctccat cagtggcgat ctccacatcc tgccggtggc atttaggggt 1380
   gactccttca cacatactcc tcctctggat ccacaggaac tggatattct gaaaaccgta 1440
   aaggaaatca cagggttttt gctgattcag gcttggcctg aaaacaggac ggacctccat 1500
   gcctttgaga acctagaaat catacgcggc aggaccaagc aacatggtca gttttctctt 1560
   gcagtcgtca gcctgaacat aacatccttg ggattacgct ccctcaagga gataagtgat 1620
   ggagatgtga taatttcagg aaacaaaaat ttgtgctatg caaatacaat aaactggaaa 1680
   aaactgtttg ggacctccgg tcagaaaacc aaaattataa gcaacagagg tgaaaacagc 1740
   tgcaaggcca caggccaggt ctgccatgcc ttgtgctccc ccgagggctg ctggggcccg 1800
   gagcccaggg actgcgtctc ttgccggaat gtcagccgag gcagggaatg cgtggacaag 1860
   tgcaaccttc tggagggtga gccaagggag tttgtggaga actctgagtg catacagtgc 1920
   cacccagagt gcctgcctca ggccatgaac atcacctgca caggacgggg accagacaac 1980
   tgtatccagt gtgcccacta cattgacggc ccccactgcg tcaagacctg cccggcagga 2040
   gtcatgggag aaaacaacac cctggtctgg aagtacgcag acgccggcca tgtgtgccac 2100
   ctgtgccatc caaactgcac ctacggatgc actgggccag gtcttgaagg ctgtccaacg 2160
   aatgggccta agatcccgtc catcgccact gggatggtgg gggccctcct cttgctgctg 2220
   gtggtggccc tggggatcgg cctcttcatg cgaaggcgcc acatcgttcg gaagcgcacg 2280
   ctgcggaggc tgctgcagga gagggagctt gtggagcctc ttacacccag tggagaagct 2340
   cccaaccaag ctctcttgag gatcttgaag gaaactgaat tcaaaaagat caaagtgctg 2400
   ggctccggtg cgttcggcac ggtgtataag ggactctgga tcccagaagg tgagaaagtt 2460
   aaaattcccg tcgctatcaa ggaattaaga gaagcaacat ctccgaaagc caacaaggaa 2520
   atcctcgatg aagcctacgt gatggccagc gtggacaacc cccacgtgtg ccgcctgctg 2580
   ggcatctgcc tcacctccac cgtgcagctc atcacgcagc tcatgccctt cggctgcctc 2640
   ctggactatg tccgggaaca caaagacaat attggctccc agtacctgct caactggtgt 2700
   gtgcagatcg caaagggcat gaactacttg gaggaccgtc gcttggtgca ccgcgacctg 2760
   gcagccagga acgtactggt gaaaacaccg cagcatgtca agatcacaga ttttgggctg 2820
   gccaaactgc tgggtgcgga agagaaagaa taccatgcag aaggaggcaa agtgcctatc 2880
   aagtggatgg cattggaatc aattttacac agaatctata cccaccagag tgatgtctgg 2940
   agctacgggg tgaccgtttg ggagttgatg acctttggat ccaagccata tgacggaatc 3000
   cctgccagcg agatctcctc catcctggag aaaggagaac gcctccctca gccacccata 3060
   tgtaccatcg atgtctacat gatcatggtc aagtgctgga tgatagacgc agatagtcgc 3120
   ccaaagttcc gtgagttgat catcgaattc tccaaaatgg cccgagaccc ccagcgctac 3180
   cttgtcattc agggggatga aagaatgcat ttgccaagtc ctacagactc caacttctac 3240
   cgtgccctga tggatgaaga agacatggac gacgtggtgg atgccgacga gtacctcatc 3300
   ccacagcagg gcttcttcag cagcccctcc acgtcacgga ctcccctcct gagctctctg 3360
   agtgcaacca gcaacaattc caccgtggct tgcattgata gaaatgggct gcaaagctgt 3420
   cccatcaagg aagacagctt cttgcagcga tacagctcag accccacagg cgccttgact 3480
   gaggacagca tagacgacac cttcctccca gtgcctgaat acataaacca gtccgttccc 3540
   aaaaggcccg ctggctctgt gcagaatcct gtctatcaca atcagcctct gaaccccgcg 3600
   cccagcagag acccacacta ccaggacccc cacagcactg cagtgggcaa ccccgagtat 3660
   ctcaacactg tccagcccac ctgtgtcaac agcacattcg acagccctgc ccactgggcc 3720
   cagaaaggca gccaccaaat tagcctggac aaccctgact accagcagga cttctttccc 3780
   aaggaagcca agccaaatgg catctttaag ggctccacag ctgaaaatgc agaataccta 3840
   agggtcgcgc cacaaagcag tgaatttatt ggagcatgac cacggaggat agtatgagcc 3900
   ctaaaaatcc agactctttc gatacccagg accaagccac agcaggtcct ccatcccaac 3960
   agccatgccc gcattagctc ttagacccac agactggttt tgcaacgttt acaccgacta 4020
   gccaggaagt acttccacct cgggcacatt ttgggaagtt gcattccttt gtcttcaaac 4080
   tgtgaagcat ttacagaaac gcatccagca agaatattgt ccctttgagc agaaatttat 4140
   ctttcaaaga ggtatatttg aaaaaaaaaa aaagtatatg tgaggatttt tattgattgg 4200
   ggatcttgga gtttttcatt gtcgctattg atttttactt caatgggctc ttccaacaag 4260
   gaagaagctt gctggtagca cttgctaccc tgagttcatc caggcccaac tgtgagcaag 4320
   gagcacaagc cacaagtctt ccagaggatg cttgattcca gtggttctgc ttcaaggctt 4380
   ccactgcaaa acactaaaga tccaagaagg ccttcatggc cccagcaggc cggatcggta 4440
   ctgtatcaag tcatggcagg tacagtagga taagccactc tgtcccttcc tgggcaaaga 4500
   agaaacggag gggatggaat tcttccttag acttactttt gtaaaaatgt ccccacggta 4560
   cttactcccc actgatggac cagtggtttc cagtcatgag cgttagactg acttgtttgt 4620
   cttccattcc attgttttga aactcagtat gctgcccctg tcttgctgtc atgaaatcag 4680
   caagagagga tgacacatca aataataact cggattccag cccacattgg attcatcagc 4740
   atttggacca atagcccaca gctgagaatg tggaatacct aaggatagca ccgcttttgt 4800
   tctcgcaaaa acgtatctcc taatttgagg ctcagatgaa atgcatcagg tcctttgggg 4860
   catagatcag aagactacaa aaatgaagct gctctgaaat ctcctttagc catcacccca 4920
   accccccaaa attagtttgt gttacttatg gaagatagtt ttctcctttt acttcacttc 4980
   aaaagctttt tactcaaaga gtatatgttc cctccaggtc agctgccccc aaaccccctc 5040
   cttacgcttt gtcacacaaa aagtgtctct gccttgagtc atctattcaa gcacttacag 5100
   ctctggccac aacagggcat tttacaggtg cgaatgacag tagcattatg agtagtgtgg 5160
   aattcaggta gtaaatatga aactagggtt tgaaattgat aatgctttca caacatttgc 5220
   agatgtttta gaaggaaaaa agttccttcc taaaataatt tctctacaat tggaagattg 5280
   gaagattcag ctagttagga gcccaccttt tttcctaatc tgtgtgtgcc ctgtaacctg 5340
   actggttaac agcagtcctt tgtaaacagt gttttaaact ctcctagtca atatccaccc 5400
   catccaattt atcaaggaag aaatggttca gaaaatattt tcagcctaca gttatgttca 5460
   gtcacacaca catacaaaat gttccttttg cttttaaagt aatttttgac tcccagatca 5520
   gtcagagccc ctacagcatt gttaagaaag tatttgattt ttgtctcaat gaaaataaaa 5580
   ctatattcat ttccactcta aaaaaaaaaa aaaaaa 5616
<210> 10
   <211> 24
   <212> DNA
   <213> Artificial Sequence
<220>
   <223> Synthetic Polynucleotide
<400> 10
   gcagctcagc caactctttg tctt 24
<210> 11
   <211> 703
   <212> PRT
   <213> Artificial Sequence
<220>
   <223> Synthetic Polypeptide
<400> 11
   Met His Arg Arg Arg Ser Arg Ser Cys Arg Glu Asp Gln Lys Pro Val
   1               5                   10                  15
   Met Asp Asp Gln Arg Asp Leu Ile Ser Asn Asn Glu Gln Leu Pro Met
               20                  25                  30
   Leu Gly Arg Arg Pro Gly Ala Pro Glu Ser Lys Cys Ser Arg Gly Ala
           35                  40                  45
   Leu Tyr Thr Gly Phe Ser Ile Leu Val Thr Leu Leu Leu Ala Gly Gln
       50                  55                  60
   Ala Thr Thr Ala Tyr Phe Leu Tyr Gln Gln Gln Gly Arg Leu Asp Lys
   65                  70                  75                  80
   Leu Thr Val Thr Ser Gln Asn Leu Gln Leu Glu Asn Leu Arg Met Lys
                   85                  90                  95
   Leu Pro Lys Pro Pro Lys Pro Val Ser Lys Met Arg Met Ala Thr Pro
               100                 105                 110
   Leu Leu Met Gln Ala Leu Pro Met Gly Ala Leu Pro Gln Gly Pro Met
           115                 120                 125
   Gln Asn Ala Thr Lys Tyr Gly Asn Met Thr Glu Asp His Val Met His
       130                 135                 140
   Leu Leu Gln Asn Ala Asp Pro Leu Lys Val Tyr Pro Pro Leu Lys Gly
   145                 150                 155                 160
   Ser Phe Pro Glu Asn Leu Arg His Leu Lys Asn Thr Met Glu Thr Ile
                   165                 170                 175
   Asp Trp Lys Val Phe Glu Ser Trp Met His His Trp Leu Leu Phe Glu
               180                 185                 190
   Met Ser Arg His Ser Leu Glu Gln Lys Pro Thr Asp Ala Pro Pro Lys
           195                 200                 205
   Asp Asp Phe Trp Ile Pro Glu Thr Ser Phe Ile Leu Thr Ile Ile Val
       210                 215                 220
   Gly Ile Phe Leu Val Val Thr Ile Pro Leu Thr Phe Val Trp His Arg
   225                 230                 235                 240
   Arg Leu Lys Asn Gln Lys Ser Ala Lys Glu Gly Val Thr Val Leu Ile
                   245                 250                 255
   Asn Glu Asp Lys Glu Leu Ala Glu Leu Arg Gly Leu Ala Ala Gly Val
               260                 265                 270
   Gly Leu Ala Asn Ala Cys Tyr Ala Ile His Thr Leu Pro Thr Gln Glu
   275 280 285
   Glu Ile Glu Asn Leu Pro Ala Phe Pro Arg Glu Lys Leu Thr Leu Arg
   290 295 300
   Leu Leu Leu Gly Ser Gly Ala Phe Gly Glu Val Tyr Glu Gly Thr Ala
   305 310 315 320
   Val Asp Ile Leu Gly Val Gly Ser Gly Glu Ile Lys Val Ala Val Lys
   325 330 335
   Thr Leu Lys Lys Gly Ser Thr Asp Gln Glu Lys Ile Glu Phe Leu Lys
   340 345 350
   Glu Ala His Leu Met Ser Lys Phe Asn His Pro Asn Ile Leu Lys Gln
   355 360 365
   Leu Gly Val Cys Leu Leu Asn Glu Pro Gln Tyr Ile Ile Leu Glu Leu
   370 375 380
   Met Glu Gly Gly Asp Leu Leu Thr Tyr Leu Arg Lys Ala Arg Met Ala
   385 390 395 400
   Thr Phe Tyr Gly Pro Leu Leu Thr Leu Val Asp Leu Val Asp Leu Cys
   405 410 415
   Val Asp Ile Ser Lys Gly Cys Val Tyr Leu Glu Arg Met His Phe Ile
   420 425 430
   His Arg Asp Leu Ala Ala Arg Asn Cys Leu Val Ser Val Lys Asp Tyr
   435 440 445
   Thr Ser Pro Arg Ile Val Lys Ile Gly Asp Phe Gly Leu Ala Arg Asp
   450 455 460
   Ile Tyr Lys Asn Asp Tyr Tyr Arg Lys Arg Gly Glu Gly Leu Leu Pro
   465 470 475 480
   Val Arg Trp Met Ala Pro Glu Ser Leu Met Asp Gly Ile Phe Thr Thr
   485 490 495
   Gln Ser Asp Val Trp Ser Phe Gly Ile Leu Ile Trp Glu Ile Leu Thr
   500 505 510
   Leu Gly His Gln Pro Tyr Pro Ala His Ser Asn Leu Asp Val Leu Asn
   515 520 525
   Tyr Val Gln Thr Gly Gly Arg Leu Glu Pro Pro Arg Asn Cys Pro Asp
       530                 535                 540
   Asp Leu Trp Asn Leu Met Thr Gln Cys Trp Ala Gln Glu Pro Asp Gln
   545                 550                 555                 560
   Arg Pro Thr Phe His Arg Ile Gln Asp Gln Leu Gln Leu Phe Arg Asn
                   565                 570                 575
   Phe Phe Leu Asn Ser Ile Tyr Lys Ser Arg Asp Glu Ala Asn Asn Ser
               580                 585                 590
   Gly Val Ile Asn Glu Ser Phe Glu Gly Glu Asp Gly Asp Val Ile Cys
           595                 600                 605
   Leu Asn Ser Asp Asp Ile Met Pro Val Ala Leu Met Glu Thr Lys Asn
       610                 615                 620
   Arg Glu Gly Leu Asn Tyr Met Val Leu Ala Thr Glu Cys Gly Gln Gly
   625                 630                 635                 640
   Glu Glu Lys Ser Glu Gly Pro Leu Gly Ser Gln Glu Ser Glu Ser Cys
                   645                 650                 655
   Gly Leu Arg Lys Glu Glu Lys Glu Pro His Ala Asp Lys Asp Phe Cys
               660                 665                 670
   Gln Glu Lys Gln Val Ala Tyr Cys Pro Ser Gly Lys Pro Glu Gly Leu
           675                 680                 685
   Asn Tyr Ala Cys Leu Thr His Ser Gly Tyr Gly Asp Gly Ser Asp
       690                 695                 700
<210> 12
   <211> 2112
   <212> DNA
   <213> Artificial Sequence
<220>
   <223> Synthetic Polynucleotide
<400> 12
   atgcacagga ggagaagcag gagctgtcgg gaagatcaga agccagtcat ggatgaccag      60
   cgcgacctta tctccaacaa tgagcaactg cccatgctgg gccggcgccc tggggccccg     120
   gagagcaagt gcagccgcgg agccctgtac acaggctttt ccatcctggt gactctgctc     180
   ctcgctggcc aggccaccac cgcctacttc ctgtaccagc agcagggccg gctggacaaa     240
   ctgacagtca cctcccagaa cctgcagctg gagaacctgc gcatgaagct tcccaagcct     300
   cccaagcctg tgagcaagat gcgcatggcc accccgctgc tgatgcaggc gctgcccatg 360
   ggagccctgc cccaggggcc catgcagaat gccaccaagt atggcaacat gacagaggac 420
   catgtgatgc acctgctcca gaatgctgac cccctgaagg tgtacccgcc actgaagggg 480
   agcttcccgg agaacctgag acaccttaag aacaccatgg agaccataga ctggaaggtc 540
   tttgagagct ggatgcacca ttggctcctg tttgaaatga gcaggcactc cttggagcaa 600
   aagcccactg acgctccacc gaaagatgat ttttggatac cagaaacaag tttcatactt 660
   actattatag ttggaatatt tctggttgtt acaatcccac tgacctttgt ctggcataga 720
   agattaaaga atcaaaaaag tgccaaggaa ggggtgacag tgcttataaa cgaagacaaa 780
   gagttggctg agctgcgagg tctggcagcc ggagtaggcc tggctaatgc ctgctatgca 840
   atacatactc ttccaaccca agaggagatt gaaaatcttc ctgccttccc tcgggaaaaa 900
   ctgactctgc gtctcttgct gggaagtgga gcctttggag aagtgtatga aggaacagca 960
   gtggacatct taggagttgg aagtggagaa atcaaagtag cagtgaagac tttgaagaag 1020
   ggttccacag accaggagaa gattgaattc ctgaaggagg cacatctgat gagcaaattt 1080
   aatcatccca acattctgaa gcagcttgga gtttgtctgc tgaatgaacc ccaatacatt 1140
   atcctggaac tgatggaggg aggagacctt cttacttatt tgcgtaaagc ccggatggca 1200
   acgttttatg gtcctttact caccttggtt gaccttgtag acctgtgtgt agatatttca 1260
   aaaggctgtg tctacttgga acggatgcat ttcattcaca gggatctggc agctagaaat 1320
   tgccttgttt ccgtgaaaga ctataccagt ccacggatag tgaagattgg agactttgga 1380
   ctcgccagag acatctataa aaatgattac tatagaaaga gaggggaagg cctgctccca 1440
   gttcggtgga tggctccaga aagtttgatg gatggaatct tcactactca atctgatgta 1500
   tggtcttttg gaattctgat ttgggagatt ttaactcttg gtcatcagcc ttatccagct 1560
   cattccaacc ttgatgtgtt aaactatgtg caaacaggag ggagactgga gccaccaaga 1620
   aattgtcctg atgatctgtg gaatttaatg acccagtgct gggctcaaga acccgaccaa 1680
   agacctactt ttcatagaat tcaggaccaa cttcagttat tcagaaattt tttcttaaat 1740
   agcatttata agtccagaga tgaagcaaac aacagtggag tcataaatga aagctttgaa 1800
   ggtgaagatg gcgatgtgat ttgtttgaat tcagatgaca ttatgccagt tgctttaatg 1860
   gaaacgaaga accgagaagg gttaaactat atggtacttg ctacagaatg tggccaaggt 1920
   gaagaaaagt ctgagggtcc tctaggctcc caggaatctg aatcttgtgg tctgaggaaa 1980
   gaagagaagg aaccacatgc agacaaagat ttctgccaag aaaaacaagt ggcttactgc 2040
   ccttctggca agcctgaagg cctgaactat gcctgtctca ctcacagtgg atatggagat 2100
   gggtctgatt aa 2112
<210> 13
   <211> 592
   <212> PRT
   <213> Artificial Sequence
<220>
   <223> Synthetic Polypeptide
<400> 13
   Met Ala Pro Trp Pro Glu Leu Gly Asp Ala Gln Pro Asn Pro Asp Lys
   1               5                   10                  15
   Tyr Leu Glu Gly Ala Ala Gly Gln Gln Pro Thr Ala Pro Asp Lys Ser
               20                  25                  30
   Lys Glu Thr Asn Lys Thr Asp Asn Thr Glu Ala Pro Val Thr Lys Ile
           35                  40                  45
   Glu Leu Leu Pro Ser Tyr Ser Thr Ala Thr Leu Ile Asp Glu Pro Thr
       50                  55                  60
   Glu Val Asp Asp Pro Trp Asn Leu Pro Thr Leu Gln Asp Ser Gly Ile
   65                  70                  75                  80
   Lys Trp Ser Glu Arg Asp Thr Lys Gly Lys Ile Leu Cys Phe Phe Gln
                   85                  90                  95
   Gly Ile Gly Arg Leu Ile Leu Leu Leu Gly Phe Leu Tyr Phe Phe Val
               100                 105                 110
   Cys Ser Leu Asp Ile Leu Ser Ser Ala Phe Gln Leu Val Gly Val Trp
           115                 120                 125
   His Arg Arg Leu Lys Asn Gln Lys Ser Ala Lys Glu Gly Val Thr Val
       130                 135                 140
   Leu Ile Asn Glu Asp Lys Glu Leu Ala Glu Leu Arg Gly Leu Ala Ala
   145                 150                 155                 160
   Gly Val Gly Leu Ala Asn Ala Cys Tyr Ala Ile His Thr Leu Pro Thr
                   165                 170                 175
   Gln Glu Glu Ile Glu Asn Leu Pro Ala Phe Pro Arg Glu Lys Leu Thr
               180                 185                 190
   Leu Arg Leu Leu Leu Gly Ser Gly Ala Phe Gly Glu Val Tyr Glu Gly
           195                 200                 205
   Thr Ala Val Asp Ile Leu Gly Val Gly Ser Gly Glu Ile Lys Val Ala
       210                 215                 220
   Val Lys Thr Leu Lys Lys Gly Ser Thr Asp Gln Glu Lys Ile Glu Phe
   225                 230                 235                 240
   Leu Lys Glu Ala His Leu Met Ser Lys Phe Asn His Pro Asn Ile Leu
                   245                 250                 255
   Lys Gln Leu Gly Val Cys Leu Leu Asn Glu Pro Gln Tyr Ile Ile Leu
               260                 265                 270
   Glu Leu Met Glu Gly Gly Asp Leu Leu Thr Tyr Leu Arg Lys Ala Arg
           275                 280                 285
   Met Ala Thr Phe Tyr Gly Pro Leu Leu Thr Leu Val Asp Leu Val Asp
       290                 295                 300
   Leu Cys Val Asp Ile Ser Lys Gly Cys Val Tyr Leu Glu Arg Met His
   305                 310                 315                 320
   Phe Ile His Arg Asp Leu Ala Ala Arg Cys Leu Val Ser Val Lys Asp
                   325                 330                 335
   Tyr Thr Ser Pro Arg Ile Val Lys Ile Gly Asp Phe Gly Leu Ala Arg
               340                 345                 350
   Asp Ile Tyr Lys Asn Asp Tyr Tyr Arg Lys Arg Gly Glu Gly Leu Leu
           355                 360                 365
   Pro Val Arg Trp Met Ala Pro Glu Ser Leu Met Asp Gly Ile Phe Thr
       370                 375                 380
   Thr Gln Ser Asp Val Trp Ser Phe Gly Ile Leu Ile Trp Glu Ile Leu
   385                 390                 395                 400
   Thr Leu Gly His Gln Pro Tyr Pro Ala His Ser Asn Leu Asp Val Leu
                   405                 410                 415
   Asn Tyr Val Gln Thr Gly Gly Arg Leu Glu Pro Pro Arg Asn Cys Pro
               420                 425                 430
   Asp Asp Leu Trp Asn Leu Met Thr Gln Cys Trp Ala Gln Glu Pro Asp
           435                 440                 445
   Gln Arg Pro Thr Phe His Arg Ile Gln Asp Gln Leu Gln Leu Phe Arg
       450                 455                 460
   Asn Phe Phe Leu Asn Ser Ile Tyr Lys Ser Arg Asp Glu Ala Asn Asn
   465                 470                 475                 480
   Ser Gly Val Ile Asn Glu Ser Phe Glu Gly Glu Asp Gly Asp Val Ile
                   485                 490                 495
   Cys Leu Asn Ser Asp Asp Ile Met Pro Val Ala Leu Met Glu Thr Lys
               500                 505                 510
   Asn Arg Glu Gly Leu Asn Tyr Met Val Leu Ala Thr Glu Cys Gly Gln
           515                 520                 525
   Gly Glu Glu Lys Ser Glu Gly Pro Leu Gly Ser Gln Glu Ser Glu Ser
       530                 535                 540
   Cys Gly Leu Arg Lys Glu Glu Lys Glu Pro His Ala Asp Lys Asp Phe
   545                 550                 555                 560
   Cys Gln Glu Lys Gln Val Ala Tyr Cys Pro Ser Gly Lys Pro Glu Gly
                   565                 570                 575
   Leu Asn Tyr Ala Cys Leu Thr His Ser Gly Tyr Gly Asp Gly Ser Asp
               580                 585                 590
<210> 14
   <211> 1782
   <212> DNA
   <213> Artificial Sequence
<220>
   <223> Synthetic Polynucleotide
<400> 14
   atggctccct ggcctgaatt gggagatgcc cagcccaacc ccgataagta cctcgaaggg      60
   gccgcaggtc agcagcccac tgcccctgat aaaagcaaag agaccaacaa aacagataac     120
   actgaggcac ctgtaaccaa gattgaactt ctgccgtcct actccacggc tacactgata     180
   gatgagccca ctgaggtgga tgacccctgg aacctaccca ctcttcagga ctcggggatc     240
   aagtggtcag agagagacac caaagggaag attctctgtt tcttccaagg gattgggaga     300
   ttgattttac ttctcggatt tctctacttt ttcgtgtgct ccctggatat tcttagtagc     360
   gccttccagc tggttggagt ctggcataga agattaaaga atcaaaaaag tgccaaggaa     420
   ggggtgacag tgcttataaa cgaagacaaa gagttggctg agctgcgagg tctggcagcc     480
   ggagtaggcc tggctaatgc ctgctatgca atacatactc ttccaaccca agaggagatt     540
   gaaaatcttc ctgccttccc tcgggaaaaa ctgactctgc gtctcttgct gggaagtgga     600
   gcctttggag aagtgtatga aggaacagca gtggacatct taggagttgg aagtggagaa     660
   atcaaagtag cagtgaagac tttgaagaag ggttccacag accaggagaa gattgaattc     720
   ctgaaggagg cacatctgat gagcaaattt aatcatccca acattctgaa gcagcttgga     780
   gtttgtctgc tgaatgaacc ccaatacatt atcctggaac tgatggaggg aggagacctt     840
   cttacttatt tgcgtaaagc ccggatggca acgttttatg gtcctttact caccttggtt     900
   gaccttgtag acctgtgtgt agatatttca aaaggctgtg tctacttgga acggatgcat     960
   ttcattcaca gggatctggc agctagaaat tgccttgttt ccgtgaaaga ctataccagt    1020
   ccacggatag tgaagattgg agactttgga ctcgccagag acatctataa aaatgattac    1080
   tatagaaaga gaggggaagg cctgctccca gttcggtgga tggctccaga aagtttgatg    1140
   gatggaatct tcactactca atctgatgta tggtcttttg gaattctgat ttgggagatt    1200
   ttaactcttg gtcatcagcc ttatccagct cattccaacc ttgatgtgtt aaactatgtg    1260
   caaacaggag ggagactgga gccaccaaga aattgtcctg atgatctgtg gaatttaatg    1320
   acccagtgct gggctcaaga acccgaccaa agacctactt ttcatagaat tcaggaccaa    1380
   cttcagttat tcagaaattt tttcttaaat agcatttata agtccagaga tgaagcaaac    1440
   aacagtggag tcataaatga aagctttgaa ggtgaagatg gcgatgtgat ttgtttgaat    1500
   tcagatgaca ttatgccagt tgctttaatg gaaacgaaga accgagaagg gttaaactat    1560
   atggtacttg ctacagaatg tggccaaggt gaagaaaagt ctgagggtcc tctaggctcc    1620
   caggaatctg aatcttgtgg tctgaggaaa gaagagaagg aaccacatgc agacaaagat    1680
   ttctgccaag aaaaacaagt ggcttactgc ccttctggca agcctgaagg cctgaactat    1740
   gcctgtctca ctcacagtgg atatggagat gggtctgatt aa                       1782
<210> 15
   <211> 34
   <212> PRT
   <213> Artificial Sequence
<220>
   <223> Synthetic Polypeptide
<400> 15
   Phe Glu Met Ser Arg His Ser Leu Glu Gln Lys Pro Thr Asp Ala Pro
   1               5                   10                  15
   Pro Lys Asp Asp Phe Trp Ile Pro Glu Thr Ser Phe Ile Leu Thr Ile
               20                  25                  30
   Ile Val
<210> 16
   <211> 21
   <212> DNA
   <213> Artificial Sequence
<220>
   <223> Synthetic Polynucleotide
<400> 16
   aagcccggau ggcaacguut t 21
<210> 17
   <211> 21
   <212> DNA
   <213> Artificial Sequence
<220>
   <223> Synthetic Polynucleotide
<400> 17
   aagccugaag gccugaacut t 21
<210> 18
   <211> 24
   <212> DNA
   <213> Artificial Sequence
<220>
   <223> Synthetic Polynucleotide
<400> 18
   cagcaagaga cgcagagtca gttt 24
<210> 19
   <211> 26
   <212> DNA
   <213> Artificial Sequence
<220>
   <223> Synthetic Polynucleotide
<400> 19
   gctgttctcc aggctgaagt atatgg 26
<210> 20
   <211> 24
   <212> DNA
   <213> Artificial Sequence
<220>
   <223> Synthetic Polynucleotide
<400> 20
   gtaaccctgg tgctagttgc aaag 24
<210> 21
   <211> 2637
   <212> DNA
   <213> Artificial Sequence
<220>
   <223> Synthetic Polynucleotide
<400> 21
   atgtcggcgg gcggtccatg cccagcagca gccggagggg gcccaggggg cgcctcctgc 60
   tccgtggggg cccctggcgg ggtatccatg ttccggtggc tggaggtgct ggagaaggag 120
   ttcgacaaag cttttgtgga tgtggatctg ctcctgggag agatcgatcc agaccaagcg 180
   gacatcactt atgaggggcg acagaagatg accagcctga gctcctgctt tgcacagctt 240
   tgccacaaag cccagtctgt gtctcaaatc aaccacaagc tggaggcaca gttggtggat 300
   ctgaaatctg aactgacaga aacccaagca gagaaagttg ttttggagaa agaagtacat 360
   gatcagcttt tacagctgca ctctattcag ctgcagcttc atgctaaaac tggtcaaagt 420
   gctgactctg gtaccattaa ggcaaaattg gaaagagagc ttgaggcaaa caaaaaagaa 480
   aaaatgaaag aagcacaact tgaagctgaa gtgaaattgt tgagaaaaga gaatgaagcc 540
   cttcgtagac atatagctgt tctccaggct gaagtatatg gggcgagact agctgccaag 600
   tacttggata aggaactggc aggaagggtc caacagatac aattgctagg acgagatatg 660
   aagggacctg ctcatgataa gctttggaac caattagaag ctgaaataca tttgcatcgt 720
   cacaaaactg tgatccgagc ctgcagagga cgtaatgact tgaaacgacc aatgcaagca 780
   ccaccaggcc atgatcaaga ttccctaaag aaaagccaag gtgttggtcc aattagaaaa 840
   gttctcctcc ttaaggaaga tcatgaaggc cttggcattt caattacagg tgggaaagaa 900
   catggtgttc caatcctcat ctctgagatc catccggggc aacctgctga tagatgcgga 960
   gggctgcacg ttggggatgc tattttggca gtcaacggag ttaacctaag ggacacaaag 1020
   cataaagaag ctgtaactat tctttctcag cagagaggag agattgaatt tgaagtagtt 1080
   tatgtggctc ctgaagtgga ttctgatgat gaaaacgtag agtatgaaga tgagagtgga 1140
   catcgttacc gtttgtacct tgatgagtta gaaggaggtg gtaaccctgg tgctagttgc 1200
   aaagacacaa gtggggaaat caaagtatta caagtctggc atagaagatt aaagaatcaa 1260
   aaaagtgcca aggaaggggt gacagtgctt ataaacgaag acaaagagtt ggctgagctg 1320
   cgaggtctgg cagccggagt aggcctggct aatgcctgct atgcaataca tactcttcca 1380
   acccaagagg agattgaaaa tcttcctgcc ttccctcggg aaaaactgac tctgcgtctc 1440
   ttgctgggaa gtggagcctt tggagaagtg tatgaaggaa cagcagtgga catcttagga 1500
   gttggaagtg gagaaatcaa agtagcagtg aagactttga agaagggttc cacagaccag 1560
   gagaagattg aattcctgaa ggaggcacat ctgatgagca aatttaatca tcccaacatt 1620
   ctgaagcagc ttggagtttg tctgctgaat gaaccccaat acattatcct ggaactgatg 1680
   gagggaggag accttcttac ttatttgcgt aaagcccgga tggcaacgtt ttatggtcct 1740
   ttactcacct tggttgacct tgtagacctg tgtgtagata tttcaaaagg ctgtgtctac 1800
   ttggaacgga tgcatttcat tcacagggat ctggcagcta gaaattgcct tgtttccgtg      1860
   aaagactata ccagtccacg gatagtgaag attggagact ttggactcgc cagagacatc      1920
   tataaaaatg attactatag aaagagaggg gaaggcctgc tcccagttcg gtggatggct      1980
   ccagaaagtt tgatggatgg aatcttcact actcaatctg atgtatggtc ttttggaatt      2040
   ctgatttggg agattttaac tcttggtcat cagccttatc cagctcattc caaccttgat      2100
   gtgttaaact atgtgcaaac aggagggaga ctggagccac caagaaattg tcctgatgat      2160
   ctgtggaatt taatgaccca gtgctgggct caagaacccg accaaagacc tacttttcat      2220
   agaattcagg accaacttca gttattcaga aatttttctt taaatagcat ttataagtcc      2280
   agagatgaag caaacaacag tggagtcata aatgaaagct ttgaaggtga agatggcgat      2340
   gtgatttgtt tgaattcaga tgacattatg ccagttgctt taatggaaac gaagaaccga      2400
   gaagggttaa actatatggt acttgctaca gaatgtggcc aaggtgaaga aaagtctgag      2460
   ggtcctctag gctcccagga atctgaatct tgtggtctga ggaaagaaga gaaggaacca      2520
   catgcagaca aagatttctg ccaagaaaaa caagtggctt actgcccttc tggcaagcct      2580
   gaaggcctga actatgcctg tctcactcac agtggatatg gagatgggtc tgattaa         2637
<210> 22
   <211> 878
   <212> PRT
   <213> Artificial Sequence
<220>
   <223> Synthetic Polypeptide
<400> 22
   Met Ser Ala Gly Gly Pro Cys Pro Ala Ala Ala Gly Gly Gly Pro Gly
   1               5                   10                  15
   Gly Ala Ser Cys Ser Val Gly Ala Pro Gly Gly Val Ser Met Phe Arg
               20                  25                  30
   Trp Leu Glu Val Leu Glu Lys Glu Phe Asp Lys Ala Phe Val Asp Val
           35                  40                  45
   Asp Leu Leu Leu Gly Glu Ile Asp Pro Asp Gln Ala Asp Ile Thr Tyr
       50                  55                  60
   Glu Gly Arg Gln Lys Met Thr Ser Leu Ser Ser Cys Phe Ala Gln Leu
   65                  70                  75                  80
   Cys His Lys Ala Gln Ser Val Ser Gln Ile Asn His Lys Leu Glu Ala
                   85                  90                  95
   Gln Leu Val Asp Leu Lys Ser Glu Leu Thr Glu Thr Gln Ala Glu Lys
               100                 105                 110
   Val Val Leu Glu Lys Glu Val His Asp Gln Leu Leu Gln Leu His Ser
           115                 120                 125
   Ile Gln Leu Gln Leu His Ala Lys Thr Gly Gln Ser Ala Asp Ser Gly
       130                 135                 140
   Thr Ile Lys Ala Lys Leu Glu Arg Glu Leu Glu Ala Asn Lys Lys Glu
   145                 150                 155                 160
   Lys Met Lys Glu Ala Gln Leu Glu Ala Glu Val Lys Leu Leu Arg Lys
                   165                 170                 175
   Glu Asn Glu Ala Leu Arg Arg His Ile Ala Val Leu Gln Ala Glu Val
               180                 185                 190
   Tyr Gly Ala Arg Leu Ala Ala Lys Tyr Leu Asp Lys Glu Leu Ala Gly
           195                 200                 205
   Arg Val Gln Gln Ile Gln Leu Leu Gly Arg Asp Met Lys Gly Pro Ala
       210                 215                 220
   His Asp Lys Leu Trp Asn Gln Leu Glu Ala Glu Ile His Leu His Arg
   225                 230                 235                 240
   His Lys Thr Val Ile Arg Ala Cys Arg Gly Arg Asn Asp Leu Lys Arg
                   245                 250                 255
   Pro Met Gln Ala Pro Pro Gly His Asp Gln Asp Ser Leu Lys Lys Ser
               260                 265                 270
   Gln Gly Val Gly Pro Ile Arg Lys Val Leu Leu Leu Lys Glu Asp His
           275                 280                 285
   Glu Gly Leu Gly Ile Ser Ile Thr Gly Gly Lys Glu His Gly Val Pro
       290                 295                 300
   Ile Leu Ile Ser Glu Ile His Pro Gly Gln Pro Ala Asp Arg Cys Gly
   305                 310                 315                 320
   Gly Leu His Val Gly Asp Ala Ile Leu Ala Val Asn Gly Val Asn Leu
                   325                 330                 335
   Arg Asp Thr Lys His Lys Glu Ala Val Thr Ile Leu Ser Gln Gln Arg
               340                 345                 350
   Gly Glu Ile Glu Phe Glu Val Val Tyr Val Ala Pro Glu Val Asp Ser
           355                 360                 365
   Asp Asp Glu Asn Val Glu Tyr Glu Asp Glu Ser Gly His Arg Tyr Arg
       370                 375                 380
   Leu Tyr Leu Asp Glu Leu Glu Gly Gly Gly Asn Pro Gly Ala Ser Cys
   385                 390                 395                 400
   Lys Asp Thr Ser Gly Glu Ile Lys Val Leu Gln Val Trp His Arg Arg
                   405                 410                 415
   Leu Lys Asn Gln Lys Ser Ala Lys Glu Gly Val Thr Val Leu Ile Asn
               420                 425                 430
   Glu Asp Lys Glu Leu Ala Glu Leu Arg Gly Leu Ala Ala Gly Val Gly
           435                 440                 445
   Leu Ala Asn Ala Cys Tyr Ala Ile His Thr Leu Pro Thr Gln Glu Glu
       450                 455                 460
   Ile Glu Asn Leu Pro Ala Phe Pro Arg Glu Lys Leu Thr Leu Arg Leu
   465                 470                 475                 480
   Leu Leu Gly Ser Gly Ala Phe Gly Glu Val Tyr Glu Gly Thr Ala Val
                   485                 490                 495
   Asp Ile Leu Gly Val Gly Ser Gly Glu Ile Lys Val Ala Val Lys Thr
               500                 505                 510
   Leu Lys Lys Gly Ser Thr Asp Gln Glu Lys Ile Glu Phe Leu Lys Glu
           515                 520                 525
   Ala His Leu Met Ser Lys Phe Asn His Pro Asn Ile Leu Lys Gln Leu
       530                 535                 540
   Gly Val Cys Leu Leu Asn Glu Pro Gln Tyr Ile Ile Leu Glu Leu Met
   545                 550                 555                 560
   Glu Gly Gly Asp Leu Leu Thr Tyr Leu Arg Lys Ala Arg Met Ala Thr
                   565                 570                 575
   Phe Tyr Gly Pro Leu Leu Thr Leu Val Asp Leu Val Asp Leu Cys Val
               580                 585                 590
   Asp Ile Ser Lys Gly Cys Val Tyr Leu Glu Arg Met His Phe Ile His
   595 600 605
   Arg Asp Leu Ala Ala Arg Asn Cys Leu Val Ser Val Lys Asp Tyr Thr
   610 615 620
   Ser Pro Arg Ile Val Lys Ile Gly Asp Phe Gly Leu Ala Arg Asp Ile
   625 630 635 640
   Tyr Lys Asn Asp Tyr Tyr Arg Lys Arg Gly Glu Gly Leu Leu Pro Val
   645 650 655
   Arg Trp Met Ala Pro Glu Ser Leu Met Asp Gly Ile Phe Thr Thr Gln
   660 665 670
   Ser Asp Val Trp Ser Phe Gly Ile Leu Ile Trp Glu Ile Leu Thr Leu
   675 680 685
   Gly His Gln Pro Tyr Pro Ala His Ser Asn Leu Asp Val Leu Asn Tyr
   690 695 700
   Val Gln Thr Gly Gly Arg Leu Glu Pro Pro Arg Asn Cys Pro Asp Asp
   705 710 715 720
   Leu Trp Asn Leu Met Thr Gln Cys Trp Ala Gln Glu Pro Asp Gln Arg
   725 730 735
   Pro Thr Phe His Arg Ile Gln Asp Gln Leu Gln Leu Phe Arg Asn Phe
   740 745 750
   Phe Leu Asn Ser Ile Tyr Lys Ser Arg Asp Glu Ala Asn Asn Ser Gly
   755 760 765
   Val Ile Asn Glu Ser Phe Glu Gly Glu Asp Gly Asp Val Ile Cys Leu
   770 775 780
   Asn Ser Asp Asp Ile Met Pro Val Ala Leu Met Glu Thr Lys Asn Arg
   785 790 795 800
   Glu Gly Leu Asn Tyr Met Val Leu Ala Thr Glu Cys Gly Gln Gly Glu
   805 810 815
   Glu Lys Ser Glu Gly Pro Leu Gly Ser Gln Glu Ser Glu Ser Cys Gly
   820 825 830
   Leu Arg Lys Glu Glu Lys Glu Pro His Ala Asp Lys Asp Phe Cys Gln
   835 840 845
   Glu Lys Gln Val Ala Tyr Cys Pro Ser Gly Lys Pro Glu Gly Leu Asn
       850                 855                 860
   Tyr Ala Cys Leu Thr His Ser Gly Tyr Gly Asp Gly Ser Asp
   865                 870                 875
<210> 23
   <211> 1893
   <212> DNA
   <213> Artificial Sequence
<220>
   <223> Synthetic Polynucleotide
<400> 23
   atgtcggcgg gcggtccatg cccagcagca gccggagggg gcccaggggg cgcctcctgc 60
   tccgtggggg cccctggcgg ggtatccatg ttccggtggc tggaggtgct ggagaaggag 120
   ttcgacaaag cttttgtgga tgtggatctg ctcctgggag agatcgatcc agaccaagcg 180
   gacatcactt atgaggggcg acagaagatg accagcctga gctcctgctt tgcacagctt 240
   tgccacaaag cccagtctgt gtctcaaatc aaccacaagc tggaggcaca gttggtggat 300
   ctgaaatctg aactgacaga aacccaagca gagaaagttg ttttggagaa agaagtacat 360
   gatcagcttt tacagctgca ctctattcag ctgcagcttc atgctaaaac tggtcaaagt 420
   gctgactctg gtaccattaa ggcaaaattg gaaagagagc ttgaggcaaa caaaaaagaa 480
   aaaatgaaag aagcacaact tgaagctgaa gtgaaattgt tgagaaaaga gaatgaagcc 540
   cttcgtagac atatagctgt tctccaggct gaagtatatg gggcgagact agctgccaag 600
   tacttggata aggaactggc aggaagtact cttccaaccc aagaggagat tgaaaatctt 660
   cctgccttcc ctcgggaaaa actgactctg cgtctcttgc tgggaagtgg agcctttgga 720
   gaagtgtatg aaggaacagc agtggacatc ttaggagttg gaagtggaga aatcaaagta 780
   gcagtgaaga ctttgaagaa gggttccaca gaccaggaga agattgaatt cctgaaggag 840
   gcacatctga tgagcaaatt taatcatccc aacattctga agcagcttgg agtttgtctg 900
   ctgaatgaac cccaatacat tatcctggaa ctgatggagg gaggagacct tcttacttat 960
   ttgcgtaaag cccggatggc aacgttttat ggtcctttac tcaccttggt tgaccttgta 1020
   gacctgtgtg tagatatttc aaaaggctgt gtctacttgg aacggatgca tttcattcac 1080
   agggatctgg cagctagaaa ttgccttgtt tccgtgaaag actataccag tccacggata 1140
   gtgaagattg gagactttgg actcgccaga gacatctata aaaatgatta ctatagaaag 1200
   agaggggaag gcctgctccc agttcggtgg atggctccag aaagtttgat ggatggaatc 1260
   ttcactactc aatctgatgt atggtctttt ggaattctga tttgggagat tttaactctt 1320
   ggtcatcagc cttatccagc tcattccaac cttgatgtgt taaactatgt gcaaacagga 1380
   gggagactgg agccaccaag aaattgtcct gatgatctgt ggaatttaat gacccagtgc 1440
   tgggctcaag aacccgacca aagacctact tttcatagaa ttcaggacca acttcagtta 1500
   ttcagaaatt ttttcttaaa tagcatttat aagtccagag atgaagcaaa caacagtgga 1560
   gtcataaatg aaagctttga aggtgaagat ggcgatgtga tttgtttgaa ttcagatgac 1620
   attatgccag ttgctttaat ggaaacgaag aaccgagaag ggttaaacta tatggtactt 1680
   gctacagaat gtggccaagg tgaagaaaag tctgagggtc ctctaggctc ccaggaatct 1740
   gaatcttgtg gtctgaggaa agaagagaag gaaccacatg cagacaaaga tttctgccaa 1800
   gaaaaacaag tggcttactg cccttctggc aagcctgaag gcctgaacta tgcctgtctc 1860
   actcacagtg gatatggaga tgggtctgat taa 1893
<210> 24
   <211> 630
   <212> PRT
   <213> Artificial Sequence
<220>
   <223> Synthetic Polypeptide
<400> 24
   Met Ser Ala Gly Gly Pro Cys Pro Ala Ala Ala Gly Gly Gly Pro Gly
   1               5                   10                  15
   Gly Ala Ser Cys Ser Val Gly Ala Pro Gly Gly Val Ser Met Phe Arg
               20                  25                  30
   Trp Leu Glu Val Leu Glu Lys Glu Phe Asp Lys Ala Phe Val Asp Val
           35                  40                  45
   Asp Leu Leu Leu Gly Glu Ile Asp Pro Asp Gln Ala Asp Ile Thr Tyr
       50                  55                  60
   Glu Gly Arg Gln Lys Met Thr Ser Leu Ser Ser Cys Phe Ala Gln Leu
   65                  70                  75                  80
   Cys His Lys Ala Gln Ser Val Ser Gln Ile Asn His Lys Leu Glu Ala
                   85                  90                  95
   Gln Leu Val Asp Leu Lys Ser Glu Leu Thr Glu Thr Gln Ala Glu Lys
               100                 105                 110
   Val Val Leu Glu Lys Glu Val His Asp Gln Leu Leu Gln Leu His Ser
           115                 120                 125
   Ile Gln Leu Gln Leu His Ala Lys Thr Gly Gln Ser Ala Asp Ser Gly
       130                 135                 140
   Thr Ile Lys Ala Lys Leu Glu Arg Glu Leu Glu Ala Asn Lys Lys Glu
   145 150 155 160
   Lys Met Lys Glu Ala Gln Leu Glu Ala Glu Val Lys Leu Leu Arg Lys
   165 170 175
   Glu Asn Glu Ala Leu Arg Arg His Ile Ala Val Leu Gln Ala Glu Val
   180 185 190
   Tyr Gly Ala Arg Leu Ala Ala Lys Tyr Leu Asp Lys Glu Leu Ala Gly
   195 200 205
   Ser Thr Leu Pro Thr Gln Glu Glu Ile Glu Asn Leu Pro Ala Phe Pro
   210 215 220
   Arg Glu Lys Leu Thr Leu Arg Leu Leu Leu Gly Ser Gly Ala Phe Gly
   225 230 235 240
   Glu Val Tyr Glu Gly Thr Ala Val Asp Ile Leu Gly Val Gly Ser Gly
   245 250 255
   Glu Ile Lys Val Ala Val Lys Thr Leu Lys Lys Gly Ser Thr Asp Gln
   260 265 270
   Glu Lys Ile Glu Phe Leu Lys Glu Ala His Leu Met Ser Lys Phe Asn
   275 280 285
   His Pro Asn Ile Leu Lys Gln Leu Gly Val Cys Leu Leu Asn Glu Pro
   290 295 300
   Gln Tyr Ile Ile Leu Glu Leu Met Glu Gly Gly Asp Leu Leu Thr Tyr
   305 310 315 320
   Leu Arg Lys Ala Arg Met Ala Thr Phe Tyr Gly Pro Leu Leu Thr Leu
   325 330 335
   Val Asp Leu Val Asp Leu Cys Val Asp Ile Ser Lys Gly Cys Val Tyr
   340 345 350
   Leu Glu Arg Met His Phe Ile His Arg Asp Leu Ala Ala Arg Asn Cys
   355 360 365
   Leu Val Ser Val Lys Asp Tyr Thr Ser Pro Arg Ile Val Lys Ile Gly
   370 375 380
   Asp Phe Gly Leu Ala Arg Asp Ile Tyr Lys Asn Asp Tyr Tyr Arg Lys
   385 390 395 400
   Arg Gly Glu Gly Leu Leu Pro Val Arg Trp Met Ala Pro Glu Ser Leu
   405 410 415
   Met Asp Gly Ile Phe Thr Thr Gln Ser Asp Val Trp Ser Phe Gly Ile
   420 425 430
   Leu Ile Trp Glu Ile Leu Thr Leu Gly His Gln Pro Tyr Pro Ala His
   435 440 445
   Ser Asn Leu Asp Val Leu Asn Tyr Val Gln Thr Gly Gly Arg Leu Glu
   450 455 460
   Pro Pro Arg Asn Cys Pro Asp Asp Leu Trp Asn Leu Met Thr Gln Cys
   465 470 475 480
   Trp Ala Gln Glu Pro Asp Gln Arg Pro Thr Phe His Arg Ile Gln Asp
   485 490 495
   Gln Leu Gln Leu Phe Arg Asn Phe Phe Leu Asn Ser Ile Tyr Lys Ser
   500 505 510
   Arg Asp Glu Ala Asn Asn Ser Gly Val Ile Asn Glu Ser Phe Glu Gly
   515 520 525
   Glu Asp Gly Asp Val Ile Cys Leu Asn Ser Asp Asp Ile Met Pro Val
   530 535 540
   Ala Leu Met Glu Thr Lys Asn Arg Glu Gly Leu Asn Tyr Met Val Leu
   545 550 555 560
   Ala Thr Glu Cys Gly Gln Gly Glu Glu Lys Ser Glu Gly Pro Leu Gly
   565 570 575
   Ser Gln Glu Ser Glu Ser Cys Gly Leu Arg Lys Glu Glu Lys Glu Pro
   580 585 590
   His Ala Asp Lys Asp Phe Cys Gln Glu Lys Gln Val Ala Tyr Cys Pro
   595 600 605
   Ser Gly Lys Pro Glu Gly Leu Asn Tyr Ala Cys Leu Thr His Ser Gly
   610 615 620
   Tyr Gly Asp Gly Ser Asp
   625 630
<210> 25
   <211> 3030
   <212> DNA
   <213> Artificial Sequence
<220>
   <223> Synthetic Polynucleotide
<400> 25
   atgtcggcgg gcggtccatg cccagcagca gccggagggg gcccaggggg cgcctcctgc 60
   tccgtggggg cccctggcgg ggtatccatg ttccggtggc tggaggtgct ggagaaggag 120
   ttcgacaaag cttttgtgga tgtggatctg ctcctgggag agatcgatcc agaccaagcg 180
   gacatcactt atgaggggcg acagaagatg accagcctga gctcctgctt tgcacagctt 240
   tgccacaaag cccagtctgt gtctcaaatc aaccacaagc tggaggcaca gttggtggat 300
   ctgaaatctg aactgacaga aacccaagca gagaaagttg ttttggagaa agaagtacat 360
   gatcagcttt tacagctgca ctctattcag ctgcagcttc atgctaaaac tggtcaaagt 420
   gctgactctg gtaccattaa ggcaaaattg gaaagagagc ttgaggcaaa caaaaaagaa 480
   aaaatgaaag aagcacaact tgaagctgaa gtgaaattgt tgagaaaaga gaatgaagcc 540
   cttcgtagac atatagctgt tctccaggct gaagtatatg gggcgagact agctgccaag 600
   tacttggata aggaactggc aggaagggtc caacagatac aattgctagg acgagatatg 660
   aagggacctg ctcatgataa gctttggaac caattagaag ctgaaataca tttgcatcgt 720
   cacaaaactg tgatccgagc ctgcagagga cgtaatgact tgaaacgacc aatgcaagca 780
   ccaccaggcc atgatcaaga ttccctaaag aaaagccaag gtgttggtcc aattagaaaa 840
   gttctcctcc ttaaggaaga tcatgaaggc cttggcattt caattacagg tgggaaagaa 900
   catggtgttc caatcctcat ctctgagatc catccggggc aacctgctga tagatgcgga 960
   gggctgcacg ttggggatgc tattttggca gtcaacggag ttaacctaag ggacacaaag 1020
   cataaagaag ctgtaactat tctttctcag cagagaggag agattgaatt tgaagtagtt 1080
   tatgtggctc ctgaagtgga ttctgatgat gaaaacgtag agtatgaaga tgagagtgga 1140
   catcgttacc gtttgtacct tgatgagtta gaaggaggtg gtaaccctgg tgctagttgc 1200
   aaagacacaa gtggggaaat caaagtatta caagctggag tcccaaataa accaggcatt 1260
   cccaaattac tagaagggag taaaaattca atacagtggg agaaagctga agataatgga 1320
   tgtagaatta catactatat ccttgagata agaaagagca cttcaaataa tttacagaac 1380
   cagaatttaa ggtggaagat gacatttaat ggatcctgca gtagtgtttg cacatggaag 1440
   tccaaaaacc tgaaaggaat atttcagttc agagtagtag ctgcaaataa tctagggttt 1500
   ggtgaatata gtggaatcag tgagaatatt atattagttg gagatgattt ttggatacca 1560
   gaaacaagtt tcatacttac tattatagtt ggaatatttc tggttgttac aatcccactg 1620
   acctttgtct ggcatagaag attaaagaat caaaaaagtg ccaaggaagg ggtgacagtg 1680
   cttataaacg aagacaaaga gttggctgag ctgcgaggtc tggcagccgg agtaggcctg 1740
   gctaatgcct gctatgcaat acatactctt ccaacccaag aggagattga aaatcttcct 1800
   gccttccctc gggaaaaact gactctgcgt ctcttgctgg gaagtggagc ctttggagaa 1860
   gtgtatgaag gaacagcagt ggacatctta ggagttggaa gtggagaaat caaagtagca 1920
   gtgaagactt tgaagaaggg ttccacagac caggagaaga ttgaattcct gaaggaggca 1980
   catctgatga gcaaatttaa tcatcccaac attctgaagc agcttggagt ttgtctgctg 2040
   aatgaacccc aatacattat cctggaactg atggagggag gagaccttct tacttatttg 2100
   cgtaaagccc ggatggcaac gttttatggt cctttactca ccttggttga ccttgtagac 2160
   ctgtgtgtag atatttcaaa aggctgtgtc tacttggaac ggatgcattt cattcacagg 2220
   gatctggcag ctagaaattg ccttgtttcc gtgaaagact ataccagtcc acggatagtg 2280
   aagattggag actttggact cgccagagac atctataaaa atgattacta tagaaagaga 2340
   ggggaaggcc tgctcccagt tcggtggatg gctccagaaa gtttgatgga tggaatcttc 2400
   actactcaat ctgatgtatg gtcttttgga attctgattt gggagatttt aactcttggt 2460
   catcagcctt atccagctca ttccaacctt gatgtgttaa actatgtgca aacaggaggg 2520
   agactggagc caccaagaaa ttgtcctgat gatctgtgga atttaatgac ccagtgctgg 2580
   gctcaagaac ccgaccaaag acctactttt catagaattc aggaccaact tcagttattc 2640
   agaaattttt tcttaaatag catttataag tccagagatg aagcaaacaa cagtggagtc 2700
   ataaatgaaa gctttgaagg tgaagatggc gatgtgattt gtttgaattc agatgacatt 2760
   atgccagttg ctttaatgga aacgaagaac cgagaagggt taaactatat ggtacttgct 2820
   acagaatgtg gccaaggtga agaaaagtct gagggtcctc taggctccca ggaatctgaa 2880
   tcttgtggtc tgaggaaaga agagaaggaa ccacatgcag acaaagattt ctgccaagaa 2940
   aaacaagtgg cttactgccc ttctggcaag cctgaaggcc tgaactatgc ctgtctcact 3000
   cacagtggat atggagatgg gtctgattaa 3030
<210> 26
   <211> 1009
   <212> PRT
   <213> Artificial Sequence
<220>
   <223> Synthetic Polypeptide
<400> 26
   Met Ser Ala Gly Gly Pro Cys Pro Ala Ala Ala Gly Gly Gly Pro Gly
   1 5 10 15
   Gly Ala Ser Cys Ser Val Gly Ala Pro Gly Gly Val Ser Met Phe Arg
   20 25 30
   Trp Leu Glu Val Leu Glu Lys Glu Phe Asp Lys Ala Phe Val Asp Val
   35 40 45
   Asp Leu Leu Leu Gly Glu Ile Asp Pro Asp Gln Ala Asp Ile Thr Tyr
   50 55 60
   Glu Gly Arg Gln Lys Met Thr Ser Leu Ser Ser Cys Phe Ala Gln Leu
   65 70 75 80
   Cys His Lys Ala Gln Ser Val Ser Gln Ile Asn His Lys Leu Glu Ala
   85 90 95
   Gln Leu Val Asp Leu Lys Ser Glu Leu Thr Glu Thr Gln Ala Glu Lys
   100 105 110
   Val Val Leu Glu Lys Glu Val His Asp Gln Leu Leu Gln Leu His Ser
   115 120 125
   Ile Gln Leu Gln Leu His Ala Lys Thr Gly Gln Ser Ala Asp Ser Gly
   130 135 140
   Thr Ile Lys Ala Lys Leu Glu Arg Glu Leu Glu Ala Asn Lys Lys Glu
   145 150 155 160
   Lys Met Lys Glu Ala Gln Leu Glu Ala Glu Val Lys Leu Leu Arg Lys
   165 170 175
   Glu Asn Glu Ala Leu Arg Arg His Ile Ala Val Leu Gln Ala Glu Val
   180 185 190
   Tyr Gly Ala Arg Leu Ala Ala Lys Tyr Leu Asp Lys Glu Leu Ala Gly
   195 200 205
   Arg Val Gln Gln Ile Gln Leu Leu Gly Arg Asp Met Lys Gly Pro Ala
   210 215 220
   His Asp Lys Leu Trp Asn Gln Leu Glu Ala Glu Ile His Leu His Arg
   225 230 235 240
   His Lys Thr Val Ile Arg Ala Cys Arg Gly Arg Asn Asp Leu Lys Arg
   245 250 255
   Pro Met Gln Ala Pro Pro Gly His Asp Gln Asp Ser Leu Lys Lys Ser
   260 265 270
   Gln Gly Val Gly Pro Ile Arg Lys Val Leu Leu Leu Lys Glu Asp His
   275 280 285
   Glu Gly Leu Gly Ile Ser Ile Thr Gly Gly Lys Glu His Gly Val Pro
   290 295 300
   Ile Leu Ile Ser Glu Ile His Pro Gly Gln Pro Ala Asp Arg Cys Gly
   305 310 315 320
   Gly Leu His Val Gly Asp Ala Ile Leu Ala Val Asn Gly Val Asn Leu
   325 330 335
   Arg Asp Thr Lys His Lys Glu Ala Val Thr Ile Leu Ser Gln Gln Arg
   340 345 350
   Gly Glu Ile Glu Phe Glu Val Val Tyr Val Ala Pro Glu Val Asp Ser
   355 360 365
   Asp Asp Glu Asn Val Glu Tyr Glu Asp Glu Ser Gly His Arg Tyr Arg
   370 375 380
   Leu Tyr Leu Asp Glu Leu Glu Gly Gly Gly Asn Pro Gly Ala Ser Cys
   385 390 395 400
   Lys Asp Thr Ser Gly Glu Ile Lys Val Leu Gln Ala Gly Val Pro Asn
   405 410 415
   Lys Pro Gly Ile Pro Lys Leu Leu Glu Gly Ser Lys Asn Ser Ile Gln
   420 425 430
   Trp Glu Lys Ala Glu Asp Asn Gly Cys Arg Ile Thr Tyr Tyr Ile Leu
   435 440 445
   Glu Ile Arg Lys Ser Thr Ser Asn Asn Leu Gln Asn Gln Asn Leu Arg
   450 455 460
   Trp Lys Met Thr Phe Asn Gly Ser Cys Ser Ser Val Cys Thr Trp Lys
   465 470 475 480
   Ser Lys Asn Leu Lys Gly Ile Phe Gln Phe Arg Val Val Ala Ala Asn
   485 490 495
   Asn Leu Gly Phe Gly Glu Tyr Ser Gly Ile Ser Glu Asn Ile Ile Leu
   500 505 510
   Val Gly Asp Asp Phe Trp Ile Pro Glu Thr Ser Phe Ile Leu Thr Ile
   515 520 525
   Ile Val Gly Ile Phe Leu Val Val Thr Ile Pro Leu Thr Phe Val Trp
       530                 535                 540
   His Arg Arg Leu Lys Asn Gln Lys Ser Ala Lys Glu Gly Val Thr Val
   545                 550                 555                 560
   Leu Ile Asn Glu Asp Lys Glu Leu Ala Glu Leu Arg Gly Leu Ala Ala
                   565                 570                 575
   Gly Val Gly Leu Ala Asn Ala Cys Tyr Ala Ile His Thr Leu Pro Thr
               580                 585                 590
   Gln Glu Glu Ile Glu Asn Leu Pro Ala Phe Pro Arg Glu Lys Leu Thr
           595                 600                 605
   Leu Arg Leu Leu Leu Gly Ser Gly Ala Phe Gly Glu Val Tyr Glu Gly
       610                 615                 620
   Thr Ala Val Asp Ile Leu Gly Val Gly Ser Gly Glu Ile Lys Val Ala
   625                 630                 635                 640
   Val Lys Thr Leu Lys Lys Gly Ser Thr Asp Gln Glu Lys Ile Glu Phe
                   645                 650                 655
   Leu Lys Glu Ala His Leu Met Ser Lys Phe Asn His Pro Asn Ile Leu
               660                 665                 670
   Lys Gln Leu Gly Val Cys Leu Leu Asn Glu Pro Gln Tyr Ile Ile Leu
           675                 680                 685
   Glu Leu Met Glu Gly Gly Asp Leu Leu Thr Tyr Leu Arg Lys Ala Arg
       690                 695                 700
   Met Ala Thr Phe Tyr Gly Pro Leu Leu Thr Leu Val Asp Leu Val Asp
   705                 710                 715                 720
   Leu Cys Val Asp Ile Ser Lys Gly Cys Val Tyr Leu Glu Arg Met His
                   725                 730                 735
   Phe Ile His Arg Asp Leu Ala Ala Arg Asn Cys Leu Val Ser Val Lys
               740                 745                 750
   Asp Tyr Thr Ser Pro Arg Ile Val Lys Ile Gly Asp Phe Gly Leu Ala
           755                 760                 765
   Arg Asp Ile Tyr Lys Asn Asp Tyr Tyr Arg Lys Arg Gly Glu Gly Leu
       770                 775                 780
   Leu Pro Val Arg Trp Met Ala Pro Glu Ser Leu Met Asp Gly Ile Phe
   785                 790                 795                 800
   Thr Thr Gln Ser Asp Val Trp Ser Phe Gly Ile Leu Ile Trp Glu Ile
                   805                 810                 815
   Leu Thr Leu Gly His Gln Pro Tyr Pro Ala His Ser Asn Leu Asp Val
               820                 825                 830
   Leu Asn Tyr Val Gln Thr Gly Gly Arg Leu Glu Pro Pro Arg Asn Cys
           835                 840                 845
   Pro Asp Asp Leu Trp Asn Leu Met Thr Gln Cys Trp Ala Gln Glu Pro
       850                 855                 860
   Asp Gln Arg Pro Thr Phe His Arg Ile Gln Asp Gln Leu Gln Leu Phe
   865                 870                 875                 880
   Arg Asn Phe Phe Leu Asn Ser Ile Tyr Lys Ser Arg Asp Glu Ala Asn
                   885                 890                 895
   Asn Ser Gly Val Ile Asn Glu Ser Phe Glu Gly Glu Asp Gly Asp Val
               900                 905                 910
   Ile Cys Leu Asn Ser Asp Asp Ile Met Pro Val Ala Leu Met Glu Thr
           915                 920                 925
   Lys Asn Arg Glu Gly Leu Asn Tyr Met Val Leu Ala Thr Glu Cys Gly
       930                 935                 940
   Gln Gly Glu Glu Lys Ser Glu Gly Pro Leu Gly Ser Gln Glu Ser Glu
   945                 950                 955                 960
   Ser Cys Gly Leu Arg Lys Glu Glu Lys Glu Pro His Ala Asp Lys Asp
                   965                 970                 975
   Phe Cys Gln Glu Lys Gln Val Ala Tyr Cys Pro Ser Gly Lys Pro Glu
               980                 985                 990
   Gly Leu Asn Tyr Ala Cys Leu Thr  His Ser Gly Tyr Gly  Asp Gly Ser
           995                 1000                1005
   Asp
<210> 27
   <211> 278
   <212> PRT
   <213> Artificial Sequence
<220>
   <223> Synthetic Polypeptide
<400> 27
   Leu Thr Leu Arg Leu Leu Leu Gly Ser Gly Ala Phe Gly Glu Val Tyr
   1               5                   10                  15
   Glu Gly Thr Ala Val Asp Ile Leu Gly Val Gly Ser Gly Glu Ile Lys
               20                  25                  30
   Val Ala Val Lys Thr Leu Lys Lys Gly Ser Thr Asp Gln Glu Lys Ile
           35                  40                  45
   Glu Phe Leu Lys Glu Ala His Leu Met Ser Lys Phe Asn His Pro Asn
       50                  55                  60
   Ile Leu Lys Gln Leu Gly Val Cys Leu Leu Asn Glu Pro Gln Tyr Ile
   65                  70                  75                  80
   Ile Leu Glu Leu Met Glu Gly Gly Asp Leu Leu Thr Tyr Leu Arg Lys
                   85                  90                  95
   Ala Arg Met Ala Thr Phe Tyr Gly Pro Leu Leu Thr Leu Val Asp Leu
               100                 105                 110
   Val Asp Leu Cys Val Asp Ile Ser Lys Gly Cys Val Tyr Leu Glu Arg
           115                 120                 125
   Met His Phe Ile His Arg Asp Leu Ala Ala Arg Asn Cys Leu Val Ser
       130                 135                 140
   Val Lys Asp Tyr Thr Ser Pro Arg Ile Val Lys Ile Gly Asp Phe Gly
   145                 150                 155                 160
   Leu Ala Arg Asp Ile Tyr Lys Asn Asp Tyr Tyr Arg Lys Arg Gly Glu
                   165                 170                 175
   Gly Leu Leu Pro Val Arg Trp Met Ala Pro Glu Ser Leu Met Asp Gly
               180                 185                 190
   Ile Phe Thr Thr Gln Ser Asp Val Trp Ser Phe Gly Ile Leu Ile Trp
           195                 200                 205
   Glu Ile Leu Thr Leu Gly His Gln Pro Tyr Pro Ala His Ser Asn Leu
           210                 215                 220
   Asp Val Leu Asn Tyr Val Gln Thr Gly Gly Arg Leu Glu Pro Pro Arg
   225                 230                 235                 240
   Asn Cys Pro Asp Asp Leu Trp Asn Leu Met Thr Gln Cys Trp Ala Gln
                   245                 250                 255
   Glu Pro Asp Gln Arg Pro Thr Phe His Arg Ile Gln Asp Gln Leu Gln
               260                 265                 270
   Leu Phe Arg Asn Phe Phe
           275
<210> 28
   <211> 6
   <212> PRT
   <213> Artificial Sequence
<220>
   <223> Synthetic Polypeptide
<400> 28
   Val Gly Val Trp His Arg
   1               5
<210> 29
   <211> 6
   <212> PRT
   <213> Artificial Sequence
<220>
   <223> Synthetic Polypeptide
<400> 29
   Leu Val Gly Asp Asp Phe
   1               5
<210> 30
   <211> 6
   <212> PRT
   <213> Artificial Sequence
<220>
   <223> Synthetic Polypeptide
<400> 30
   Leu Val Gly Ala Gly Val
   1               5
<210> 31
   <211> 6
   <212> PRT
   <213> Artificial Sequence
<220>
   <223> Synthetic Polypeptide
<400> 31
   Pro Pro Lys Asp Asp Phe
   1 5
<210> 32
   <211> 6
   <212> PRT
   <213> Artificial Sequence
<220>
   <223> Synthetic Polypeptide
<400> 32
   Ala Gly Ser Thr Leu Pro
   1 5
<210> 33
   <211> 6
   <212> PRT
   <213> Artificial Sequence
<220>
   <223> Synthetic Polypeptide
<400> 33
   Leu Gln Val Trp His Arg
   1 5
<210> 34
   <211> 6
   <212> PRT
   <213> Artificial Sequence
<220>
   <223> Synthetic Polypeptide
<400> 34
   Val Leu Gln Ala Gly Val
   1 5
<210> 35
   <211> 1620
   <212> PRT
   <213> Artificial Sequence
<220>
   <223> Synthetic Polypeptide
<400> 35
<210> 36
   <211> 6222
   <212> DNA
   <213> Artificial Sequence
<220>
   <223> Synthetic Polynucleotide
<400> 36
   gggggcggca gcggtggtag cagctggtac ctcccgccgc ctctgttcgg agggtcgcgg      60
   ggcaccgagg tgctttccgg ccgccctctg gtcggccacc caaagccgcg ggcgctgatg     120
   atgggtgagg agggggcggc aagatttcgg gcgcccctgc cctgaacgcc ctcagctgct     180
   gccgccgggg ccgctccagt gcctgcgaac tctgaggagc cgaggcgccg gtgagagcaa     240
   ggacgctgca aacttgcgca gcgcgggggc tgggattcac gcccagaagt tcagcaggca     300
   gacagtccga agccttcccg cagcggagag atagcttgag ggtgcgcaag acggcagcct     360
   ccgccctcgg ttcccgccca gaccgggcag aagagcttgg aggagccaaa aggaacgcaa     420
   aaggcggcca ggacagcgtg cagcagctgg gagccgccgt tctcagcctt aaaagttgca     480
   gagattggag gctgccccga gaggggacag accccagctc cgactgcggg gggcaggaga 540
   ggacggtacc caactgccac ctcccttcaa ccatagtagt tcctctgtac cgagcgcagc 600
   gagctacaga cgggggcgcg gcactcggcg cggagagcgg gaggctcaag gtcccagcca 660
   gtgagcccag tgtgcttgag tgtctctgga ctcgcccctg agcttccagg tctgtttcat 720
   ttagactcct gctcgcctcc gtgcagttgg gggaaagcaa gagacttgcg cgcacgcaca 780
   gtcctctgga gatcaggtgg aaggagccgc tgggtaccaa ggactgttca gagcctcttc 840
   ccatctcggg gagagcgaag ggtgaggctg ggcccggaga gcagtgtaaa cggcctcctc 900
   cggcgggatg ggagccatcg ggctcctgtg gctcctgccg ctgctgcttt ccacggcagc 960
   tgtgggctcc gggatgggga ccggccagcg cgcgggctcc ccagctgcgg ggccgccgct 1020
   gcagccccgg gagccactca gctactcgcg cctgcagagg aagagtctgg cagttgactt 1080
   cgtggtgccc tcgctcttcc gtgtctacgc ccgggaccta ctgctgccac catcctcctc 1140
   ggagctgaag gctggcaggc ccgaggcccg cggctcgcta gctctggact gcgccccgct 1200
   gctcaggttg ctggggccgg cgccgggggt ctcctggacc gccggttcac cagccccggc 1260
   agaggcccgg acgctgtcca gggtgctgaa gggcggctcc gtgcgcaagc tccggcgtgc 1320
   caagcagttg gtgctggagc tgggcgagga ggcgatcttg gagggttgcg tcgggccccc 1380
   cggggaggcg gctgtggggc tgctccagtt caatctcagc gagctgttca gttggtggat 1440
   tcgccaaggc gaagggcgac tgaggatccg cctgatgccc gagaagaagg cgtcggaagt 1500
   gggcagagag ggaaggctgt ccgcggcaat tcgcgcctcc cagccccgcc ttctcttcca 1560
   gatcttcggg actggtcata gctccttgga atcaccaaca aacatgcctt ctccttctcc 1620
   tgattatttt acatggaatc tcacctggat aatgaaagac tccttccctt tcctgtctca 1680
   tcgcagccga tatggtctgg agtgcagctt tgacttcccc tgtgagctgg agtattcccc 1740
   tccactgcat gacctcagga accagagctg gtcctggcgc cgcatcccct ccgaggaggc 1800
   ctcccagatg gacttgctgg atgggcctgg ggcagagcgt tctaaggaga tgcccagagg 1860
   ctcctttctc cttctcaaca cctcagctga ctccaagcac accatcctga gtccgtggat 1920
   gaggagcagc agtgagcact gcacactggc cgtctcggtg cacaggcacc tgcagccctc 1980
   tggaaggtac attgcccagc tgctgcccca caacgaggct gcaagagaga tcctcctgat 2040
   gcccactcca gggaagcatg gttggacagt gctccaggga agaatcgggc gtccagacaa 2100
   cccatttcga gtggccctgg aatacatctc cagtggaaac cgcagcttgt ctgcagtgga 2160
   cttctttgcc ctgaagaact gcagtgaagg aacatcccca ggctccaaga tggccctgca 2220
   gagctccttc acttgttgga atgggacagt cctccagctt gggcaggcct gtgacttcca 2280
   ccaggactgt gcccagggag aagatgagag ccagatgtgc cggaaactgc ctgtgggttt 2340
   ttactgcaac tttgaagatg gcttctgtgg ctggacccaa ggcacactgt caccccacac 2400
   tcctcaatgg caggtcagga ccctaaagga tgcccggttc caggaccacc aagaccatgc 2460
   tctattgctc agtaccactg atgtccccgc ttctgaaagt gctacagtga ccagtgctac 2520
   gtttcctgca ccgatcaaga gctctccatg tgagctccga atgtcctggc tcattcgtgg 2580
   agtcttgagg ggaaacgtgt ccttggtgct agtggagaac aaaaccggga aggagcaagg 2640
   caggatggtc tggcatgtcg ccgcctatga aggcttgagc ctgtggcagt ggatggtgtt 2700
   gcctctcctc gatgtgtctg acaggttctg gctgcagatg gtcgcatggt ggggacaagg 2760
   atccagagcc atcgtggctt ttgacaatat ctccatcagc ctggactgct acctcaccat 2820
   tagcggagag gacaagatcc tgcagaatac agcacccaaa tcaagaaacc tgtttgagag 2880
   aaacccaaac aaggagctga aacccgggga aaattcacca agacagaccc ccatctttga 2940
   ccctacagtt cattggctgt tcaccacatg tggggccagc gggccccatg gccccaccca 3000
   ggcacagtgc aacaacgcct accagaactc caacctgagc gtggaggtgg ggagcgaggg 3060
   ccccctgaaa ggcatccaga tctggaaggt gccagccacc gacacctaca gcatctcggg 3120
   ctacggagct gctggcggga aaggcgggaa gaacaccatg atgcggtccc acggcgtgtc 3180
   tgtgctgggc atcttcaacc tggagaagga tgacatgctg tacatcctgg ttgggcagca 3240
   gggagaggac gcctgcccca gtacaaacca gttaatccag aaagtctgca ttggagagaa 3300
   caatgtgata gaagaagaaa tccgtgtgaa cagaagcgtg catgagtggg caggaggcgg 3360
   aggaggaggg ggtggagcca cctacgtatt taagatgaag gatggagtgc cggtgcccct 3420
   gatcattgca gccggaggtg gtggcagggc ctacggggcc aagacagaca cgttccaccc 3480
   agagagactg gagaataact cctcggttct agggctaaac ggcaattccg gagccgcagg 3540
   tggtggaggt ggctggaatg ataacacttc cttgctctgg gccggaaaat ctttgcagga 3600
   gggtgccacc ggaggacatt cctgccccca ggccatgaag aagtgggggt gggagacaag 3660
   agggggtttc ggagggggtg gaggggggtg ctcctcaggt ggaggaggcg gaggatatat 3720
   aggcggcaat gcagcctcaa acaatgaccc cgaaatggat ggggaagatg gggtttcctt 3780
   catcagtcca ctgggcatcc tgtacacccc agctttaaaa gtgatggaag gccacgggga 3840
   agtgaatatt aagcattatc taaactgcag tcactgtgag gtagacgaat gtcacatgga 3900
   ccctgaaagc cacaaggtca tctgcttctg tgaccacggg acggtgctgg ctgaggatgg 3960
   cgtctcctgc attgtgtcac ccaccccgga gccacacctg ccactctcgc tgatcctctc 4020
   tgtggtgacc tctgccctcg tggccgccct ggtcctggct ttctccggca tcatgattgt 4080
   gtaccgccgg aagcaccagg agctgcaagc catgcagatg gagctgcaga gccctgagta 4140
   caagctgagc aagctccgca cctcgaccat catgaccgac tacaacccca actactgctt 4200
   tgctggcaag acctcctcca tcagtgacct gaaggaggtg ccgcggaaaa acatcaccct 4260
   cattcggggt ctgggccatg gcgcctttgg ggaggtgtat gaaggccagg tgtccggaat 4320
   gcccaacgac ccaagccccc tgcaagtggc tgtgaagacg ctgcctgaag tgtgctctga 4380
   acaggacgaa ctggatttcc tcatggaagc cctgatcatc agcaaattca accaccagaa 4440
   cattgttcgc tgcattgggg tgagcctgca atccctgccc cggttcatcc tgctggagct 4500
   catggcgggg ggagacctca agtccttcct ccgagagacc cgccctcgcc cgagccagcc 4560
   ctcctccctg gccatgctgg accttctgca cgtggctcgg gacattgcct gtggctgtca 4620
   gtatttggag gaaaaccact tcatccaccg agacattgct gccagaaact gcctcttgac 4680
   ctgtccaggc cctggaagag tggccaagat tggagacttc gggatggccc gagacatcta 4740
   cagggcgagc tactatagaa agggaggctg tgccatgctg ccagttaagt ggatgccccc 4800
   agaggccttc atggaaggaa tattcacttc taaaacagac acatggtcct ttggagtgct 4860
   gctatgggaa atcttttctc ttggatatat gccatacccc agcaaaagca accaggaagt 4920
   tctggagttt gtcaccagtg gaggccggat ggacccaccc aagaactgcc ctgggcctgt 4980
   ataccggata atgactcagt gctggcaaca tcagcctgaa gacaggccca actttgccat 5040
   cattttggag aggattgaat actgcaccca ggacccggat gtaatcaaca ccgctttgcc 5100
   gatagaatat ggtccacttg tggaagagga agagaaagtg cctgtgaggc ccaaggaccc 5160
   tgagggggtt cctcctctcc tggtctctca acaggcaaaa cgggaggagg agcgcagccc 5220
   agctgcccca ccacctctgc ctaccacctc ctctggcaag gctgcaaaga aacccacagc 5280
   tgcagagatc tctgttcgag tccctagagg gccggccgtg gaagggggac acgtgaatat 5340
   ggcattctct cagtccaacc ctccttcgga gttgcacaag gtccacggat ccagaaacaa 5400
   gcccaccagc ttgtggaacc caacgtacgg ctcctggttt acagagaaac ccaccaaaaa 5460
   gaataatcct atagcaaaga aggagccaca cgacaggggt aacctggggc tggagggaag 5520
   ctgtactgtc ccacctaacg ttgcaactgg gagacttccg ggggcctcac tgctcctaga 5580
   gccctcttcg ctgactgcca atatgaagga ggtacctctg ttcaggctac gtcacttccc 5640
   ttgtgggaat gtcaattacg gctaccagca acagggcttg cccttagaag ccgctactgc 5700
   ccctggagct ggtcattacg aggataccat tctgaaaagc aagaatagca tgaaccagcc 5760
   tgggccctga gctcggtcgc acactcactt ctcttccttg ggatccctaa gaccgtggag 5820
   gagagagagg caatggctcc ttcacaaacc agagaccaaa tgtcacgttt tgttttgtgc 5880
   caacctattt tgaagtacca ccaaaaaagc tgtattttga aaatgcttta gaaaggtttt 5940
   gagcatgggt tcatcctatt ctttcgaaag aagaaaatat cataaaaatg agtgataaat 6000
   acaaggccca gatgtggttg cataaggttt ttatgcatgt ttgttgtata cttccttatg 6060
   cttctttcaa attgtgtgtg ctctgcttca atgtagtcag aattagctgc ttctatgttt 6120
   catagttggg gtcatagatg tttccttgcc ttgttgatgt ggacatgagc catttgaggg     6180
   gagagggaac ggaaataaag gagttatttg taatgactaa aa                        6222